(19) Europäisches Patentamt
European Patent Office
Office européen des brevets

(11) Veröffentlichungsnummer: **0 170 204 B1**

# (12) EUROPÄISCHE PATENTSCHRIFT

(45) Veröffentlichungstag der Patentschrift: **25.09.91**

(51) Int. Cl.[5]: **C12N 15/20**, C12P 21/02, C12N 1/21, C12N 5/10, A61K 37/66

(21) Anmeldenummer: **85109284.1**

(22) Anmeldetag: **24.07.85**

(54) **Neue genetische Sequenzen, die durch sie codierten Interferon-Peptide vom Typ I und diese sie produzierende Organismen.**

(30) Priorität: **01.08.84 DE 3428370**
**14.02.85 DE 3505060**

(43) Veröffentlichungstag der Anmeldung:
**05.02.86 Patentblatt 86/06**

(45) Bekanntmachung des Hinweises auf die Patenterteilung:
**25.09.91 Patentblatt 91/39**

(84) Benannte Vertragsstaaten:
**AT BE CH DE FR GB IT LI LU NL SE**

(56) Entgegenhaltungen:
**EP-A- 0 174 143**
**DE-A- 3 247 922**

**BIOLOGICAL ABSTRACTS, Band 29, 1984, Zusammenfassung Nr. 14526, Philadelphia, PA, US; H.M. SHEPARD et al.: "Multiple interferon-alpha gene families in the mammalian genome", & ANTIVIRAL RESEARCH(NETHERLANDS) 1984, vol. 4, no. SPEC. ISSUE p4**

(73) Patentinhaber: **BOEHRINGER INGELHEIM INTERNATIONAL GmbH**
**Postfach 20**
**W-6507 Ingelheim am Rhein(DE)**

(72) Erfinder: **Hauptmann, Rudolf, Dr.**
**Viktorgasse 25/8**
**A-1040 Wien(AT)**
Erfinder: **Meindl, Peter, Dr.**
**Hockegasse 63/1**
**A-1180 Wien(AT)**
Erfinder: **Dworkin-Rastl, Eva, Dr.**
**90 Morning Side Drive, Apt. 6/j**
**N.Y. New York 10027(US)**
Erfinder: **Adolf, Günther Dr.**
**Johannagasse 20/7**
**A-1050 Wien(AT)**
Erfinder: **Swetly, Peter, Dr.**
**Hietzinger Hauptstrasse 40B**
**A-1130 Wien(AT)**

EP 0 170 204 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ARCHIVES OF BIOCHEMISTRY AND BIOPHYSICS, Band 221, Nr. 1, 15. Februar 1983, Seiten 1-37, Academic Press, Inc., New York, US; S. PESTKA: "The human interferons - From protein purification and sequence to cloning and expression in bacteria: before, between, and beyond"

NUCLEIC ACIDS RESEARCH, Band 13, Nr. 13, Juli 1985, Seiten 4739-4749, IRL Press Ltd, Oxford, GB; R. HAUPTMANN et al.: "A novel class of human type I interferons"

CHEMICAL ABSTRACTS, Band 102, Nr. 25, 24. Juni 1985, Seite 174, Zusammenfassung Nr. 216181g, Columbus, Ohio, US; D.J. CAPON et al.: "Two distinct families of human and bovine interferon-alpha genes are coordinately expressed and encode functional polypeptides", & MOL. CELL. BIOL. 1985, 5(4), 768-79

Erfinder: **Pieler, Christian**
**Schwarzspanierstrasse 9/11**
**A-1090 Wien(AT)**
Erfinder: **Hauel, Norbert, Dr. Dipl.-Chem.**
**Händelstrasse 12**
**W-7950 Biberach 1(DE)**

## Beschreibung

Gegenstand der vorliegenden Erfindung sind neue Interferone vom Typ I sowie rekombinante DNA-Verfahren zur Herstellung dieser Peptide und Produkte, die bei diesen Verfahren benötigt werden, beispielsweise Gensequenzen, rekombinante DNA Moleküle, Expressionsvehikel und Organismen.

Interferon ist ein Begriff, der geprägt wurde, um eine Auswahl von Proteinen zu beschreiben, die endogen zu menschlichen Zellen sind und dadurch gekennzeichnet sind, daß sie teilweise überlappende und teilweise divergierende biologische Aktivitäten aufweisen. Diese Proteine modifizieren die körpereigene Immunantwort und man nimmt an, daß sie zu einem wirksamen Schutz gegen Viruserkrankungen beitragen. Die Interferone wurden beispielsweise in drei Klassen eingeteilt, nämlich in $\alpha$-, $\beta$- und $\gamma$-Interferon.

Von $\beta$- und $\gamma$-Interferon ist in menschlichem Organismus nur jeweils ein Subtyp bekannt (z.B. S. Ohno et al., Proc. Natl. Acad. Sci. 78, 5305-5309 (1981); Gray et al., Nature 295, 503-508 (1982); Taya et al., EMBO Journal 1/8, 953-958 (1982)). Demgegentiber werden vom $\alpha$-Interferon verschiedene Subtypen beschrieben (z.B. Phil. Trans. R. Soc. Lond. 299, 7-28 (1982)). Die reifen $\alpha$-Interferone unterscheiden sich hierbei untereinander durch maximal 23 % Divergenz und sind ca. 166 Aminosäuren lang. Bemerkenswert ist ferner ein Bericht über ein $\alpha$-Interferon, das ein überraschend hohes Molekulargewicht (26 000, bestimmt durch SDS Polyacrylamid/Gelelektophorese) aufweist (Goren, P. et al. Virology 130, 273-280 (1983)). Dieses Interferon wird IFN-$\alpha$ 26K genannt. Von ihm wurde festgestellt, daß es die bisher höchste spezifische antivirale und antizelluläre Aktivität aufweist.

Die bekannten Interferone scheinen bei verschiedenen Krankheiten wirksam zu sein, zeigen aber eine geringe oder überhaupt keine Wirkung bei vielen anderen Krankheiten (z.B. Powledge, Bio/Technology, March 1984, 215-228 "Interferon On Trial"). Interferone weisen zudem Nebenwirkungen auf. Beispielsweise wurde bei Prüfungen der Anticancer-Wirkung von rekombinantem $\alpha$-Interferon festgestellt, daß Dosen von etwa 50 Millionen Einheiten, die nach den Ergebnissen der Phase I Prüfungen für sicher galten, akute Verwirrungszustände, bis zur Arbeitsunfähigkeit führende Gelenkschmerzen, sehr starke Ermüdung, Appetitlosigkeit, Verlust der Orientierungsmöglichkeit, epileptische Anfälle und Lebertoxizität hervorriefen. 1982 verbot die französische Regierung Prüfungen mit IFN-$\alpha$, nachdem Krebspatienten, die damit behandelt worden waren, tödliche Herzanfälle erlitten. Über mindestens zwei cardiale Todesfälle wurde auch bei kürzlich durchgeführten Prüfungen in Amerika berichtet. Es ist zunehmend deutlich geworden, daß zumindest ein Teil der Nebenwirkungen, wie Fieber und Unpäßlichkeit, in unmittelbarem Zusammenhang mit dem Interferonmolekül selbst steht und nicht auf Verunreinigungen zurückzuführen sind.

Aufgrund der großen Hoffnungen, die durch Interferon geweckt worden waren, und angeregt durch den Wunsch, neue Interferon-ähnliche Moleküle mit verminderten Nebenwirkungen zu finden, war es Aufgabe der vorliegenden Erfindung solche neuen Substanzen aufzufinden und herzustellen.

Die vorliegende Erfindung betrifft daher bestimmte neue Interferone vom Typ I, welche ein Leader-Peptid enthalten können, und ihre N-glykosylierten Derivate (hier als omega-Interferon oder IFN-omega bezeichnet), die 168 bis 174, vorzugsweise 172 Aminosäuren enthalten, und die eine Divergenz von 30 bis 50 %, vorzugsweise von 40-48 %, gegenüber den bisher bekannten Subtypen des $\alpha$-Interferons und eine Divergenz von ungefähr 70% gegenüber $\beta$-Interferon aufweisen und einerseits ähnliche Wirkungen wie $\alpha$-Interferone zeigen, andererseits viele therapeutische Nachteile dieser bekannten Substanzen nicht besitzen.

Gegenstand der Erfindung sind somit neue Interferone in vollständig reiner Form, ihre nicht glykosylierten und glykosylierten Formen, die hierfür codierenden Gensequenzen ebenso wie rekombinante Moleküle, die diese Sequenzen enthalten. Gegenstand der Erfindung sind weiterhin Expressions-Vehikel wie Plasmide, die besagte Gensequenzen enthalten, ebenso verschiedene Wirtsorganismen wie Mikroorganismen oder Gewebskulturen, die die Herstellung des neuen Interferons durch Fermentation oder Gewebskulturenmethode ermöglichen.

Ein bevorzugter Gegenstand der Erfindung sind omega-Interferone ebenso wie die entsprechenden Gensequenzen der nachstehenden Formel:

```
                        5                       10                      15
Cys Asp Leu Pro Gln Asn His Gly Leu Leu Ser Arg Asn Thr Leu
TGT GAT CTG CCT CAG AAC CAT GGC CTA CTT AGC AGG AAC ACC TTG

                        20                      25                      30
Val Leu Leu His Gln Met Arg Arg Ile Ser Pro Phe Leu Cys Leu
GTG CTT CTG CAC CAA ATG AGG AGA ATC TCC CCT TTC TTG TGT CTC

                        35                      40                      45
Lys Asp Arg Arg Asp Phe Arg Phe Pro Gln Glu Met Val Lys Gly
AAG GAC AGA AGA GAC TTC AGG TTC CCC CAG GAG ATG GTA AAA GGG

                        50                      55                      60
Ser Gln Leu Gln Lys Ala His Val Met Ser Val Leu His Glu Met
AGC CAG TTG CAG AAG GCC CAT GTC ATG TCT GTC CTC CAT GAG ATG

                        65                      70                      75
Leu Gln Gln Ile Phe Ser Leu Phe His Thr Glu Arg Ser Ser Ala
CTG CAG CAG ATC TTC AGC CTC TTC CAC ACA GAG CGC TCC TCT GCT

                        80                      85                      90
Ala Trp Asn Met Thr Leu Leu Asp Gln Leu His Thr Gly Leu His
GCC TGG AAC ATG ACC CTC CTA GAC CAA CTC CAC ACT GGA CTT CAT

                        95                      100                     105
Gln Gln Leu Gln His Leu Glu Thr Cys Leu Leu Gln Val Val Gly
CAG CAA CTG CAA CAC CTG GAG ACC TGC TTG CTG CAG GTA GTG GGA

                        110                     115                     120
Glu Gly Glu Ser Ala Gly Ala Ile Ser Ser Pro Ala Leu Thr Leu
GAA GGA GAA TCT GCT GGG GCA ATT AGC AGC CCT GCA CTG ACC TTG

                        125                     130                     135
Arg Arg Tyr Phe Gln Gly Ile Arg Val Tyr Leu Lys Glu Lys Lys
AGG AGG TAC TTC CAG GGA ATC CGT GTC TAC CTG AAA GAG AAG AAA

                        140                     145                     150
Tyr Ser Asp Cys Ala Trp Glu Val Val Arg Met Glu Ile Met Lys
TAC AGC GAC TGT GCC TGG GAA GTT GTC AGA ATG GAA ATC ATG AAA

                        155                     160                     165
Ser Leu Phe Leu Ser Thr Asn Met Gln Glu Arg Leu Arg Ser Lys
TCC TTG TTC TTA TCA ACA AAC ATG CAA GAA AGA CTG AGA AGT AAA

                        170
Asp Arg Asp Leu Gly Ser Ser
GAT AGA GAC CTG GGC TCA TCT
```

In Position 111 kann die Sequenz GGG (codierend für Gly) durch GAG (codierend für Glu) ersetzt werden. Die bevorzugten Moleküle beinhalten ebenfalls Derivate, die an der Aminosäureposition 78 N-glykosyliert sind.

Beispielsweise können die beiden vorstehend erwähnten omega-Interferone das Leader-Peptid der Formel
Met Ala Leu Leu Phe Pro Leu Leu Ala Ala Leu Val Met Thr Ser Tyr Ser Pro Val Gly Ser Leu Gly
enthalten.

Legende zu den Zeichnungen:

| | |
|---|---|
| Abbildung 1: | Restriktionskarte des Klons E76E9 |
| Abbildung 2: | Restriktionskarte des Klons P9A2 |
| Abbildung 3: | DNA-Sequenz des Klons P9A2 |
| Abbildung 4: | DNA-Sequenz des Klons E76E9 |
| Abbildung 5: | Genomische Southern Blot Analyse unter Verwendung der DNA des Klons P9A2 als Probe |
| Abbildung 6: | Konstruktion des Expressionsklons pRHW12 |
| Abbildung 7: | Aminosäure- und Nukleotiddifferenzen zwischen Typ I-Interferonen |
| Abbildung 8a: | Auflistung singulärer Nukleotidpositionen des IFN-αA Gens |
| Abbildung 8b: | Auflistung singulärer Nukleotidpositionen des IFN-omegal Gens |
| Abbildung 8c: | Auflistung singulärer Nukleotidpositionen des IFN-αD Gens |
| Abbildung 9: | Schematische Darstellung der Synthese Interferon-Subtyp spezifischer Proben |
| Abbildung 10: | Nachweis Interferon-Subtyp spezifischer mRNA's |
| Abbildung 11: | DNA-Sequenz des IFN-omegal Gens |
| Abbildung 12: | DNA-Sequenz des IFN-pseudo-omega2 Gens |
| Abbildung 13: | DNA-Sequenz des IFN-pseudo-omega3 Gens |
| Abbildung 14: | DNA-Sequenz des IFN-pseudo-omega4 Gens |
| Abbildung 15: | Korrigierte Auflistung der IFN-omega Gen Sequenzen |
| Abbildung 16: | Homologien der Signalsequenzen |
| Abbildung 17: | Homologien der "reifen" Proteinsequenzen |
| Abbildung 18: | Homologien der 4 DNA-Sequenzen zueinander |

Erfindungsgemäß erhält man die neuen omega-Interferone und die hierfür codierenden DNA-Sequenzen wie folgt:
Eine humane B-Lymphom-Zellinie, z.B. Namalwa-Zellen (siehe G. Klein et al., Int. J. Cancer 10, 44 (1972)), kann durch Behandlung mit einem Virus, z.B. Sendai Virus, zur gleichzeitigen Produktion von α- und β-Interferon angeregt werden. Wird hierbei die aus stimulierten Namalwa-Zellen gebildete mRNA isoliert, so kann diese als Template zur cDNA-Synthese dienen. Um die Ausbeute an Interferon-spezifischen Sequenzen beim Kloniervorgang zu erhöhen, wird die mRNA-Präparation über einen Zuckerdichtegradienten entsprechend der verschiedenen Länge der individuellen mRNA-Moleküle aufgetrennt. Vorteilhafterweise wird die mRNA in dem Bereich um 12S (ungefähr 800 bis 1000 Basen Länge der mRNA) gesammelt. In diesem Bereich sedimentiert die für α- und β-Interferone spezifische mRNA. Die mRNA aus diesem Gradientenbereich wird durch Präzipitation und Auflösen in Wasser konzentriert.

Die Erstellung einer cDNA Bibliothek folgt im wesentlichen literaturbekannten Methoden (siehe z.B. E. Dworkin-Rastl, M.B. Dworkin und P. Swetly, Journal of Interferon Research 2/4, 575-585 (1982)). Die mRNA wird durch Zusatz von oligo-dT geprimt. Anschließend wird durch Zusatz der vier Desoxynukleosidtriphosphate (dATP, dGTP, dCTP, dTTP) und dem Enzym Reverse Transkriptase in einer entsprechend gepufferten Lösung die cDNA 1 Stunde bei 45 °C synthetisiert. Durch Chloroformextraktion und Chromatographie über eine Gelsäule, z.B. über Sephadex® G50, wird das cDNA/mRNA-Hybrid gereinigt. Die RNA wird durch Alkalibehandlung (0,3 Mol NaOH bei 50 °C, 1 Stunde) hydrolysiert, und die cDNA nach Neutralisieren mit saurer Natriumazetatlösung mit Äthanol präzipitiert. Nach Zusatz der vier Desoxynukleosidtriphosphate und E.coli DNA-Polymerase I in entsprechender Lösung wird die Doppelstrangsynthese ausgeführt, wobei die cDNA zugleich als Template und als Primer durch Haarnadelstrukturbildung an ihrem 3'-Ende fungiert (6 Stunden bei 15 °C) (siehe A. Eftratiadis et al., Cell 7, 279 (1976)). Nach Phenolextraktion, Sephadex G50 Chromatographie und Äthanolpräzipitation wird die DNA in geeigneter Lösung einer Behandlung mit der Einzelstrangspezifischen Endonuklease SI unterzogen. Dabei werden die Haarnadelstruktur, sowie nicht in Doppelstrang umgesetzte cDNA abgebaut. Nach Chloroformextraktion und Präzipitation mit Äthanol wird die Doppelstrang-DNA (dsDNA) auf einem Zuckerdichtegradienten der Größe nach aufgetrennt. Für die folgenden Schritte des Klonierens wird bevorzugt nur dsDNA der Größe 600 bp und mehr verwendet, um die Wahrscheinlichkeit zu steigern, daß nur Klone erhalten werden, welche die komplette codierende Sequenz für die neuen Interferone enthalten. dsDNA von mehr als 600 bp Länge wird aus dem Gradienten durch Präzipitation mit Äthanol und Auflösen in Wasser konzentriert.

Um die entstandenen dsDNA-Moleküle zu vermehren, müssen diese zunächst in einem entsprechenden Vektor und anschließend in das Bakterium E. coli eingebracht werden. Der verwendete Vektor ist vorzugsweise das Plasmid pBR322 (F. Bolivar et al., Gene 2, 95 (1977)). Dieses Plasmid besteht im wesentlichen aus einem Replikon und zwei Selektionsmarkern. Diese verleihen dem Wirt Resistenz gegen die Antibiotika Ampicillin und Tetracyclin ($Ap^r$, $Tc^r$). Das Gen für die $\beta$-Lactamase ($Ap^r$) enthält die Erkennungssequenz für die Restriktionsendonuklease PstI. pBR322 kann mit PstI geschnitten werden. Die überhängenden 3'-Enden werden mit dem Enzym terminale Desoxynukleotid-Transferase (TdT) unter Vorlage von dGTP in entsprechend gepufferter Lösung verlängert. Gleichzeitig wird die dsDNA ebenfalls mit dem Enzym TdT unter Verwendung von dCTP an den 3'-Enden verlängert. Die Homopolymerenden von Plasmid und dsDNA sind komplementär und hybridisieren, wenn Plasmid DNA und dsDNA im entsprechenden Konzentrationsverhältnis und unter geeigneten Salz-, Puffer- und Temperaturbedingungen gemischt werden (T. Nelson et al., Methods in Enzymology 68, 41-50 (1980)).

E. coli vom Stamm HB 101 (Genotyp F-, hsdS20 (r-B, m-B) recA13, ara-14, proA2, lacY1, galK2, rpsL20 (Smr), xyl-5, mtl-1, supE44, lambda-) wird zur Aufnahme der rekombinanten Vektor-dsDNA-Moleküle durch Waschen mit einer $CaCl_2$-Lösung vorbereitet. Kompetente E.coli HB 101 werden mit der DNA gemischt, und nach Inkubation bei 0 °C wird die so erhaltene Plasmid-DNA durch Hitzeschock bei 42 °C für 2 Minuten transformiert (M. Dagert et al., Gene 6, 23-28 (1979)). Anschließend werden die transformierten Bakterien auf Tetracyclin-haltigen Agarplatten (10 µg pro ml) plattiert. Auf diesem Agar können nur E.coli HB 101 wachsen, die ein Vektor- oder rekombinantes Vektormolekül aufgenommen haben ($Tc^r$). Rekombinante Vektor-dsDNA Moleküle vermitteln dem Wirt den Genotyp $Ap^sTc^r$, da durch das Einbringen der dsDNA in das $\beta$-Lactamasegen die Information für die $\beta$-Lactamase zerstört worden ist. Die Klone werden auf Agarplatten übertragen, die 50 µg/ml Ampicillin enthalten. Nur etwa 3 % wuchsen an, was bedeutet, daß 97 % der Klone die Insertion eines dsDNA Moleküls enthielten. Ausgehend von 0,5 µg dsDNA wurden mehr als 30000 Klone erhalten. 28600 Klone davon wurden individuell in die Näpfe von Mikrotiterplatten übertragen, die Nährmedium, 10 µg/ml Tetracyclin und Glyzerin enthielten. Nachdem die Klone darin angewachsen waren, werden die Platten zur Aufbewahrung bei -70 °C gehalten (cDNA-Bibliothek).

Zum Durchsuchen der cDNA-Bibliothek auf neue Interferon-Gene enthaltende Klone werden die Klone nach dem Auftauen auf Nitrozellulosefilter übertragen. Diese Filter liegen auf Tetracyclin-haltigem Nähragar. Nach dem Anwachsen der Bakterienkolonien wird die DNA der Bakterien auf dem Filter fixiert.

Als Probe dient vorteilhafterweise das Insert des Klons pER33 (E. Rastl et al., Gene 21, 231-248 (1983) - siehe auch EP-A-0.115.613), das das Gen für IFN-$\alpha$2-Arg enthält. Durch Nicktranslation wird dieses Stück DNA radioaktiv markiert, wobei DNA-Polymerase I, dATP, dGTP, dTTP und $\alpha$-$^{32}$P-dCTP verwendet wird. Die Nitrozellulosefilter werden unter geeigneten, nicht stringenten Hybridisierbedingungen ohne Zugabe der radioaktiven Probe zunächst vorbehandelt und danach ca. 16 Stunden unter Zusatz der radioaktiven Probe hybridisiert. Danach werden die Filter ebenfalls unter nicht stringenten Bedingungen gewaschen. Durch die niedrige Stringenz der Hybridisierung und Waschung werden nicht nur Interferon-$\alpha$2-Arg-haltige Kloneerfaßt, sondern auch andere Interferon-haltige Klone, deren Sequenzen beträchtlich von der der bisher bekannten Interferon-$\alpha$ abweichen können. Nach dem Trocknen werden die Filter auf einen Röntgenfilm exponiert. Eine Schwärzung, die deutlich über dem Niveau des Hintergrundes liegt, zeigt das Vorhandensein eines Klons mit Interferon-spezifischen Sequenzen an.

Da die Radioaktivitätssignale unterschiedlicher Güte sind, werden die positiven Klone bzw. die positivverdächtig reagierenden Klone im Kleinmaßstab angezüchtet. Die Plasmid-DNA-Moleküle werden isoliert, mit der Restriktionsendonuklease PstI verdaut und auf einem Agarosegel elekrophoretisch der Größe nach aufgetrennt (Birnboim et al., Nucl. Acid. Res. 7, 1513 (1979)). Die DNA im Agarosegel wird nach der Methode von Southern (E.M. Southern, J. Mol. Biol. 98, 503-517 (1975)) auf ein Nitrozellulosefilter übertragen. Die DNA dieses Filters wird mit der radioaktiven, IFN-Gen-haltigen, denaturierten Probe hybridisiert. Als positive Kontrolle dient das Plasmid 1F7 (hinterlegt bei der DSM unter der DSM-Nummer 2362), das das Gen für Interferon-$\alpha$2-Arg enthält. Das Autoradiogramm macht deutlich, daß der Klon E76E9 und der Klon P9A2 eine Sequenz enthalten, welche unter nicht stringenten Bedingungen mit dem Gen des Interferons-$\alpha$2-Arg hybridisiert. Um die dsDNA Inserts der Klone E76E9 und P9A2 näher zu charakterisieren, werden die Plasmide dieser Klone in größerem Maßstab hergestellt. Die DNA wird mit verschiedenen Restriktionsendonukleasen verdaut, so z.B. mit AluI, Sau3A, BglII, HinfI, PstI und HaeIII. Die entstehenden Fragmente werden in einem Agarosegel aufgetrennt. Durch Vergleich mit entsprechenden Größenmarkern, so z.B. den Fragmenten, die durch Verdauung von pBR322 mit der Restriktionsendonuklease HinfI bzw.

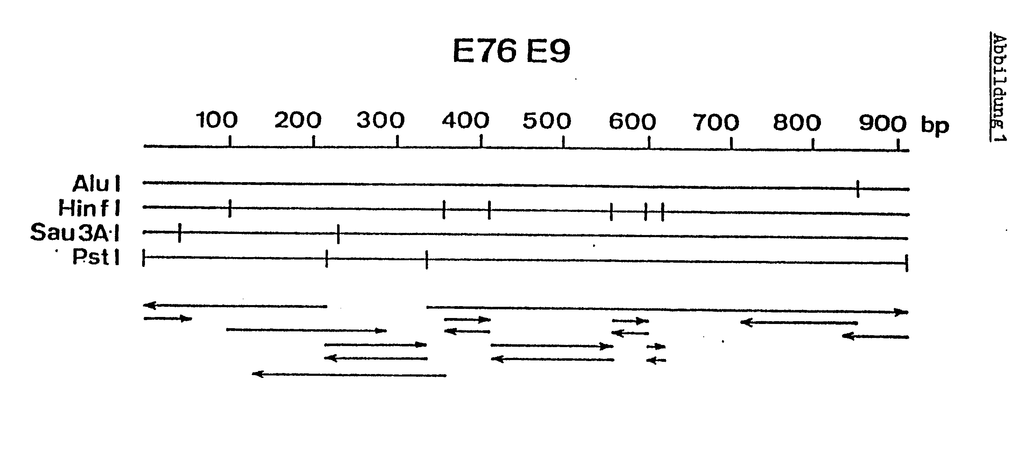

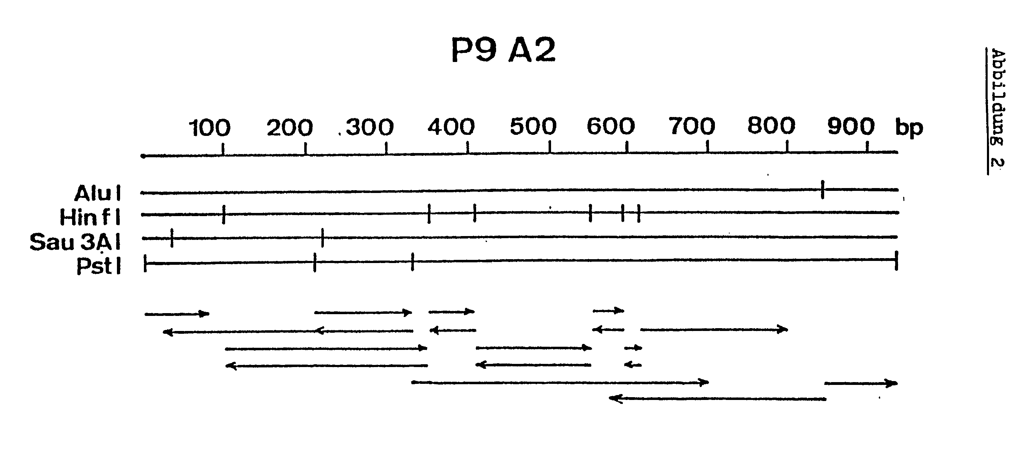

HaeIII entstehen, werden die Größen der Fragmente bestimmt. Durch Kartieren nach Smith und Birnstiel (H.O. Smith et al. Nucl. Acid. Res. 3, 2387-2398 (1967)) wird die Reihenfolge dieser Fragmente bestimmt. Aus den so ermittelten Restriktionsenzymkarten (Abbildung 1 und 2) läßt sich überraschenderweise ableiten, daß es sich bei den Inserts der Klone E76E9 und P9A2 um bisher unbekannte Interferongene, nämlich um jene der omega-Interferone, handelt.

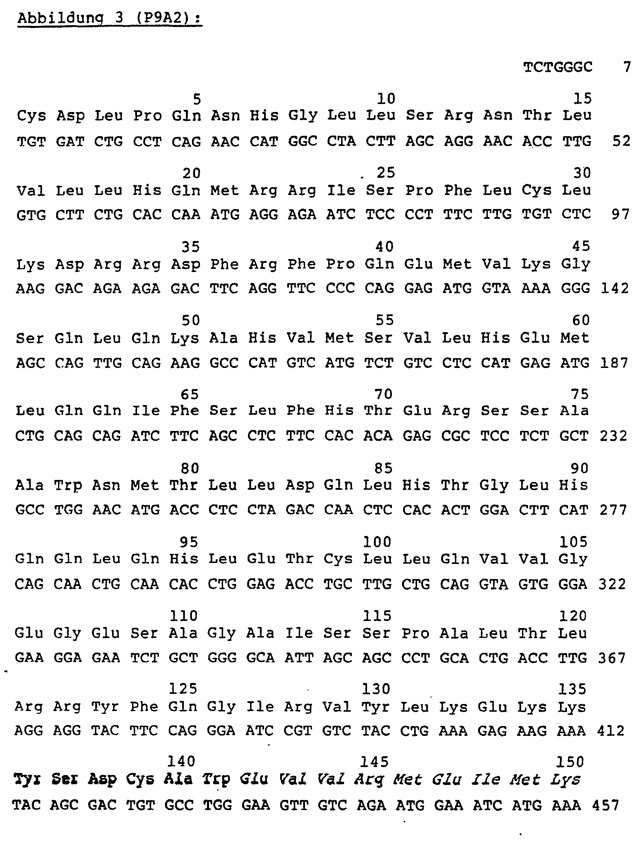

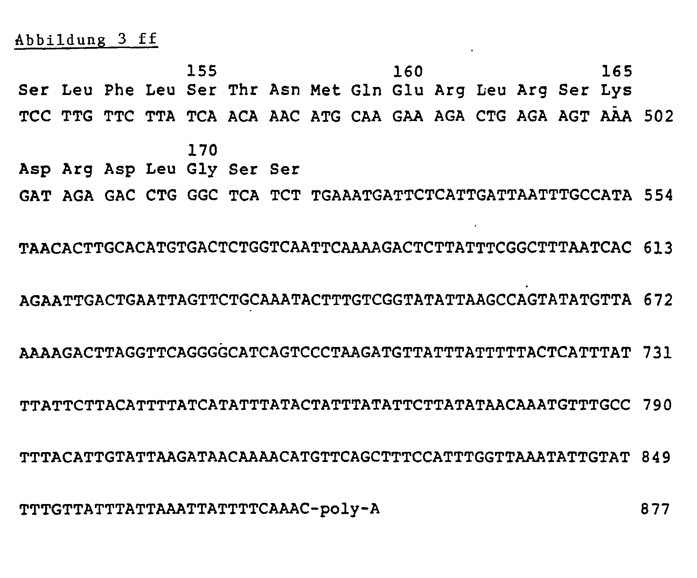

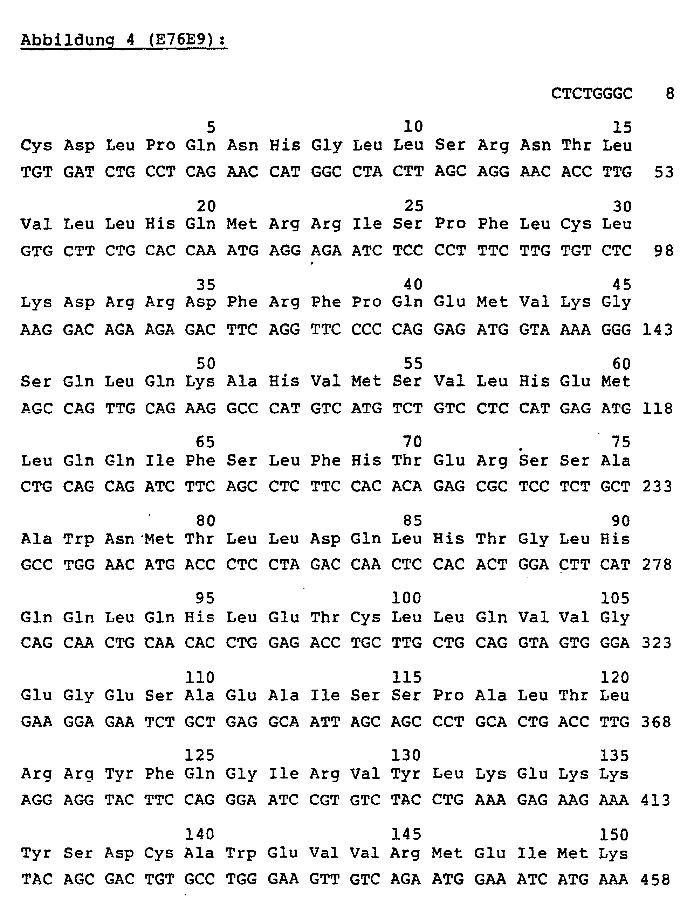

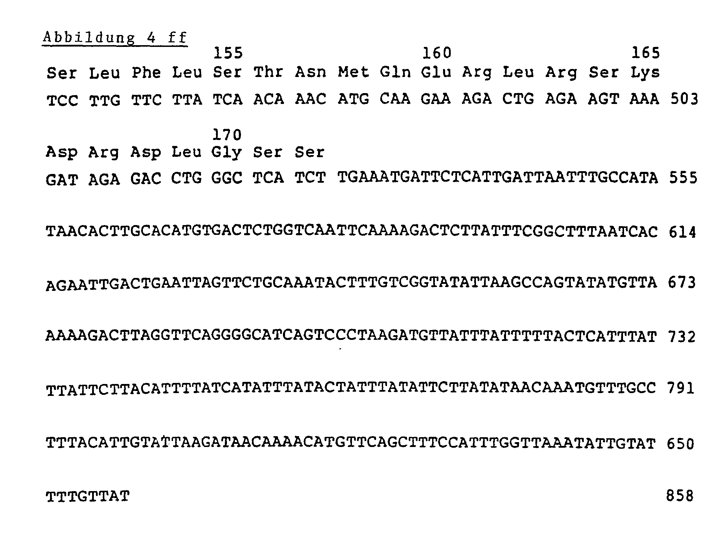

Diese Information über die omega-Interferone wird benutzt, um das cDNA-Insert mit geeigneten Restriktionsendonukleasen zu verdauen. Die entstehenden Fragmente werden in die dsDNA-Form (replikative Form) des Bakteriophagen M13 mp9 ligiert (J. Messing et al., Gene 19, 269-276 (1982)) und mit der Sanger'schen Dideoxymethode sequenziert (F. Sanger et al., Proc. Natl. Acad. Sci. 74, 5463-5467 (1977)). Die Einzelstrang-DNA der rekombinanten Phagen wird isoliert. Nach dem Binden eines synthetischen Oligomers werden in vier separaten Ansätzen Zweitstrangsynthesen unter Verwendung des großen Fragments der DNA-Polymerase I aus E. coli (Klenow-Fragment) durchgeführt. Zu jeder der vier Teilreaktionen wird eines der vier Didesoxynukleosidtriphosphate (ddATP, ddGTP, ddCTP, ddTTP) zugegeben. Das führt zu statistisch verteilten Kettenabbrüchen an jenen Stellen, an denen sich in der Template-DNA die zu dem jeweiligen Didesoxynukleosidtriphosphat komplementäre Base befindet. Außerdem wird zusätzlich radioaktiv markiertes dATP verwendet. Nach Beendigung der Synthesereaktionen werden die Produkte denaturiert, und die Einzelstrang-DNA-Fragmente der Größe nach in einem denaturierenden Polyacrylamidgel getrennt (F. Sanger et al., FEBS Letters 87, 107-111 (1978)). Danach wird das Gel auf einen Röntgenfilm exponiert. Aus dem Autoradiogramm läßt sich die DNA-Sequenz des rekombinanten M13 Phagen ablesen. Die Sequenzen der Inserts der verschiedenen rekombinanten Phagen werden mittels geeigneter Computerprogramme verarbeitet (R. Staden, Nucl. Acid. Res. 10, 4731-4751 (1982)). Abbildung 1 und 2 zeigen die Strategie des Sequenzierens. Abbildung 3 zeigt die DNA-Sequenz des Inserts des Klons P9A2, Abbildung 4 die des Klons E76E9. Der nicht kodierende DNA-Strang ist in 5'-> 3' Richtung gezeigt, zusammen mit der daraus abzuleitenden Aminosäuresequenz.

Die isolierte cDNA des Klons E76E9 für omega(Glu)-Interferon ist 858 Basenpaare lang und besitzt eine 3' nicht translatierte Region. Die Region, die für reifes omega(Glu)-Interferon kodiert, reicht von dem Nukleotid 9 bis zum Nukleotid 524. Die isolierte cDNA des Klons P9A2 für omega(Gly)-Interferon ist 877 Basenpaare lang, wobei die für reifes omega-Interferon kodierende Sequenz von dem Nukleotid 8 bis zum Nukleotid 523 reicht. Die 3' nicht translatierte Region reicht im Fall des P9A2 bis zum poly-A-Abschnitt.

Die für reifes omega-Interferon kodierenden DNA-Sequenzen sind in den Klonen E76E9 und P9A2 komplett enthalten. Sie beginnen am N-terminalen Ende mit den Aminosäuren Cystein-Asparaginsäure-Leucin. Überraschenderweise sind die beiden reifen omega-Interferone 172 Aminosäuren lang, was deutlich von der Länge von den bisher bekannten Interferonen, z.B. 166 (bzw. 165) Aminosäuren bei $\alpha$-Interferonen abweicht. Überraschenderweise besitzen die beiden omega-Interferone eine potentielle N-Glykosylierungsstelle an der Aminosäureposition 78 (Asparagin-Methionin-Threonin).

Ein Vergleich der DNA der Klone E76E9 und P9A2 zeigt einen Unterschied. Das für die Aminosäure 111 kodierende Triplett im Klon E76E9 ist GAG und kodiert für Glutaminsäure, während dieses Triplett im Klon P9A2 GGG ist, und für Glycin kodiert. Die beiden omega-Interferon-Proteine unterscheiden sich daher in einer Aminosäure und werden mit omega(Glu)-Interferon (E76E9) und omega(Gly)-Interferon (P9A2) bezeichnet.

Der Vergleich der beiden omega-Interferone mit den bisher bekannten menschlichen $\alpha$-Interferon Subtypen ergibt folgendes Bild:

| | omega | alpha |
|---|---|---|
| Länge des Proteins in Aminosäuren | 172 | 166 (+) |
| potentielle N-Glykosylierungsssstelle an Position | 78 | - (++) |

+) Interferon alpha A besitzt nur 165 Aminosäuren

++) Interferon alpha H besitzt eine potentielle N-Glykosylierungsstelle an Position 75 (D. Goeddel et al., Nature 290, 20-26 (1981)).

E. coli HB 101 mit dem Plasmid E76E9 und E. coli 101 mit dem Plasmid P9A2 sind bei der Deutschen Sammlung für Mikroorganismen (DSM Göttigen) unter der Nummer DSM 3003 respektive DSM 3004 am 3. Juli 1984 hinterlegt worden.

Zum Beleg, daß die neu aufgefundenen Klone eine Interferon-ähnliche-Aktivität produzieren, wird beispielsweise der Klon E76E9 kultiviert und das Lysat des Bakteriums nach dessen Wachstum in einem Plaque-Reduktions-Test überprüft. Das Bakterium produzierte erwartungsgemäß Interferon-ähnliche Aktivität (siehe Beispiel 3).

Ferner wurde zum Beleg, daß es sich bei den beiden neuaufgefundenen Interferonen um Mitglieder einer neuen Interferonfamilie handelt, die gesamte DNA aus Namalwa-Zellen isoliert und mit verschiedenen Restrictionsendonucleasen verdaut. Hierdurch kann die Anzahl der Gene, welche durch die cDNA's der Klone P9A2 und E76E9 codiert werden, abgeschätzt werden. Hierzu werden die erhaltenen DNA-Fragmente an Agarosegel nach der Methode von Southern (E.M. Southern et al., J. Mol. Biol. 98, 503-517 (1975)) aufgetrennt, auf Nitrocellulose-Filter gebracht und unter relativ strengen Bedingungen mit radioaktiv markierter spezifischer DNA des Klons P9A2 hybridisiert.

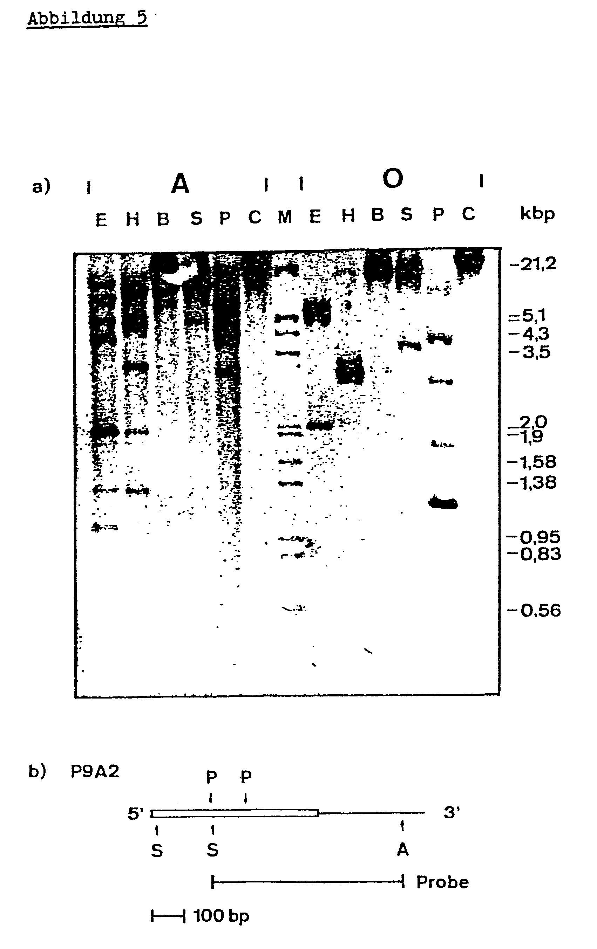

Die nach der Hybridisierung mit DNA's aus dem Plasmid P9A2 und pER33 erhaltenen Ergebnisse werden in Abbildung 5a dargestellt:

Hierbei sind die einzelnen Spuren mit Buchstaben gekennzeichnet, die die verschieden verdauten DNA-Proben anzeigen (E = EcoRI, H = HindIII, B = BamHI, S = SphI, P = PstI, C = ClaI). Die linke Hälfte des Filters wurde mit der Interferon-α-Gen-Probe ("A") und die rechte Hälfte mit dem cDNA-Insert des Klons P9A2 ("O") hybridisiert. Der Satz der Banden, die entweder mit der α-Interferon-Gen-Probe oder mit der neuen Interferon-Gen-Probe hybridisieren, ist verschieden. Bei den zwei verschiedenen Proben konnte keine Kreuzhybridisierung festgestellt werden, wenn man die entsprechenden Spuren beurteilt.

In Abbildung 5b ist die cDNA des Klons P9A2 und das für die Hybridisierung benützte Fragment abgebildet. Die Schnittstellen einiger Restrictions-Enzyme sind dargestellt (P = PstI, S = Sau3A, A = AluI). Die Probe umfaßt nur zwei der möglichen drei PstI-Fragmente. Das Hybridisierungsmuster zeigt nur ein hybridisierendes Fragment mit ungefähr 1300 Basenpaaren (bp), welches zu dem homologen Gen gehört. Das kleinere 120 bp lange Fragment war vom Gel ausgelaufen. Die dem 5'-Teil des Gens zugehörige Bande kann nicht entdeckt werden, da die Probe diese Region nicht enthält. Mindestens 6 verschiedene Banden können in dieser PstI-Spur entdeckt werden. Dies bedeutet, daß einige andere Gene, die mit den neuen Sequenzen verwandt sind, in dem menschlichen Genom vorhanden sein müssen. Sollten in diesen Genen eine oder mehrere PstI-Schnittstellen vorhanden sein, so kann erwartet werden, daß noch mindestens 3 zusätzliche Gene isoliert werden können.

Diese Gene können vorzugsweise mittels Hybridisierung aus einer menschlichen Gen-Bibliothek, die in einem Plasmid-Vektor, Phagen-Vektor oder Cosmid-Vektor enthalten sind, isoliert werden (siehe Beispiel 4e).

An dieser Stelle sei erwähnt, daß es sich bei den erfindungsgemäßen omega-Interferonen nicht nur um die zwei reifen Interferone, die im einzelnen beschrieben sind, handelt, sondern ebenfalls um jedwede Modifikation dieser Polypeptide, die die IFN-omega-Aktivität unbeeinflußt läßt. Diese Modifikationen beinhalten z.B. Verkürzung des Moleküls am N- oder C-terminalen Ende, Austausch von Aminosäuren durch andere Reste, wodurch die Aktivität nicht wesentlich verändert wird, chemische oder biochemische Bindungen des Moleküls an andere Moleküle, die inert oder aktiv sind. Bei den letztgenannten Modifikationen kann es sich beispielsweise um Hybridmoleküle aus einem oder mehreren omega-Interferonen und/oder bekannten $\alpha$- oder $\beta$-Interferonen handeln.

Um die Unterschiede der Aminosäure- und Nukleotidsequenzen der neuen Interferone, insbesondere des omega(Gly)- und des omega(Glu)-Interferons, im Vergleich zu den bereits für die $\alpha$-Interferone und für das $\beta$-Interferon publizierten Aminosäure- und Nukleotidsequenzen (C. Weissmann et al., Phil. Trans. R. Soc. London 299, 7-28 (1982); A. Ullrich et al., J. Molec. Biol. 156, 467-486 (1982);, T.Taniguchi et al., Proc. Nat. Acad. Sci. 77, 4003-4006 (1980); K. Tokodoro et al., EMBO J. 3, 669-670 (1984)) vergleichen zu können, werden die entsprechenden Sequenzen paarweise angeordnet und die Unterschiede an einzelnen Positionen gezählt.

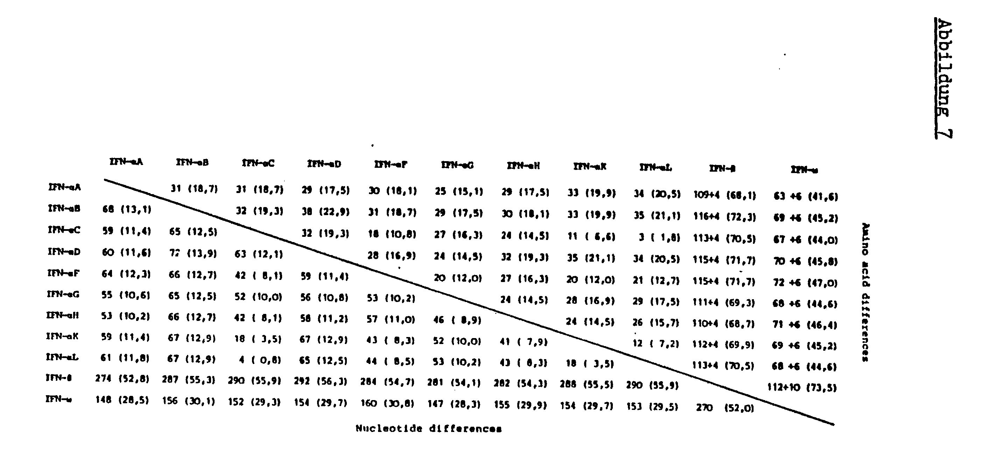

Aus den in der Abbildung 7 dargestellten Ergebnissen geht hervor, daß die DNA-Sequenzen der Klone P9A2 und E76E9 verwandt sind mit den Sequenzen der Typ I Interferone (z.B. $\alpha$- und $\beta$-Interferone). Andererseits wird dargestellt, daß die Unterschiede der Aminosäuresequenzen zwischen den einzelnen $\alpha$-Interferonen und den neuen Sequenzen größer als 41,6 % und kleiner als 47,0 % sind. Die Unterschiede zwischen den neuen Sequenzen sowie denen der einzelnen $\alpha$-Interferone und der des $\beta$-Interferons liegt in der Größenordnung von ungefähr 70 %. Unter Berücksichtigung der Ergebnisse des Beispiels 4, in welchem die Existenz eines ganzen Satzes verwandter Gene belegt wird, und unter Berücksichtigung der vorgeschlagenen Nomenklatur für die Interferone (J. Vilceck et al., J. Gen. Virol. 65, 669-670 (1984)) wird angenommen, daß die cDNA-Inserte der Klone P9A2 und E76E9 für eine neue Klasse der Typ I-Interferon codieren, welche als Interferon-omega bezeichnet werden.

Desweiteren wird belegt, daß die omega-Interferon-Gen-Expression analog der von einem Interferon-TypI-Gen erfolgt. Hierzu wird die Transkription der einzelnen Mitglieder der Multigenfamilien der $\alpha$- und omega-Interferone basierend auf der SI-Mapping-Methode (A.J. Berk et al., Cell 12, 721 (1977)) untersucht (siehe Beispiel 7) und gezeigt, daß die Expression omega-1-mRNA virusinduzierbar ist. Da sich die Transkripte einer solchen Genfamilie nur durch einige wenige Basen von ungefähr 1000 unterscheiden, ist die Hybridisierung allein kein genug empfindliches Unterscheidungsmerkmal, um die verschiedenen IFN mRNA's zu unterscheiden.

Hierzu werden die mRNA-Sequenzen von 9 $\alpha$-Interferonen, von Interferon-omega-1 und von $\beta$-Interferon dargestellt. Mit Großbuchstaben sind hierbei diejenigen Basen gekennzeichnet, welche für die oberste Sequenz spezifisch sind. Solche spezifischen Stellen können leicht durch ein triviales Computer-Programm gefunden werden. Eine Hybridisierungsprobe, welche von einer spezifischen Stelle ausgeht, kann nur mit der mRNA eines bestimmten Subtyps perfekt hybridisieren. Alle übrigen mRNA's können nicht an der spezifischen Stelle des Subtyps hybridisieren. Wenn die Hybridisierungsprobe an ihrem spezifischen 5'-Ende radioaktiv markiert ist, sind nur jene radioaktiven Markierungen gegen Verdauungen mit einer Einzelstrang-spezifischen Nuclease (vorzugsweise SI-Nuclease) geschützt, welche mit der Interferon-Subtyp-mRNA, für welche die Probe konstruiert worden war, hybridisiert haben.

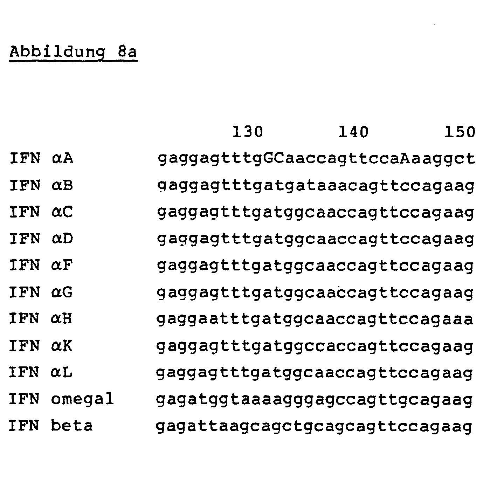

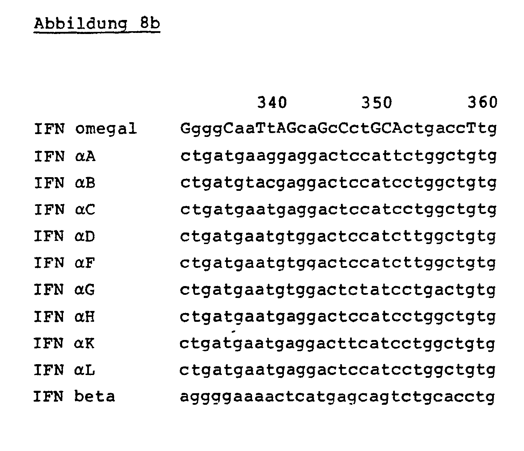

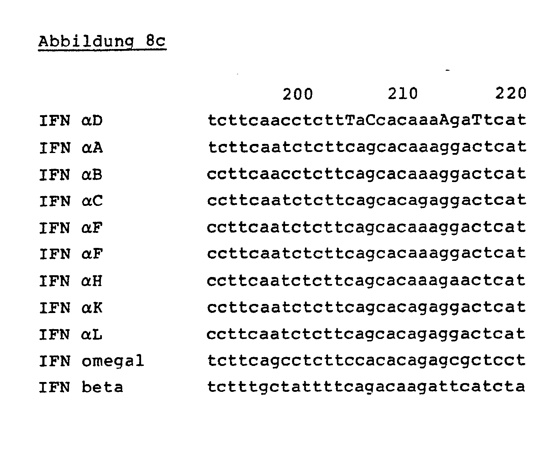

Dieses Prinzip ist nicht auf Interferon beschränkt, dieses kann vielmehr auf jede Gruppe bekannter Sequenzen, die die in Abbildung 8 beschriebenen spezifischen Stellen aufweisen, angewendet werden.

Die oben erwähnten spezifischen Stellen der Subtypen sind in den meisten Fällen keine Restriction-Stellen, so daß das Schneiden der cDNA's mit Restrictions-Endonucleasen nicht dazu geeignet ist, Subtypspezifische Hybridisierungsproben herzustellen. Die in diesem Beispiel benutzten Proben sind daher durch Erweiterung eines am 5'-Ende radioaktiv markierten Oligonucleotids, welches complementär zu der mRNA von Interferon-omegal oberhalb der spezifischen Stelle ist, hergestellt worden.

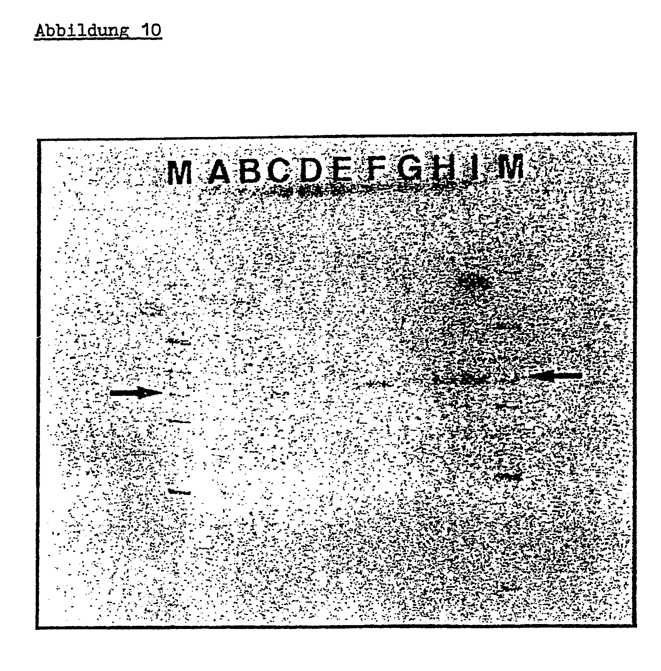

Aus Abbildung 10 geht hervor, daß erwartungsgemäß omega-1-mRNA in Namalwa- und NC37-Zellen induzierbar ist.

Gegenstand der Erfindung sind daher nicht nur Gen-Sequenzen, die spezifisch für die angegebenen omega-Interferone codieren, sondern ebenfalls Modifikationen, die leicht und routinemäßig durch Mutation, Abbau, Transposition oder Addition erhalten werden können. Jede Sequenz, die für die beschriebenen humanen omega-Interferone codiert (d.h. die das biologische Aktivitätsspektrum aufweist, das hier beschrieben ist) und im Vergleich mit den gezeigten degeneriert ist, ist ebenfalls eingeschlossen; Fachleute auf diesem Gebiet sind in der Lage, DNA-Sequenzen der codierenden Regionen zu degenerieren. Ebenso ist jede Sequenz, die für ein Polypeptid mit dem Aktivitätsspektrum des IFN-omega codiert, und die mit den gezeigten Sequenzen (oder Teilen davon) unter stringenten Bedingungen hybridisiert (beispielsweise Bedingungen, die für mehr als 85 %, bevorzugt mehr als 90 % Homologie selektieren) beinhaltet.

Die Hybridisierungen werden in 6 x SSC/5 x Denhardt's Lösung/0,1 % SDS bei 65° C durchgeführt. Der Grad der Stringenz wird im Waschschritt festgelegt. So sind für eine Selektionierung auf DNA-Sequenzen mit ca. 85 % oder mehr Homologie die Bedingungen 0,2 x SSC/0,01 %, SDS/65° C und für eine Selektionierung auf DNA-Sequenzen mit ca. 90 % oder mehr Homologie die Bedingungen 0,1 x SSC/0,01 % SDS/65° C geeignet.

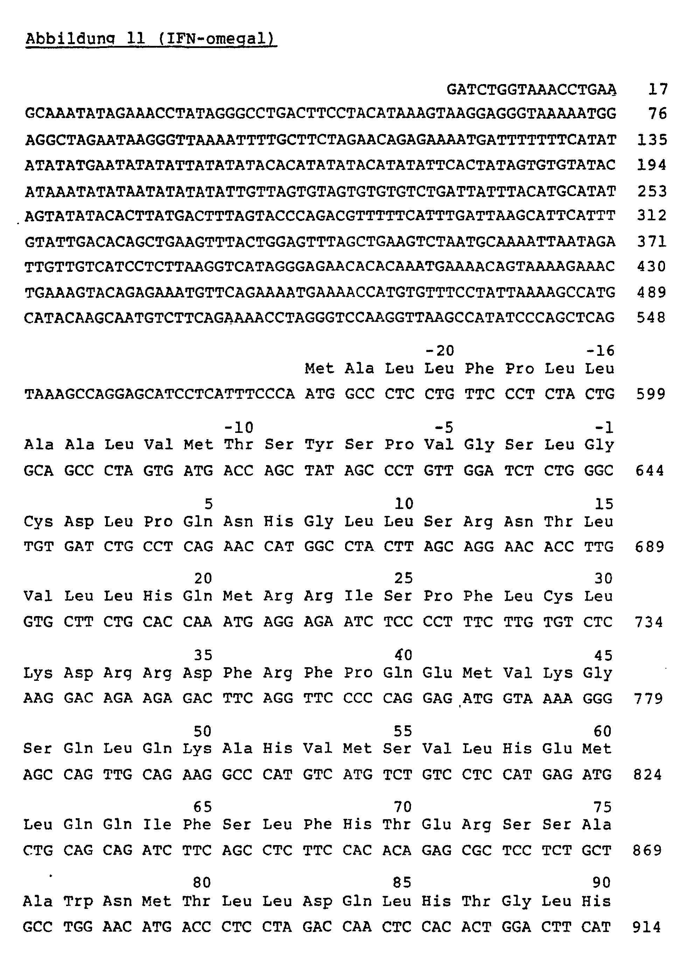

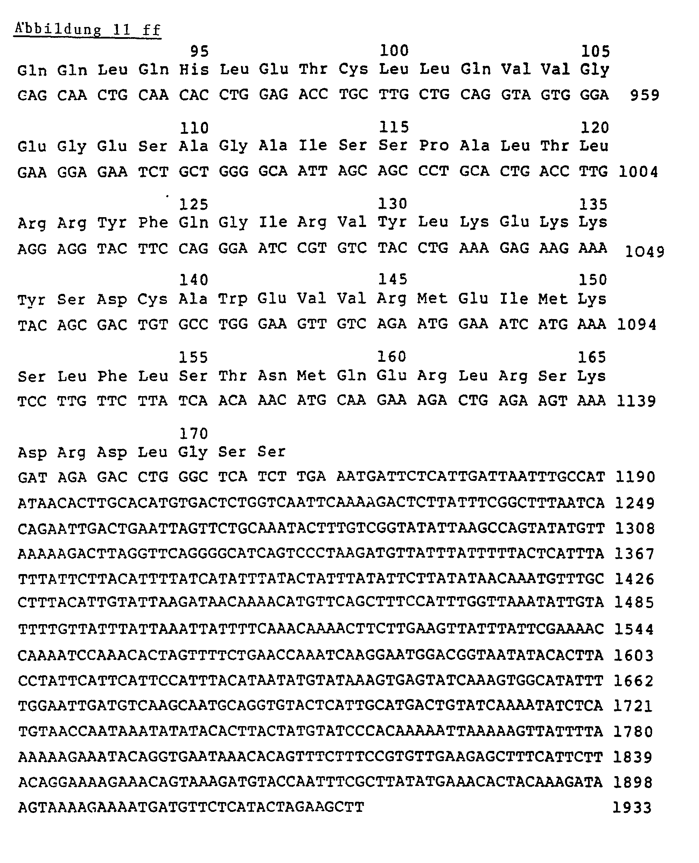

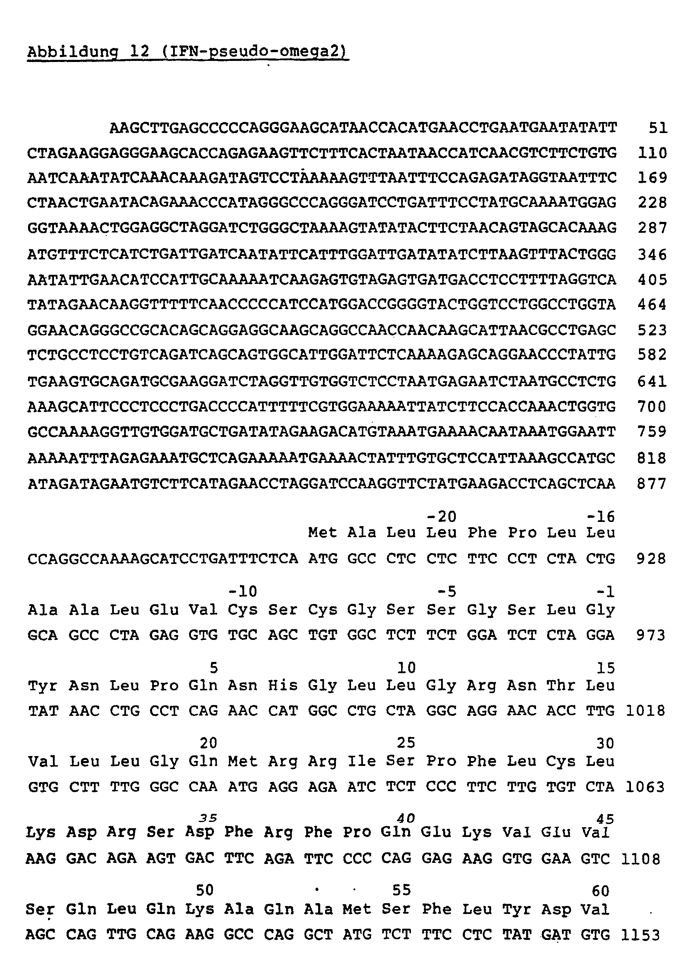

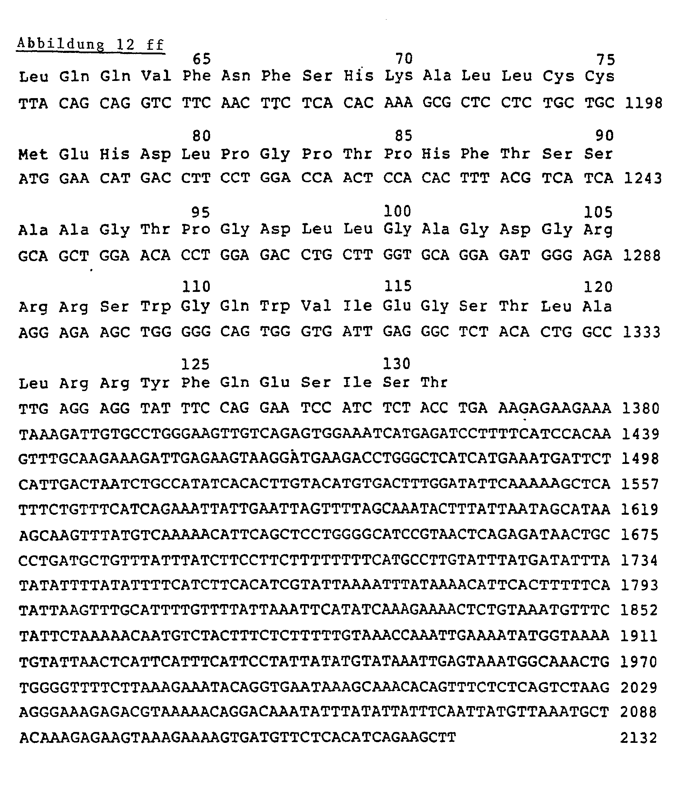

Beim Durchsuchen einer Cosmid-Bibliothek unter diesen Bedingungen (0.2 x SSC) erhält man einige mit einer IFN-omegal Probe hybridisierende Cosmide. Die Sequenzanalyse von daraus isolierten Restriktionsenzymfragmenten ergibt das authentische IFN-omegal Gen (siehe Abbildung 11), sowie drei weitere, dazu verwandte Gene, die als IFN-pseudo-omega2, IFN-pseudo-omega3 und IFN-pseudo-omega4 (siehe Abbildungen 12-14) bezeichnet wurden. Diese sind ein weiterer Gegenstand der vorliegenden Erfindung sowie die durch sie codierten Peptide. Dieser Gegenstand wird in den Ansprüchen 43 bis 52 gekennzeichnet.

Die DNA-Vergleiche ergeben eine rund 85%ige Homologie der Pseudogene zum IFN-omegal-Gen (Interferon-omega-eins-Gen).

Desweiteren zeigt das IFN-omegal Gen, daß bei der Transkription die mRNA die Information für ein funktionelles Interferonprotein beinhaltet, d.h., es wird ein 23 Aminosäure langes Signalpeptid der Formel Met Ala Leu Leu Phe Pro Leu Leu Ala Ala Leu Val Met Thr Ser Tyr Ser Pro Val Gly Ser Leu Gly codiert, das vom reifen 172 Aminosäuren langen IFN-omegal gefolgt wird.

Interferon-omega-Gene können unter Bedingungen in jeden Organismus eingebracht werden, die zu hohen Ausbeuten führen. Geeignete Wirte und Vektoren sind dem Fachmann bestens bekannt; als Beispiel sei auf die EP-A-0.093.619 hingewiesen.

Insbesondere sind Prokaryoten für die Expression bevorzugt. Beispielsweise ist E. coli K 12, Stamm 294 (ATCC No. 31 446) geeignet. Andere Stämme, die geeignet sind, beinhalten E. coli X1776 (ATCC Nr. 31.537). Ebenso wie die vorerwähnten stamme können auch E. coli W 3110 ($F^-$, $Lambda^-$, Prototroph, ATCC Nr. 27325), Bazillen wie Bacillus subtilis, und andere Enterobacteriaceae, wie Salmonella typhimurium oder Serratia marcensens und verschiedene Pseudomonaden verwendet werden.

Im allgemeinen können Plasmid-Vektoren, die Replikon und Kontrollsequenzen, die aus Spezies stammen, die kompatibel mit den Wirtszellen sind, enthalten, in Verbindung mit diesen Wirten verwendet werden. Der Vektor trägt üblicherweise neben einer Replikationsstelle Erkennungssequenzen, die es ermöglichen, die transformierten Zellen phenotypisch zu selektieren. Zum Beispiel wird E. coli üblicherweise mit pBR322 transformiert, ein Plasmid, das aus E. coli Spezies stammt (Bolivar, et al., Gene 2, 95 (1977)). pBR322 enthält Gene für Ampicillin- und Tetracyclin-Resistenz und liefert damit einfache Mittel, transformierte Zellen zu identifizieren. Das pBR322-Plasmid oder auch andere Plasmide müssen außerdem von sich aus Promotoren enthalten oder müssen dahingehend so modifiziert sein, daß sie Promotoren enthalten, die vom mikrobiellen Organismus zur Expression ihrer eigenen Proteine verwendet werden können. Die Promotoren, die am häufigsten bei der Herstellung rekombinanter DNA verwendet werden, beinhalten die Beta-Lactamase (Penicillinase) und Lactose-Promotor-Systeme (Chang et al., Nature 275, 615 (1978); Itakura et al., Science 198, 1056 (1977); Goeddel et al., Nature 281, 544 (1979)) und Tryptophan(trp) Promotor-Systeme (Goeddel et al., Nucleic Acids Res. 8, 4057 (1980); EP-A-0.036.776). Während die erwähnten die gebräuchlichsten Promotoren sind, sind darüber hinaus auch andere mikrobielle Promotoren entwickelt und benutzt worden. Die Gen-Sequenz für IFN-omega kann beispielsweise unter der Kontrolle des Leftward-Promotors des Bakteriophagen Lambda ($P_L$) eingesetzt werden. Dieser Promotor ist einer der als besonders stark bekannten Promotoren, der steuerbar ist. Die Steuerung wird möglich durch den Lambda-Repressor, von dem benachbarte Restriktionsschnittstellen bekannt sind.

Ein temperaturempfindliches Allel dieses Repressor-Gens kann in einem Vektor, der eine vollständige IFN-omega-Sequenz enthält, eingefügt werden. Wird die Temperatur auf 42° C erhöht, wird der Repressor inaktiviert und der Promotor bis zu seiner maximalen Konzentration exprimiert. Die Summe der mRNA, die unter diesem Bedingungen produziert wird, sollte ausreichend sein, um eine Zelle zu erhalten, die unter ihren neuen synthetischen Ribonukleinsäuren ungefähr 10 % enthält, die von dem $P_L$-Promotor stammt. Auf diese Weise ist es möglich, eine Clon-Bank zu etablieren, in der eine funktionelle IFN-omega-Sequenz in Nachbarschaft zu einer Ribosom-Bindungsstelle plaziert wird in variierenden Abständen zu dem Lambda-$P_L$-Promotor. Diese Klone können dann überprüft und der mit der höchsten Ausbeute selektiert werden.

Die Expression und Translation einer IFN-omega-Sequenz kann auch unter Kontrolle anderer Regulationssysteme, die als "homolog" zu dem Organismus in seiner untransformierten Form gelten können, ablaufen. So enthält z.B. chromosomale DNA von einem Lactose-abhängigen E. coli ein Lactose oder Lac-Operon, der durch Ausschüttung des Enzyms Beta-Galactosidase den Lactose-Abbau ermöglicht.

Die Lac-Kontrollelemente können aus dem Bacteriophagen Lambda-plac5, der infektös für E. coli ist, erhalten werden. Das Lac-Operon des Phagen kann durch Transduktion aus derselben Bakterien-Spezies stammen. Regulationssysteme, die bei dem erfindungsgemäßen Verfahren Verwendung finden können, können aus plasmidischer DNA stammen, die dem Organismus eigen ist. Das Lac-Promotor-Operator-System kann durch IPTG induziert werden.

Andere Promotor-Operator-Systeme oder Teile hiervon können genausogut verwendet werden: beispielsweise Arabinose-Operator, Colicine $E_1$-Operator, Galactose-Operator, alkalischer Phosphatase-Operator, trp-Operator, Xylose-A-Operator, tac-Promotor u.ä..

Zusätzlich zu Prokaryoten können auch eukaryotische Mikroorganismen, wie Hefekulturen verwendet werden Saccharomyces cerevisiae ist die am meisten verwendete unter den eukaryotischen Mikroorganismen, obwohl eine Anzahl anderer Spezies allgemein erhältlich ist. Zur Expression in Saccharomyces wird beispielsweise das Plasmid YRp7 (Stinchcomb et al. Natur 282, 39 (1979); Kingsman et al., Gene 7, 141 (1979); Tschumper et al., Gene 10, 157 (1980)) und das Plasmid YEp 13 (Bwach et al., Gene 8, 121-133 (1979)) üblicherweise verwendet. Das Plasmid YRp7 enthält das TRP1-Gen, das eine Selektionierungsmarkierung für eine Hefemutante, die unfähig ist, in trypthophanhaltigem Medium zu wachsen, bereitstellt; beispielsweise ATCC Nr. 44076.

Das Vorhandensein des TRP1 Schadens als Charakteristikum des Hefe-Wirts Genoms stellt dann ein wirksames Hilfsmittel dar, um die Transformation nachzuweisen, indem ohne Tryptophan kultiviert wird. Ganz ähnlich verhält es sich bei dem Plasmid YEp13, das das Hefe-Gen LEU 2, das zur Ergänzung einer LEU-2-minus-Mutante verwendet werden kann, enthält. Geeignete Promotor-Sequenzen für Hefe Vektoren beinhalten die 5'-flankierende Region der Gene des ADH I (Ammerer G., Methods of Enzymology 101, 192-201 (1983)), 3-Phosphoglycerate-Kinase (Hitzeman et al., J. Biol. Chem. 255, 2073 (1980)) oder andere glykolytische Enzyme (Kawaski und Fraenkel, BBRC 108, 1107-1112 (1982)) wie Enolase, Glyceraldehyd-3-phosphat, Dehydrogenase, Hexokinase, Pyruvat, Decarboxylase, Phosphofructokinase, Glucose-6-Phosphat-Isomerase, Phosphoglucose-Isomerase und -Glucokinase. Bei der Konzentration geeigneter Expres- sions Plasmide können die mit diesen Genen assoziierten Terminationssequenzen ebenfalls in den Expressions-Vektor am 3'-Ende der zu exprimierenden Sequenz eingesetzt werden, um Polyadenylierung und Termination der mRNA vorzusehen.

Andere Promotoren, die zudem noch den Vorteil der durch Wachstumsbedingungen kontrollierten Transkription besitzen, sind die Promotor-Regionen der Gene für Alkohol-Dehydrogenase-2, Isocytochrom 6, Saure-Phosphatase abbauende Enzyme, die mit dem Stickstoff-Metabolismus gekoppelt sind, die oben erwähnte Glyceraldehyd-3-Phosphat-Dehydrogenase und Enzyme, die für die Verarbeitung von Maltose und Galaktose verantwortlich sind. Promotoren, die durch den Hefe Mating Typ Locus reguliert werden, beispielsweise Promotoren der Gene BARI, MF$\alpha$1, STE2, STE3, STE5 können bei temperaturregulierten Systemen durch die Verwendung von temperaturabhängigen siv Mutationen eingesetzt werden. (Rhine PH.D. in Thesis, University of Oregon, Eugene, Oregon (1979), Herskowitz and Oshima, The Molecular Biology of the Yeast Saccharomyces, part I, 181-209 (1981), Cold Spring Harbor Laboratory). Diese Mutationen beeinflussen die Expression der ruhenden Mating Typ Kassetten von Hefen und dadurch indirekt die Mating Typ abhängigen Promotoren. Generell ist jedoch jeder Plasmid Vektor, der einen Hefekompatiblen Promotor, originäre Replikations- und Terminationssequenzen enthält, geeignet. Zusätzlich zu Mikroorganismen sind Kulturen multizellulärer Organismen ebenfalls geeignete Wirtsorganismen. Im Prinzip ist jede dieser Kulturen einsetzbar, ob von Wirbeltier- oder wirbellosen Tierkulturen. Größtes Interesse besteht jedoch an Wirbeltier-Zellen, so daß die Vermehrung von Wirbeltierzellen in Kultur (Gewebe-Kultur) in den letzten Jahren zu einer routinemäßigen Methode wurde (Tissue, Culture, Academic Press, Kruse and Patterson, Editors (1973)). Beispiele solcher nützlichen Wirtszellinien sind VERO- und HeLa-Zellen, Goldhamster-Eierstock (CHO)-Zellen und W138, BHK, COS-7 und MDCK-Zellinien. Expressionsvektoren für diese Zellen enthalten üblicherweise (wenn nötig) eine Replikationsstelle, einen Promotor, der vor dem zu exprimierenden Gen lokalisiert ist, gemeinsam mit jeder notwendigen Ribosomenbindungsstelle, RNA-Splicing-Stelle, Polyadenylierungsstelle und transkriptionelle Terminations-Sequenzen.

Bei der Verwendung in Säugetierzellen werden die Kontrollfunktionen auf den Expressions-Vektoren oftmals aus viralem Material vorgesehen. Beispielsweise stammen die üblicherweise verwendeten Promotoren aus Polyoma Adenovirus 2, und besonders häufig aus Simian Virus 40 (SV 40). Die frühen und späten Endpromotoren des SV 40 sind besonders nützlich, da beide leicht aus dem Virus als Fragment zu erhalten sind, das auch noch die virale Replikationsstelle des SV 40 enthält. (Fiers et al., Nature 273, 113 (1978)). Auch können kleinere oder größere Fragmente des SV 40 verwendet werden, vorausgesetzt, sie enthalten die annähernd 250 bp lange Sequenz, die von der HindIII Schnittstelle bis zur Bgl 1 Schnittstelle in der viralen Replikationsstelle reicht. Außerdem ist es ebenfalls möglich und oft empfehlenswert, Promotor- oder Kontroll-Sequenzen zu verwenden, die normalerweise mit den gewünschten Gensequenzen verknüpft sind, vorausgesetzt, diese Kontroll-Sequenzen sind kompatibel zu den Wirtszellsystemen.

Eine Replikationsstelle kann entweder durch entsprechende Vektorkonstruktion vorgesehen werden, um eine exogene Stelle einzubauen, beispielsweise aus SV 40 oder anderen viralen Quellen (z.B. Polyoma, Adeno, VSV, PBV, etc.) oder kann durch die chromosomalen Replikationsmechanismen der Wirtszelle vorgesehen werden. Wird der Vektor in das Wirtszellenchromosom integriert, reicht die zuletztgenannte Maßnahme meistens aus.

Die Gene können jedoch vorzugsweise in einem Expressions-Plasmid pER103 (E. Rastl-Dworkin et al., Gene 21, 237-248 (1983) und EP-A-0.115-613 - hinterlegt bei der DSM unter der Nummer DSM 2773 am 20. Dezember 1983) exprimiert werden, da dieser Vektor alle Regulationselemente enthält, die zu einer hohen Expressionsrate der klonierten Gene führen. Erfindungsgemäß wurde also in dem Plasmid pBR322 das plasmideigene kürzere EcoRI/BamHI-Fragment durch die DNA-Sequenz der Formel

```
EcoRI      Sau3A
gaattcacgctGATCGCTAAAACATTGTGCAAAAAGAGGGTTGACTTTGCCTTCGCGA  59

                 ↓mRNA-Start                             Met
ACCAGTTAACTAGTACACAAGTTCACGGCAACGGTAAGGAGGTTTAAGCTTAAAG ATG 116
                                    RBS     HindIII

Cys Asp
TGT GAT C ―― IFN-omega-Gen ――→
    Sau3A
```

ersetzt.

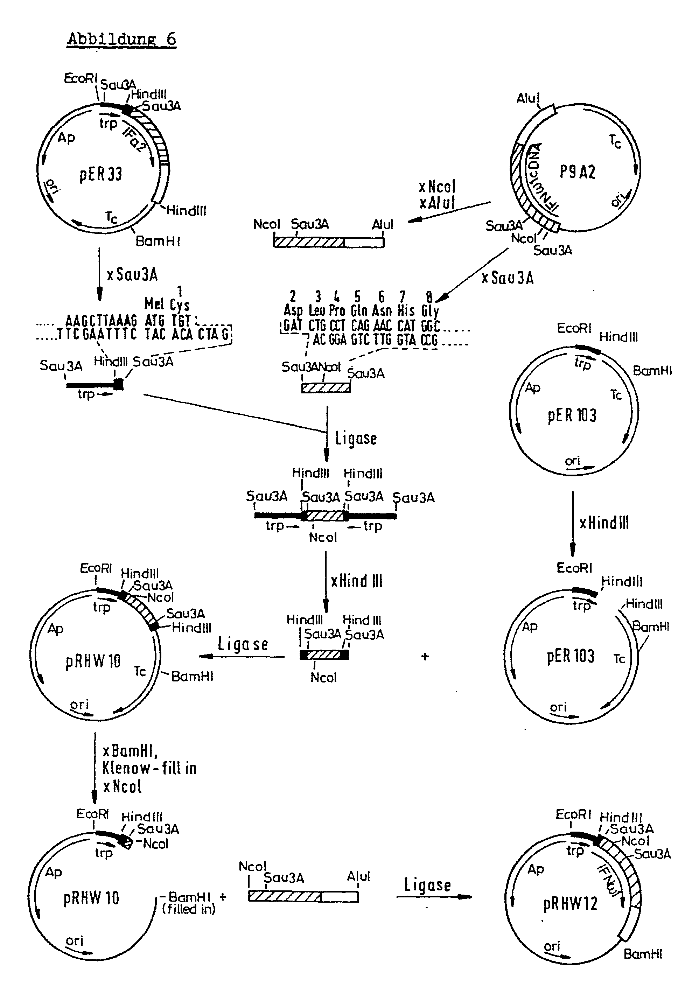

Um das erfindungsgemäße Ziel zu erreichen, wird gemäß Abbildung 6 beispielsweise wie folgt verfahren:

I. Herstellung der hierfür erforderlichen einzelnen DNA-Fragmente:

Fragment a

Zur Herstellung des Fragments a) wird ein Plasmid, das ein Gen für IFN-omega enthält, z.B. das Plasmid P9A2, mit der Restrictionsendonuclease AvaII verdaut. Nach Chromatographie und Reinigung des erhaltenen cDNA-Inserts wird dieses mit den Restrictionsendonucleasen NcoI und AluI zweifach weiterverdaut und anschließend mittels Chromatographie und Elektroelution isoliert. Dieses Fragment enthält den größten Teil des entsprechenden omega-Interferon-Gens. So weist beispielsweise das omega(Gly)-Interferon-Gen des Klons P9A2 folgende Struktur auf:

```
                    5                       10                      15
                                   His Gly Leu Leu Ser Arg Asn Thr Leu
                                 c CAT GGC CTA CTT AGC AGG AAC ACC TTG  28
                                   NcoI

                    20                      25                      30
Val Leu Leu His Gln Met Arg Arg Ile Ser Pro Phe Leu Cys Leu
GTG CTT CTG CAC CAA ATG AGG AGA ATC TCC CCT TTC TTG TGT CTC  73

                    35                      40                      45
Lys Asp Arg Arg Asp Phe Arg Phe Pro Gln Glu Met Val Lys Gly
AAG GAC AGA AGA GAC TTC AGG TTC CCC CAG GAG ATG GTA AAA GGG 118

                    50                      55                      60
Ser Gln Leu Gln Lys Ala His Val Met Ser Val Leu His Glu Met
AGC CAG TTG CAG AAG GCC CAT GTC ATG TCT GTC CTC CAT GAG ATG 163

                    65                      70                      75
Leu Gln Gln Ile Phe Ser Leu Phe His Thr Glu Arg Ser Ser Ala
CTG CAG CAG ATC TTC AGC CTC TTC CAC ACA GAG CGC TCC TCT GCT 208

                    80                      85                      90
Ala Trp Asn Met Thr Leu Leu Asp Gln Leu His Thr Gly Leu His
GCC TGG AAC ATG ACC CTC CTA GAC CAA CTC CAC ACT GGA CTT CAT 253

                    95                      100                     105
Gln Gln Leu Gln His Leu Glu Thr Cys Leu Leu Gln Val Val Gly
CAG CAA CTG CAA CAC CTG GAG ACC TGC TTG CTG CAG GTA GTG GGA 298
```

```
                    110                      115                      120
Glu Gly Glu Ser Ala Gly Ala Ile Ser Ser Pro Ala Leu Thr Leu
GAA GGA GAA TCT GCT GGG GCA ATT AGC AGC CCT GCA CTG ACC TTG 343

                    125                      130                      135
Arg Arg Tyr Phe Gln Gly Ile Arg Val Tyr Leu Lys Glu Lys Lys
AGG AGG TAC TTC CAG GGA ATC CGT GTC TAC CTG AAA GAG AAG AAA 388

                    140                      145                      150
Tyr Ser Asp Cys Ala Trp Glu Val Val Arg Met Glu Ile Met Lys
TAC AGC GAC TGT GCC TGG GAA GTT GTC AGA ATG GAA ATC ATG AAA 433

                    155                      160                      165
Ser Leu Phe Leu Ser Thr Asn Met Gln Glu Arg Leu Arg Ser Lys
TCC TTG TTC TTA TCA ACA AAC ATG CAA GAA AGA CTG AGA AGT AAA 478

                    170
Asp Arg Asp Leu Gly Ser Ser
GAT AGA GAC CTG GGC TCA TCT TGAAATGATTCTCATTGATTAATTTGCCATA 530

TAACACTTGCACATGTGACTCTGGTCAATTCAAAAGACTCTTATTTCGGCTTTAATCAC 589
AGAATTGACTGAATTAGTTCTGCAAATACTTTGTCGGTATATTAAGCCAGTATATGTTA 648
AAAAGACTTAGGTTCAGGGGCATCAGTCCCTAAGATGTTATTTATTTTTACTCATTTAT 707
TTATTCTTACATTTTATCATATTTATACTATTTATATTCTTATATAACAAATGTTTGCC 766
TTTACATTGTATTAAGATAACAAAACATGTTCAGct                        802
                                 AluI
```

Fragment b:

Zur Herstellung des Fragments b) wird das Plasmid P9A2 mit der Restrictionsendonuclease AvaII verdaut. Nach Chromatographie und Reinigung des erhaltenen cDNA-Inserts wird dieses mit der Restrictionsendonuclease Sau3A weiterverdaut und das gewünschte 189bp lange Fragment mittels Chromatographie und Elektroelution isoliert. Dieses weist folgende Struktur auf:

```
                    5                   10                  15
    Asp Leu Pro Gln Asn His Gly Leu Leu Ser Arg Asn Thr Leu
    GAT CTG CCT CAG AAC CAT GGC CTA CTT AGC AGG AAC ACC TTG   42
    Sau3A               NcoI

                20                  25                  30
Val Leu Leu His Gln Met Arg Arg Ile Ser Pro Phe Leu Cys Leu
GTG CTT CTG CAC CAA ATG AGG AGA ATC TCC CCT TTC TTG TGT CTC   87

                35                  40                  45
Lys Asp Arg Arg Asp Phe Arg Phe Pro Gln Glu Met Val Lys Gly
AAG GAC AGA AGA GAC TTC AGG TTC CCC CAG GAG ATG GTA AAA GGG  132

                50                  55                  60
Ser Gln Leu Gln Lys Ala His Val Met Ser Val Leu His Glu Met
ACG CAG TTG CAG AAG GGC CAT GTC ATG TCT GTC CTC CAT GAG ATG  177

Leu Gln Gln Ile
CTG CAG CAg atc                                              189
          Sau3A
```

Fragment c

Zur Herstellung des Fragments c) wird das Plasmid pER33 (siehe E. Rastl-Dworkin et al., Gene 21, 237-248 (1983) und EP-A-0.115.613) mit den Restrictionsenzymen EcoRI und PvuII zweifach verdaut. Das nach Agarosegel-Fraktionierung und Reinigung erhaltene 389bp lange Fragment, welches den Trp-Promotor, die ribosomale Bindungsstelle und das Startcodon enthält, wird anschließend mit Sau3A verdaut. Das gewünschte 108bp lange Fragment erhält man durch Agarosegel-Elektrophorese, Elektroelution und Elutip®-Säulenreinigung. Dieses weist folgende Struktur auf:

```
EcoRI      Sau3A
gaattcacgctGATCGCTAAAACATTGTGCAAAAAGAGGGTTGACTTTGCCTTCGCGA   59

                 mRNA-Start                                    Met
ACCAGTTAACTAGTACACAAGTTCACGGCAACGGTAAGGAGGTTTAAGCTTAAAG ATG  116
                                     RBS       HindIII

Cys Asp
TGT gat c                                                    123
    Sau3A
```

Ligierung der Fragmente b und c:

Die Fragmente b und c werden mit T4-Ligase ligiert und nach Zerstörung des Enzyms mit HindIII geschnitten. Dieses ligierte Fragmente weist folgende Struktur auf:

```
HindIII              Sau3A                  NcoI
aAGCTTAAAG ATGTGTGATC TGCCTCAGAA CCATGGCCTA CTTAGCAGGA     50

ACACCTTGGT GCTTCTGCAC CAAATGAGGA GAATCTCCCC TTTCTTGTGT    100

CTCAAGGACA GAAGAGACTT CAGGTTCCCC CAGGAGATGG TAAAAGGGAG    150

CCAGTTGCAG AAGGCCCATG TCATGTCTGT CCTCCATGAG ATGCTGCAGC    200

AGATCACACA TCTTTAagct t
 Sau3A          HindIII
```

Alternativ kann dieses für die Herstellung des Plasmids pRHW10 notwendige DNA-Fragment auch durch die Verwendung von zwei synthetisch hergestellten Oligonukleotiden erzeugt werden:

Das Oligonukleotid der Formel

5'-AGCTTAAAGATGTGT-3'

bleibt an seinem 5'-Ende dephosphoryliert.

Das Oligonukleotid der Formel

5'-GATCACACATCTTTA-3'

wird mittels der $T_4$-Polynukleotidkinase und ATP am 5'-Ende kinasiert.

Beim Hybridisieren beider Oligonukleotide erhält man folgendes kurzes DNA-Fragment:

```
5'-AGCTTAAAGATGTGT           3'
3'-     ATTTCTACACACTAGp     5'
```

Es entsteht dadurch an einem Ende der für HindIII typische 5'-Überhang und am anderen Ende der für Sau3A typische 5'-Überhang.

Das Fragment b) wird unter Verwendung von Kalbsdarmphosphatase dephosphoryliert. Fragment b) und das oben beschriebene Fragment werden zusammengebracht und mittels $T_4$-Ligase miteinander verbunden.

Da die Ligase mindestens ein 5'-Phosphat-hältiges Ende benötigt, können nur das synthetische DNA-Stück mit Fragment b) oder aber 2 synthetische Fragmente an ihren Sau3A-Enden verknüpft werden. Da sich die beiden resultierenden Fragmente in ihrer Länge unterscheiden, können sie durch selektive Isopropanolfällung getrennt werden. Das so gereinigte Fragment wird mittels $T_4$-Polynukleotidkinase und ATP phosphoryliert.

II. Herstellung der Expressionsplasmide

a) Herstellung des Plasmids pRHW10:

Man linearisiert das Expressionsplasmid pER103 (E. Rastl-Dworkin et al., Gene 21, 237-284 (1983) und EP-A-0.115.613 - hinterlegt bei der DSM unter der DSM-Nummer 2773) mit HindIII und behandelt anschließend mit Kalbsdarmphosphatase. Nach Isolierung und Reinigung der so erhaltenen DNA wird diese dephosphoryliert und anschließend mit ligierten Fragmenten b und c (nach deren HindIII-Verdauung) ligiert. Anschließend wird E. coli HB 101 mit der erhaltenen Ligierungsmischung transformiert und auf LB-Agar plus 50 µg/ml Ampicillin kultiviert. Das die gewünschte Struktur aufweisende Plasmid erhielt die Bezeichnung pRHW 10 (siehe Abbildung 6), welches nach seiner Replication als Zwischenprodukt zur Herstellung von weiteren Plasmiden diente.

b) Herstellung des Plasmids pRHW12:

Zu dem mit BamHI geschnittenem Plasmid pRHW10 gibt man das Klenow-Fragment der DNA-Polymerase I und die 4 Deoxynucleosidtriphosphate. Das nach Inkubation erhaltene linearisierte, mit stumpfen Enden versehene Plasmid wird gereinigt und anschließend mit NcoI geschnitten. Das größere Fragment, welches mittels Agarosegel-Elektrophorese, Elektroelution und Elutip®-Reinigung gewonnen wird, wird mit dem Fragment a ligiert. Anschließend wird die Ligierungsmischung mit E. coli HB 101 transformiert und auf LB-Agar plus 50 µg/ml Ampicillin kultiviert. Das die gewünschte Struktur aufweisende Plasmid erhielt die Bezeichnung pRHW12 (siehe Abbildung 6), welches das gewünschte omega(Gly)-Interferon exprimiert.

Beispielsweise enthält 1 l der so erhaltenen Bakterienkultur (optische Dichte: 0,6 bei 600 nm) 1 x $10^6$ Internationale Einheiten an Interferon.

c) Herstellung des Plasmids pRHW11:

Zu dem mit BamHI geschnittenem Plasmid pRHW10 gibt man das Klenow-Fragment der DNA-Polymerase I und die 4 Desoxynucleosidtriphosphate. Das nach Inkubation erhaltene linearisierte, mit geraden Enden versehene Plasmid wird gereinigt und anschließend mit NcoI geschnitten. Das größere Fragment, welches mittels Agarosegel-Elektrophorese, Elektroelution und Elutip®-Reinigung gewonnen wird, wird mit dem aus dem Plasmid E79E9 analog gewonnenem Fragment a, in welchem lediglich das für die 111. Aminosäure(Gly) codierende GGG-Codon durch das GAG-Codon (Glu) ersetzt ist, ligiert. Anschließend wird die erhaltene Ligierungsmischung mit E. coli HB 101 transformiert und auf LB-Agar kultiviert. Das die gewünschte Struktur aufweisende Plasmid erhielt die Bezeichnung pRHW11 (siehe analog Abbildung 6), welches das gewünschte omega(Glu)-Interferon exprimiert.

Transformation der Zellen mit den Vehikeln kann durch eine Vielzahl an Verfahren erreicht werden. Beispielsweise kann sie durch Kalzium erfolgen, wobei entweder die Zellen in Magnesium gewaschen und die DNA den in Kalzium suspendierten Zellen zugegeben oder die Zellen einem Kopräzipitat von DNA und Kalziumphosphat ausgesetzt werden. Bei nachfolgender Genexpression werden die Zellen auf Medien übertragen, die für transformierte Zellen selektieren.

Nach erfolgter Transformation des Wirtes, Expression des Gens und Fermentation oder Zellkultivierung unter Bedingungen, bei denen IFN-omega exprimiert wird, kann das Produkt üblicherweise durch bekannte chromatographische Trennmethoden extrahiert werden, um so ein Material zu erhalten, das IFN-omega mit oder ohne Leader und Tailing-Sequenzen enthält. IFN-omega kann mit einer Leader-Sequenz am N-Terminus exprimiert werden (Pre-IFN-omega), die von einigen Wirtszellen entfernt werden kann. Wenn nicht, so ist eine Abspaltung des Leader-Polypeptids (wenn vorhanden) erforderlich, um reifes IFN-omega zu erhalten. Alternativ kann der IFN-omega Klon so modifiziert werden, daß das reife Protein im Mikroorganismus direkt statt des Pre-IFN-omega produziert wird. Für diesen Fall kann die Precursor-Sequenz des Hefe-Mating-Pheromons MF-alpha-1 verwendet werden, um eine korrekte "Reifung" des fusionierten Proteins und die Ausscheidung der Produkte in das Wachstumsmedium oder den periplasmischen Raum zu gewährleisten. Die DNA-Sequenz für funktionelles oder reifes IFN-omega kann mit MF-alpha-1 an der vermuteten Kathepsin-ähnlichen Schnittstelle (nach Lys-Arg) an Position 256 vom Initiationscodon ATG aus (Kurjan, Herskowitz, Cell 30, 933-943 (1982)) verbunden sein.

Basierend auf ihrem biologischen Wirkungsspektrum sind die neuen, erfindungsgemäßen Interferone verwendbar für jede Art der Behandlung für die auch die bekannten Interferone eingesetzt werden. Diese beinhalten beispielsweise Herpes, Rhinovirus, opportunistische AIDS-Infektionen, verschiedene Krebsarten und ähnliches. Die neuen Interferone können alleine oder in Kombination mit anderen bekannten Interferonen oder biologisch aktiven Produkten eingesetzt werden, beispielsweise mit IFN-alpha, IL-2, anderen Immun-Modulatoren und ähnlichen.

IFN-omega kann parenteral verabreicht werden in Fällen, in denen Antitumor oder antivirale Behandlung erforderlich ist und in Fällen, in denen sich immunosuppressive Eigenschaften zeigen. Dosierung und Dosierungsrate können ähnlich denen sein, die zur Zeit bei klinischen Untersuchungen für IFN-$\alpha$-Materialien gelten z.B. ca. (1-10) x $10^6$ Einheiten täglich und bei Präparaten, die zu mehr als 1 % rein sind, bis zu beispielsweise 5 x $10^7$ Einheiten täglich.

Beispielsweise können für eine zweckmäßige Dosierungsform bei einem im wesentlichen homogenen, bakteriell produzierten IFN-omega, bei parenteraler Verwendung 3 mg IFN-omega in 25 ml 5%igem menschlichem Serum Albumin gelöst werden. Diese Lösung wird dann durch ein bakteriologisches Filter gegeben und die gefilterte Lösung aseptisch auf 100 Fläschchen verteilt, von dem jedes 6 x $10^6$ Einheiten reines IFN-omega zur parenteralen Applikation geeignet, enthält. Die Gläschen werden vor Verwendung vorzugsweise in der Kälte (-20° C) aufbewahrt. Die erfindungsgemäßen Substanzen können in bekannter Weise formuliert werden, um pharmazeutisch verwendbare Mittel zu erhalten, wobei das erfindungsgemäße Polypeptid mit einer pharmazeutischen, akzeptablen Trägersubstanz vermischt wird. Gebräuchliche Trägerstoffe und ihre Formulierung sind bei E.W. Martin in Remingtom's Pharmaceutical Sciences beschrieben, worauf ausdrücklich Bezug genommen wird. IFN-omega wird mit einer angemessenen Menge des Trägerstoffes vermischt, um geeignete pharmazeutische Mittel zu schaffen, die für eine effektive Anwendung beim Empfänger (Patienten) geeignet sind. Bevorzugt wird eine parenterale Applikation vorgenommen.

Die folgenden Beispiele, die die Erfindung nicht eingrenzen sollen, beschreiben die Erfindung im Detail.

Beispiel 1

Auffinden IFN-Sequenz-spezifischer Klone

a) Herstellung der cDNA-Bibliothek

mRNA aus Sendai-Virus-stimulierten Zellen wird als Ausgangsmaterial zur Herstellung einer cDNA-Bibliothek nach literaturbekannten Methoden verwendet (E. Dworkin-Rastl et al., Journal of Interferon Research Vol 2/4, 575-585 (1982)). Die angefallenen 30.000 Klone werden individuell in die Näpfe von Mikrotiterplatten übertragen. Folgendes Medium wird zum Wachstum und Einfrieren der Kolonien verwendet:

| | | | |
|---|---|---|---|
| | 10 | g | Trypton |
| | 5 | g | Yeast Extract |
| | 5 | g | NaCl |
| | 0,51 | g | Na-Citrat x 2 $H_2O$ |
| | 7,5 | g | $K_2HPO_4$ x 2 $H_2O$ |
| | 1,8 | g | $KH_2PO_4$ |
| | 0,09 | g | $MgSO_4$ x 7 $H_2O$ |
| | 0,9 | g | $(NH_4)_2SO_4$ |
| | 44 | g | Glyzerin |
| | 0,01 | g | Tetracyclin x HCl |
| ad | 1 | l | $H_2O$ |

Die Mikrotiterplatten mit den individuellen Klonen werden über Nacht bei 37° C inkubiert und danach bei -70° C aufbewahrt.

b) Hybridisierungsprobe

Als Ausgangsmaterial für die Hybridisierungsprobe dient das rekombinante Plasmid pER33 (E. Dworkin-Rastl et al., Gene 21, 237-248 (1983)). Dieses Plasmid enthält die kodierende Region für das reife Interferon IFN-α2arg plus 190 Basen der 3' nichttranslatierten Region. 20 µg pER33 werden mit 30 Einheiten HindIII Restriktionsendonuklease in 200 µl Reaktionslösung (10 mM Tris-HCl pH-7,5, 10 mM $MgCl_2$, 1 mM Dithiotreitol (DTT), 50 mM NaCl) 1 Stunde bei 37° C inkubiert. Die Reaktion wird durch Zusatz von 1/25 vol 0,5 M Äthylendinitrilotetraessigsäure (EDTA) und Erhitzen auf 70° C für 10 Minuten beendet. Nach Zusatz von 1/4 vol 5 x Puffer (80 % Glyzerin, 40 mM Tris-Essigsäure pH=7,8, 50 mM EDTA, 0,05 % Natriumdodecylsulfat (SDS), 0,1 % Bromphenolblau) werden die entstandenen Fragmente der Größe nach elektrophoretisch in einem 1 % Agarosegel getrennt [Gel- und Laufpuffer (TBE): 10,8 g/l Trishydroxymethylaminomethan (TrisBase), 5,5 g/l Borsäure, 0,93 g/l EDTA]. Nach Inkubation des Gels in einer 0,5 µg/ml Ethidiumbromidlösung werden die DNA-Banden im UV-Licht sichtbar gemacht, und der Gelbereich, der das IFN-Gen-hältige DNA Stück (ca. 800 bp lang) enthält, ausgeschnitten. Durch Anlegen einer Spannung wird die DNA in 1/10 x TBE-Puffer elektroeluiert. Die DNA Lösung wird einmal mit Phenol und viermal mit Äther extrahiert und die DNA durch Zusatz von 1/10 vol 3 M Natriumacetat (NaAc) pH-5,8 und 2,5 vol EtOH aus der wäßrigen Lösung bei -20° C präzipitiert. Nach dem Abzentrifugieren wird die DNA einmal mit 70 % Äthanol gewaschen und 5 Minuten im Vakuum getrocknet. Die DNA wird in 50 µl Wasser aufgenommen (ca. 50µg/µl). Diese DNA wird durch Nicktranslation (modifiziert nach T. Maniatis et al., Molecular Cloning, ed. CSH) radioaktiv markiert. 50 µl Inkubationslösung enthalten: 50 mM Tris pH=7,8, 5 mM $MgCl_2$, 10 mM Mercaptoäthanol, 100 ng Insert-DNA von pER33, 16 pg DNaseI, 25 µMol dATP, 25 µMol dGTP, 25 µMol dTTP, 20 µCi $\alpha^{32}$P-dCTP (> 3000 Ci/mMol) sowie 3 units DNA-Polymerase I (E.coli). Die Inkubation erfolgte bei 14° C für 45 Minuten. Die Reaktion wird durch Zusatz von 1 vol 50 mM EDTA, 2 % SDS, 10 mM Tris pH=7,6 Lösung und Erhitzen auf 70° C für 10 Minuten beendet. Die DNA wird durch Chromatographie über Sephadex® G-100 in TE-puffer (10 mM Tris pH=8,0, 1 mM EDTA) von nicht eingebauter Radioaktivität getrennt. Die radioaktiv markierte Probe weist eine spezifische Aktivität von ca. 4 x $10^7$ cpm/µg auf.

c) Screening der Klone auf IFN-Gen-haltige Inserts

Die in den Mikrotiterplatten eingefrorenen Bakterienkulturen werden aufgetaut (a). Ein Stück Nitrozellulosefilter der entsprechenden Größe (Schleicher und Schüll, BA 85, 0,45 µm Porengröße) wird auf LB-Agar aufgelegt (LB-Agar: 10 g/l Trypton, 5 g/l Yeast Extrakt, 5 g/l NaCl, 15 g/l Bacto Agar, 20 mg/l Tetracyclin-HCl). Mit einem den Mikrotiterplatten angepaßten Stempel werden die individuellen Klone auf das Nitrozellulosefilter übertragen. Die Bakterien wachsen über Nacht bei 37° C zu etwa 5 mm Durchmesser großen Kolonien. Zum Zerstören der Bakterien und Denaturieren der DNA werden die Nitrozellulosefilter der Reihe nach auf einen Stapel mit folgenden Lösungen getränkte Whatman 3MM Filter gelegt: 1) 8 Minuten auf 0,5 M NaOH, 2) 2 Minuten 1 M Tris pH=7,4, 3) 2 Minuten 1 M Tris pH=7,4 und 4) 4 Minuten 1,5 M NaCl, 0,5 M Tris pH=7,4. Die Filter werden luftgetrocknet und dann 2 Stunden bei 80° C gehalten. Die Vorbehandlung der Filter erfolgte 4 Stunden bei 65° C in der Hybridisierlösung, bestehend aus 6 x SSC (1 x SSC entspricht 0,15 M NaCl; 0,015 M Trinatriumcitrat; pH=7,0), 5 x Denhardt's Lösung (1x Denhardt's Lösung entspricht 0,02 % PVP (Polyvinylpyrrolidon); 0,02 % Ficoll (MG: 40.000 D); 0,02 % BSM (Bovine Serum Albumine) und 0,1 % SDS (Sodium dodecylsulfate). Ca 1 x $10^6$ cpm pro Filter der in b) hergestellten Probe werden durch Kochen denaturiert und der Hybridisierlösung zugesetzt. Hybridisierung erfolgt 16 Stunden bei 65° C. Das Waschen der Filter erfolgt bei 65° C viermal 1 Stunde mit 3 x SSC/0,1 % SDS. Die Filter werden luftgetrocknet, mit Saran Wrap® bedeckt und auf Kodak X-OmatS® Film exponiert.

## Beispiel 2

### Southern Transfer zur Bestätigung von IFN-Gen-haltigen rekombinanten Plasmiden

Von den positiv oder positiv verdächtig reagierenden Kolonien werden 5 ml Kulturen in L-Broth (10 g/l Trypton, 5 g/l Yeast Extract, 5 g/l NaCl, 20 mg/l Tetracyclin x HCl) über Nacht bei 37° C gezüchtet. Die Plasmid-DNA wird modifiziert nach Birnboim und Doly (Nucl. Acid Res. 7, 1513, (1979)) isoliert. Die Zellen von 1,5 ml Suspension werden abzentrifugiert (Eppendorf Zentrifuge) und bei 0° C in 100 µl Lysozymlösung bestehend aus 50 mM Glucose, 10 mM EDTA, 25 mM Tris-HCl pH=8,0 und 4 mg/ml Lysozym resuspendiert. Nach 5 Minuten Inkubation bei Raumtemperatur werden 2 Volumteile eiskalter 0,2 M NaOH, 1 % SDS Lösung zugegeben und weitere 5 Minuten inkubiert. Dann werden 150 µl eiskalte Kaliumacetatlösung pH= 4,8 zugesetzt und 5 Minuten inkubiert. Die ausgefallenen Zellbestandteile werden abzentrifugiert. Die DNA-Lösung wird mit 1 Volumteil Phenol/$CHCl_3$ (1:1) extrahiert und die DNA durch Zugabe von 2 Volumteilen Äthanol ausgefällt. Nach dem Abzentrifuieren wird das Pellet einmal mit 70 % Ethanol gewaschen und 5 Minuten im Vakuum getrocknet. Die DNA wird in 50 µl (TE)-Puffer gelöst. 10 µl davon werden in 50 µl Reaktionslösung (10 mM Tris-HCl pH=7,5, 10 mM $MgCl_2$, 50 mM NaCl, 1 mM DTT) mit 10 Einheiten PstI-Restriktionsendonuklease 1 Stunde bei 37° C verdaut. Nach Zusatz von 1/25 Volumteilen 0,5 M EDTA sowie 1/4 Volumteilen 5 x Puffer (siehe Beispiel 1b)) wird 10 Minuten bei 70° C erhitzt und die DNA anschließend in einem 1 % Agarosegel (TBE-puffer) elektrophoretisch aufgetrennt. Die DNA im Agarosegel wird nach der Methode von Southern (E.M. Southern, J. Mol. Biol. 98, 503-517 (1975)) auf ein Nitrozellulosefilter übertragen. Die DNA im Gel wird durch einstündige Inkubation des Gels mit einer 1,5 M NaCl/0,5 M NaOH Lösung denaturiert. Anschließend wird 1 Stunde mit einer 1 M Tris x HCl pH=8/1,5 M NaCL Lösung neutralisiert. Die DNA wird mit 10 x SSC (1,5 M NaCl, 0,15 M Natriumcitrat, pH=7,0) auf das Nitrozellulosefilter übertragen. Nach vollendetem Transfer (ca. 16 Stunden) wird das Filter kurz in 6 x SSC Puffer gespült, dann luftgetrocknet und abschließend 2 Stunden bei 80 ° C gebacken. Das Filter wird 4 Stunden mit einer 6 x SSC/5 x Denhardt's Lösung/0,1 % SDS (siehe Beispiel 1c) bei 65 ° C vorbehandelt. Ca. 2 x $10^6$ cpm der Hybridisierungsprobe (siehe Beispiel 1b) werden durch Erhitzen auf 100 ° C denaturiert und anschließend in die Hybridisierlösung eingebracht. Die Hybridisierdauer beträgt 16 Stunden bei 65 ° C. Anschließend wird das Filter 4 x 1 Stunde bei 65 ° C mit einer 3 x SSC/0,1 % SDS-Lösung gewaschen. Nach dem Lufttrocknen wird das Filter mit Saran Wrap® bedeckt und auf Kodak X-Omat® S Film exponiert.

Beispiel 3

Nachweis von Interferonaktivität im Klon E76E9

Eine 100 ml Kultur des Klons E76E9 wird in L-Broth (10 g/l Trypton, 5 g/l Yeast Extract, 5 g/l NaCl, 5 g/l Glucose, 20 mg Tetracyclin x HCl pro 1) bei 37 ° C bis zu der optischen Dichte $A_{600}$ = 0,8 angezüchtet. Die Bakterien werden 10 Minuten mit 7000 rpm abzentrifugiert, einmal mit einer 50 mM Tris x HCl pH = 8,0, 30 mM NaCl Lösung gewaschen und in 1,5 ml Waschlösung resuspendiert. Nach Inkubation mit 1 mg/ml Lysozym bei 0 ° C eine halbe Stunde lang, wird die Bakteriensuspension fünfmal gefroren und getaut. Die Zelltrümmer werden durch Zentrifugation mit 40.000 rpm eine Stunde lang pelletiert. Der Überstand wird sterilfiltriert und auf Interferonaktivität geprüft. Als Test verwendet man den Plaque Reduktionstest mit V3-Zellen und Vesicular Stomatitis Virus (G.R. Adolf et al., Arch. Virol. 72, 169-178 (1982)). Überraschenderweise produziert der Klon bis zu 9000 IU Interferon pro Liter Ausgangskultur.

Beispiel 4

Genomischer Southern Blot zur Bestimmung der Anzahl der Gene, die den neuen Sequenzen zugeordnet sind

a) Isolierung der DNA aus Namalwa-Zellen

400 ml einer Namalwa-Zellkultur werden bei 1000 rpm in einer JA21-Zentrifuge zentrifugiert, um die Zellen zu pelletieren. Die erhaltenen Pellets werden vorsichtig gewaschen, in dem diese in NP40-Puffer resuspendiert (NP40-Puffer: 140 mM NaCl, 1,5 mM $MgCl_2$, 10 mM Tris/Cl pH = 7,4) und erneut bei 1000 rpm pelletiert werden. Die erhaltenen Pellets werden erneut in 20 ml NP40®-Puffer suspendiert und zur Zerstörung der Zellwände mit 1 ml einer 10%igen NP40®-Lösung versetzt. Nach 5-minütigem Stehen in einem Eisbad werden die intakten Zellkerne durch Zentrifugation bei 1000 rpm pelletiert und der Überstand verworfen. Die Zellkerne werden in 10 ml einer Lösung, bestehend aus 50 mM Tris/Cl pH = 8.0, 10 mM EDTA und 200 mM NaCl, resuspendiert und anschließend mit 1 ml 20%iger SDS versetzt, um die Proteine zu entfernen. Die entstandene viskose Lösung wird zweimal mit der gleichen Menge Phenol (gesättigt mit 10 mM Tris/Cl pH = 8.0) und zweimal mit Chloroform extrahiert. Die DNA wird durch Zugabe von Ethanol ausgefällt, durch Zentrifugation abgetrennt, anschließend das erhaltene DNA-Pellet einmal mit 70%igem Äthanol gewaschen, 5 Minuten lang im Vakuum getrocknet und in 6 ml TE-Puffer (TE-Puffer: 10 mM Tris/Cl pH = 8.0, 1 mM EDTA) gelöst. Die Konzentration der DNA beträgt 0,8 mg/ml.

b) Restriktions-Endonuclease-Verdauung der DNA aus Namalwa-Zellen

Die Restriction-Endonuclease-Verdauungen werden jeweils gemäß den nach den vom Hersteller (New England Biolabs) angegebenen Bedingungen durchgeführt. 1 µg DNA wird mit 2 Einheiten der geeigneten Restriction-Endonuclease in einem Volumen von 10 µl bei 37 ° C 2 Stunden lang oder länger verdaut. EcoRI, HindIII, BamHI, SphI, PstI und ClaI werden als Restriction-Endonucleasen verwendet. 20 µg DNA werden bei jeder Verdauung eingesetzt. Um die Vollständigkeit der Verdauung zu kontrollieren werden jeweils beim Start der Reaktion 10 µl (aliquote Teile) abgetrennt und mit 0,4 µg Lambda Phagen-DNA versetzt. Nach 2-stündiger Inkubation werden diese aliquoten Teile mittels Agarose-Gel-Elektrophorese kontrolliert und die Vollständigkeit der Verdauung wird mit Hilfe eines Musters der gefärbten Lambda Phagen-DNA-Fragmente beurteilt.

Nach Durchführung dieser Kontrolle werden die Reaktionen durch Zugabe von EDTA bis zu einer Endkonzentration von 20 mM und 10-minütiges Erhitzen der Lösung auf 70 ° C gestoppt. Die DNA wird durch Zugabe von 0.3 M NaAc pH = 5.6 und 2.5 Volumteilen Äthanol gefällt, Nach 30-minütiger Inkubation bei -70 ° C wird die DNA in einer Eppendorf-Zentrifuge pelletiert, einmal mit 70%igem Äthanol gewaschen und getrocknet. Die erhaltene DNA wird in 30 µl TE-Puffer aufgenommen.

c) Gel Elektrophorese und Southern Transfer

Die verdauten DNA-Proben werden entsprechend ihrer Größe in einem 0,8%igem Agarose-Gel in TBE-Puffer (10,8 g/l TrisBase, 5,5 g/l Borsäure, 0,93 g/l EDTA) fraktioniert. Hierzu werden 15 µl der DNA-Probe mit 4 µl Beladungs-Puffer (0,02 % SDS, 5 x TBE-Puffer, 50 mM EDTA, 50 % Glycerin, 0,1 % Bromphenolblau) vermischt, kurz auf 70° C erhitzt und in die vorgesehenen Tröge des Gels aufgetragen. Lambda-DNA, welche mit EcoRI und HindIII geschnitten wurden wird in einem entsprechenden Trog aufgetragen und dient als Marker für die Größe der DNA. Die Gel-Electrophorese wird 24 Stunden lang bei ungefähr 1 V/cm durchgeführt. Anschließend wird die DNA nach der Methode von Southern auf ein Nitrocellulose-Filter (Schleicher und Schuell, BA85) gebracht, hierbei wird als Transfer-Puffer 10 x SSC (1 x SSC: 150 mM Trinatriumcitrat, 15 mM NaCl, pH = 7,0) verwendet. Nach dem Trocknen des Filters bei Raumtemperatur wird dieses 2 Stunden lang auf 80° C erhitzt, um die DNA an dieses zu binden.

d) Hybridisierungsprobe

20 µg des Plasmids P9A2 werden mit AvaII geschnitten, wobei ein ungefähr 1100 bp langes Fragment freigesetzt wird, welches das ganze cDNA-Insert enthält. Dieses DNA-Stück wird erneut mit Sau3A und AluI geschnitten und das größte DNA-Stück nach Agarose-Gel-Elektrophorese (siehe Abbildung 5b), mittels Elektroelution und Elutip®-Säulenchromatographie isoliert. Die so erhaltene DNA (1,5 µg) wird in 15 µl Wasser gelöst.

20 µg des Plasmids pER33 werden mit HindIII geschnitten, wobei dieses Expressionsplasmid für IFN-α2-Arg (E. Rastl.-Dworkin et al. Gene 21, 237-248 (1983)) zweimal geschnitten wird. Das kleinere DNA-Fragment enthält das Gen für Interferon-α2-Arg und wird auf die gleiche Weise wie bei dem Plasmid P9A2 isoliert.

Beide DNA's werden nach der von P.W.J. Rigby et al. (J. Mol. Biol. 113, 237-251 (1977)) vorgeschlagenen Methode nicktranslatiert. Die Nick-Translation wird jeweils mit 0,2 µg DNA in einer Lösung von 50 µl, bestehend aus 1 x Nick-Puffer (1 x Nick-Puffer: 50 mM Tris/Cl pH = 7,2, 10 mM $MgSO_4$, 0,1 mM DTT, 50 µg/ml BSA), je 100 µMol dATP, dGTP und dTTP, 150 µCi α-$^{32}$P-dCTP (Amersham, 3 000 Ci/mMol) und 5 Einheiten DNA-Polymerase I (Boehringer-Mannheim, Nick-Translation Qualität) durchgeführt. Nach 2 Stunden bei 14° C wird die Reaktion durch Zugabe der gleichen Menge einer EDTA-Lösung (40 mMol) gestoppt und das nicht umgesetzte radioaktive Material mittels Sephadex® G50-Säulenchromatographie in TE-Puffer abgetrennt. Die verbleibende spezifische Radioaktivität beträgt ungefähr 100 x $10^6$ cpm/µg DNA.

e) Hybridisierung und Autoradiography

Das Nitrocellulosefilter wird in zwei Hälften geschnitten. Jede Hälfte enthält einen identischen Satz von Spuren mit Namalwa-DNA, welche mit den Restrictions-Enzymen, die in Beispiel 4a erwähnt sind, behandelt worden sind. Die Filter werden in einer Lösung enthaltend 6 x SSC, 5 x Denhardt's (1 x Denhardt's: 0,02 % Rinderserum Albumin (BSA), 0,02 % Polyvinylpyrrolidon (PVP), 0,02 % Ficoll 400), 0,5 % SDS, 0,1 mg/ml denaturierte Kalbsthymus-DNA und 10 mM EDTA 2 Stunden lang bei 65° C vorhybridisiert. Die Hybridisierung wird in einer Lösung enthaltend 6 x SSC, 5 x Denhardt's, 10 mM EDTA, 0,5 % SDS und ungefähr 10 x $10^6$cpm nicktranslatierter DNA während 16 Stunden bei 65° C durchgeführt. Die eine Hälfte des Filters wird mit Interferon-α2-Arg-DNA und die andere Hälfte mit Interferon-DNA, die aus dem Plasmid P9A2 isoliert wurde, hybridisiert. Nach der Hybridisierung werden beide Filter bei Raumtemperatur viermal mit einer Lösung bestehend aus 2 x SSC und 0,1 % SDS und dann zweimal bei 65° C 45 Minuten lang mit einer Lösung bestehend aus 0,2 x SSC und 0,01 % SDS gewaschen. Anschließend werden die Filter getrocknet und einem Kodak X-Omat® S Film exponiert.

Beispiel 5

Herstellung der Expressionsplasmide pRHW 12 und pRHW 11

Vorbemerkung:

Die Herstellung der Expressionsplasmide wird in Abbildung 6 dargestellt (nicht maßstabgerecht), ferner werden alle Restrictionsenzymverdauungen nach den Angaben der Enzymhersteller durchgeführt.

a) Herstellung des Plasmids pRHW 10

100 µg des Plasmids P9A2 werden mit 100 Einheiten der Restriction-Endonuclease AvaII (New Englands Biolabs) verdaut. Nach der Verdauung wird das Enzym durch Erhitzen auf 70° C inaktiviert und die erhaltenen Fragmente an einem 1,4%igen Agarose Gel mit TBE-Puffer (TBE-Puffer: 10,8 g/l Tris-Base, 5,5 g/l Borsäure, 0,93 g/l EDTA) entsprechend ihrer Größe fraktioniert. Die Bande, welche das ganze cDNA-Insert enthält, wird elektroeluiert und an einer Elutip®-Säule (Schleicher & Schuell) gereinigt. Von den erhaltenen 20 µg werden 6 µg mit der Restriction-Endonuclease Sau3a (20 Einheiten in insgesamt 100 µl Lösung) weiter verdaut. Die Fragmente trennt man mittels 2%igen Agarose Gel in TBE-Puffer. Nach Färben mit Ethidium Bromid (EtBr) wird das 189 bp lange DNA-Stück elektroeluiert und wie oben beschrieben gereinigt ( = Fragment b in Abbildung 6).

Um das Interferongen mit einem Promotor, einer ribosomalen Bindungsstelle und einem Startcodon zu verknüpfen, wird das entsprechende DNA-Stück aus dem Expressionsplasmid pER 33 (E.Rastl-Dworkin et al., Gene 21, 237-248 (1983)) isoliert. Hierzu werden 50 µg pER 33 mit den Restrictionenzymen EcoRI und PvuII zweifach verdaut und die erhaltenen Fragmente entsprechend ihrer Größe an einem 1,4%igen Agarose-Gel in TBE-Puffer fraktioniert. Das 389 bp lange DNA-Stück, welches den Trp-Promotor, die ribosomale Bindungsstelle und das Startcodon enthält, wird elektroeluiert und über eine Elutip-Säule gereinigt. Das so erhaltene Fragment wird anschließend mit Sau3A verdaut, und das gewünschte 108 bp lange Fragment erhält man mittels Agarose-Gel-Elektrophorese, Elektro-elution und Elutip®-Säulenreinigung in einer Ausbeute von ungefähr 100 ng ( = Fragmente c in Abbildung 6).

20 ng des Fragmentes b werden mit 20 ng des Fragmentes c in einem Volumen von 40 µl unter Verwendung von 10 Einheiten T4-Ligase in einer Lösung, welche 50 mM Tris/Cl pH = 7,5, 10 mM $MgCl_2$, 1 mM DTT und 1 mM ATP enthält, bei 14° C 18 Stunden lang ligiert. Anschließend wird das Enzym durch Erhitzen auf 70° C zerstört und die erhaltene DNA mit HindIII in einem Gesamtvolumen von 50 µl geschnitten.

10 µg des Expressionsplasmids pER 103 (E. Rastl-Dworkin et al., Gene 21, 237-248 (1983)) linearisiert man mit HindIII in einem Gesamtvolumen von 100 µl. Nach 2 Stunden bei 37° C wird 1 Volumen 2 x Phosphatase Puffer (20 mM Tris/Cl pH = 9,2, 0,2 mM EDTA) zusammen mit einer Einheit Kalbsdarmphosphatase (CIP) hinzugegeben. Nach 30 Minuten bei 45° C gibt man eine weitere Einheit CIP zu und inkubiert nochmals 30 Minuten lang. Die so erhaltene DNA wird durch zweimalige Phenolextraktion, einmalige Chloroformextraktion und durch Fällung mittels Zugabe von 0,3 M Natriumacetat (pH = 5,5) und 2,5 Vol Äthanol gereinigt. Anschließend wird dephosphoryliert, um beim nächsten Ligierungsschritt die Religierung des Vektors zu verhindern.

100 ng des linearisierten pER 103 und die ligierten Fragmente b und c (nach der HindIII-Verdauung) werden in eine Lösung von 100 µl, welche Ligase Puffer enthält, eingetragen und bei 14° C 18 Stunden lang mit $T_4$-DNA-Ligase ligiert.

200 µl kompetente E. coli HB 101 (E. Dworkin et al., Dev. Biol. 76, 435-448 (1980)) werden mit 20 µl der Ligierungs-Mischung vermischt und 45 Minuten lang auf Eis inkubiert. Danach wird die DNA-Aufnahme durch einen Hitzeschock von 2 Minuten bei 42° C vollendet. Die Zellsuspension wird weitere 10 Minuten auf Eis inkubiert und schließlich auf LB-Agar (10 g/l Trypton, 5 g/l Hefeextrakt, 5 g/l NaCl, 1,5 % Agar), welcher 50 mg/l Ampicillin enthält, aufgebracht. Die Plasmide, die in den erhaltenen 24 Kolonien enthalten sind, werden nach der Methode von Birnboim und Doly (siehe Nucl. Acid. Res. 7, 1513-1523 (1979)) isoliert. Nach der Verdauung mit verschiedenen Restrictionsenzymen weist ein Plasmid die gewünschte Struktur auf. Dieses erhielt die Bezeichnung pRHW 10 (siehe Abbildung 6).

b) Herstellung des Plasmids pRHW 12

Ca. 10 µg des Plasmids pRHW 10 werden mit BamHI geschnitten. Anschließend wird das Klenow-Fragment der DNA-Polymerase I und die 4 Deoxynucleosid-triphosphate zugegeben und 20 Minuten lang bei Raumtemperatur inkubiert. Das erhaltene linearisierte und mit geraden Enden versehene Plasmid wird durch Phenolextraktion und durch Fällung gereinigt und anschließend mit der Restrictions-Endonuclease NcoI in einem Volumen von 100 µl geschnitten. Das größere Fragment wird mittels Agarose-Gel-Elektrophorese, Elektroelution und Elutip-Reinigung gewonnen. Das Fragment a) (siehe Abbildung 6) erhält man durch Verdauung von 4 µg AvaII-Fragment, welches das P9A2-cDNA-Insert enthält (siehe oben) mit NcoI und AluI, wobei man ungefähr 2 µg an Fragment a) erhält.

Im letzten Ligierungsschritt wird das Fragment a) und das hinzugegebene mit BamHI/NcoI zweifach verdaute pRHW 10 in einem Volumen von 10 µl ligiert, wobei je DNA jeweils 10 ng eingesetzt werden. Die Ligierung einer aufgefüllten BamHI-Schnittstelle an eine durch AluI geschnittene DNA ergibt wieder eine BamHI-Schnittstelle.

Die erhaltene Ligierungsmischung wird mit kompetenten E.coli HB 101 wie oben beschrieben transformiert. Von den erhaltenen 40 Kolonien wählt man eine aus, diese erhält die Bezeichnung pRHW 12.

Das Plasmid wird isoliert und das EcoRI/BamHI-Insert nach der Methode von Sanger (F. Sanger et al., Proc. Nat. Acad. Sci 74, 5463-5467 (1979)) sequenziert. Dieses weist die erwartete Sequenz auf.

c) Herstellung des Plasmids pRHW 11

Diese erfolgt analog Beispiel 5b. 1 µg des Plasmids pRHW 10 wird mit BamHI verdaut. Die klebrigen (sticky) Enden der erhaltenen DNA werden mittels des Klenow-Fragments der DNA-Polymerase I und der 4 Deoxynucleosid-triphosphate stumpf gemacht, anschließend wird die linearisierte DNA mit NcoI geschnitten. Das größere Fragment wird mittels Agarose-Gel-Elektrophorese, Elektroelution und Elutip®-Säulenchromatographie gewonnen.

Das NcoI-AluI-Fragment isoliert man aus dem Klon E76E9 analog Beispiel 5b. Anschließend ligiert man 10 ng des Vektor-Teils mit 10 ng des cDNA-Teils in einem Volumen von 10 µl unter geeigneten Bedingungen und unter Verwendung von 1 Einheit T4-Ligase. Nach Transformation der erhaltenen DNA-Mischung in E. coli HB 101 und Selection der erhaltenen 45 Kolonien auf Ampicillin enthaltenden LB-Platten wird ein Klon ausgewählt, welche die Bezeichnung pRHW 11 erhält. Nach Züchtung des entsprechenden Klons isoliert man die Plasmid-DNA. Deren Struktur wird durch die Anwesenheit von mehreren spezifischen Restriction-Endonuclease-Schnittstellen (AluI, EcoRI, HindIII, NcoI, PstI) belegt.

d) Expression der Interferon-Aktivität durch E. coli HB 101 enthaltend das Plasmid pRHW 12

100 ml der Bakterienkultur werden bis zu einer optischen Dichte von 0,6 bei 600 nm in M9 minimal Medium, welches alle Aminosäuren ausgenommen Trypthophan (20 µg/ml pro Aminosäure), 1 µg/ml Thiamin, 0,2 % Glucose und 20 µg/ml Indol(3)-acrylsäure (IAA), den Induktor des Tryptophan-Operons enthält, inkubiert. Anschließend werden die Bakterien durch Zentrifugieren (10 Minuten bei 7000 rpm) pelletiert, einmal mit 50 mM Tris/Cl pH = 8, 30 mM NaCl gewaschen und schließlich in 1,5 ml desselben Puffers suspendiert. Nach 30-minütiger Inkubation mit 1 mg/ml Lysozym auf Eis werden die Bakterien 5 x gefroren und aufgetaut. Die Zelltrümmer entfernt man durch 1-stündige Zentrifugieren bei 40 000 rpm. Der Überstand wird steril filtriert und auf Interferon-Aktivität im Plaque-Reduction-Assay unter Verwendung von menschlichen A549 Zellen und Encophalo-myocarditisvirus geprüft.

Ergebnis: 1 1 der hergestellten Bakterienkultur enthält
1 x $10^6$ Internationale Einheiten an Interferon
(A. Billiau, Antiviral Res. 4, 75-98 (1984)).

Beispiel 6

Zusammenstellung der Unterschiede der Aminosäure- und Nukleotid-Sequenzen von TypI-Interferonen

a) Vergleich der Aminosäuresequenzen

Der paarweise Vergleich der Aminosäuresequenzen erhält man durch Auflisten des ersten Cystein-Restes des reifen α-Interferons mit dem ersten Cysteinrest der Aminosäuresequenzen, welche durch die cDNA-Inserte der Plasmide P9A2 und E76E9 codiert werden. Beide Sequenzen werden in Abbildung 7 als IFN-omega dargestellt, da bei den ermittelten Werten keine Unterschiede zwischen den spezifischen Sequenzen der P9A2- oder E76E9-Klone festgestellt werden konnten. Die einzige durchgeführte Korrektur war eine Einfügung einer Lücke bei Position 45 des Interferons-αA, welche als Fehlstelle gezählt werde. Wenn die Sequenz des omega-Interferons ein Partner ist, wurde der Vergleich unter Berücksichtigung der üblichen 166 Aminosäuren durchgeführt. Dieser Wert wird in Abbildung 7 zusammen mit den zusätzlichen 6 Aminosäuren, die durch die Klone P9A2 und E76E9 codiert werden, dargestellt. Die prozentualen Unterschiede werden durch Dividierung der ermittelten Unterschiede durch die Zahl 1.66 erhalten. Eine zusätzliche Aminosäure stellt somit die Prozentsatzzahl 0,6 dar. Das ergibt 3,6 % für die 6 zusätzlichen Aminosäuren des IFN-omega, welche bereits in der Prozentsatzzahl enthalten sind.

Die Vergleiche mit dem β-Interferon wird durch Auflisten der 3. Aminosäure des reifen β-Interferons mit der ersten Aminosäure des reifen α-Interferons oder der ersten Aminosäure, welche durch die Plasmides P9A2 und E76E9 codiert werden, durchgeführt. Die längste Vergleichsstruktur eines α-Interferons mit β-Interferon geht somit über 162 Aminosäuren, was 2 zusätzliche Aminosäuren jeweils für das α-Interferon und das β-Interferon ergibt. Diese werden als Fehlstellen gezählt und werden getrennt in Abbildung 7 dargestellt, sind aber in der Prozentsatzzahl enthalten. Die Auflistung des β-Interferons mit den Aminosäuresequenzen der Klone P9A2 oder E76E9 wird in derselben Weise durchgeführt. Dies ergibt aber insgesamt 10 zusätzliche Aminosäuren.

b) Vergleich der Nucleotid-Sequenzen

Die Auflistung der zu vergleichenden Sequenzen erfolgt analog Beispiel 6b. Das erste Nucleotid von der DNA des reifen α-Interferons ist das erste Nucleotid des für Cystein codierenden Tripletts des reifen α-Interferons. Das erste Nucleotid von der DNA der Plasmide P9A2 oder E76E9 ist ebenfalls das erste Nucleotid des Codons für Cystein-1. Das erste Nucleotid von der DNA des β-Interferons ist das erste Nucleotid des 3. Tripletts. Der Vergleich erfolgt über insgesamt 498 Nucleotide, wenn die einzelnen DNA's der α-Interferone mit der DNA des β-Interferons verglichen werden, und über 516 Nucleotide, wenn die DNA-Sequenzen der einzelnen α-Interferone oder des β-Interferons mit den der Plasmide P9A2 und E76E9 verglichen werden. Die absolute Zahl der Fehlstellen wird im linken Teil der Tabelle der Abbildung 7 angegeben und dann in Klammern die entsprechenden Prozentangaben.

Beispiel 7

Virusinduzierbare Expression der omega-1-mRNA und NC37-Zellen

a) Synthese einer omega-Interferon spezifischen Hybridisierungsprobe

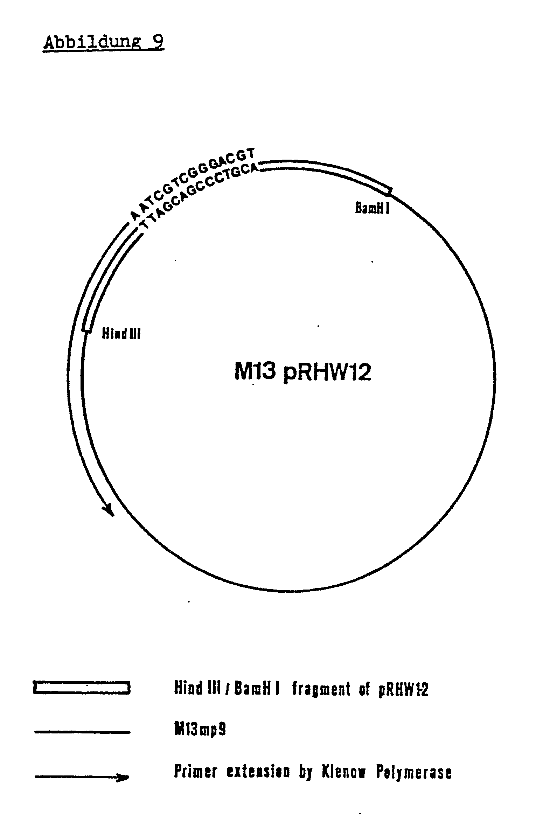

10 pMol des oligonucleotids d(TGCAGGGCTGCTAA) werden mit 12 pMol gamma- $^{32}P$-ATP (spezifische Aktivität: > 5000 Ci/mMol) und 10 Einheiten Polynucleotid-Kinase in einem Gesamtvolumen von 10 µl (70 mM Tris/Cl pH = 7,6, 10 mM $MgCl_2$, 50 mM DTT) vermischt und eine Stunde bei 37 °C stehen gelassen. Anschließend wird die Umsetzung durch 10-minütiges Erhitzen auf 70 °C gestoppt. Das erhaltene radioaktiv markierte Obligonucleotid wird mit 5 pMol M13pRHW 12 ssDNA (siehe Abbildung 9) in einem Gesamtvolumen von 35 µl (100 mM NaCl) durch einstündiges Stehen bei 50 °C hybridisiert.

Nach Kühlen auf Raumtemperatur wird Nick-Translation-Puffer, die 4 Deoxynucleosid-triphosphate und 10 Einheiten Klenow-Polymerase bis zu einem Gesamtvolumen von 50 µl (50 mM Tris/Cl pH = 7,2, 10 mM $MgCl_2$, 50 µg/ml BSA, 1 mM pro Nucleotid) zugegeben. Die Polymerisation wird durch einstündiges Stehen bei Raumtemperatur durchgeführt, anschließend die Umsetzung durch fünfminütiges Erhitzen auf 70 °C gestoppt.

Bei der Umsetzung erhält man eine teilweise doppelsträngige zirculäre DNA. Diese wird anschließend in einem Gesamtvolumen von 500 µl mit 25 Einheiten AvaII geschnitten, wobei der vom Hersteller beschriebene Puffer verwendet wird. Hierbei werden die doppelsträngigen Regionen zu einheitlichen Größen geschnitten. Anschließend wird die Umsetzung durch 5-minütiges Erhitzen auf 70 °C gestoppt.

b) Herstellung der RNA aus durch Virus infizierten Zellen

100 x $10^6$ Zellen (0,5 x $10^6$/ml) werden mit 100 µMol Dexamethason 48 bis 72 Stunden lang behandelt - die Kontrolle enthält kein Dexamethason. Zur Interferon-Induktion werden die Expression-Zellen in serumfreiem Medium in einer Konzentration von 5 x $10^6$/ml suspendiert und mit $2^{10}$ Einheiten/ml Sendai Virus infiziert. Aliquote Teile der Zellkulturüberstande werden im Plaque-Reduction-Assay (Beispiel 5b) auf IFN-Aktivität geprüft. Die Zellen werden 6 Stunden nach der Virus-Infizierung mittels Zentrifugieren (1000 g, 10 Minuten) geerntet, in 50 ml NP40®-Puffer (Beispiel 4a) gewaschen, in 9,5 ml eiskaltem NP40®-Puffer resuspendiert und durch Zugabe von 0,5 ml 10%iger NP40® 5 Minuten lang auf Eis lysiert. Nach Entfernen der Kerne durch Zentrifugieren (1000 x g, 10 Minuten) wird der Überstand mit 50 mM Tris/Cl, 0,5 % Sarkosin® und 5 mM EDTA auf pH = 8 eingestellt und bei -20 °C aufbewahrt. Zur Isolierung der gesamten RNA aus dem Überstand wird einmal mit Phenol, einmal mit Phenol/Chloroform/Isoamylalkohol und einmal mit Chloroform/Isoamylalkohol extrahiert. Die wäßrige Phase wird auf einen 4 ml 5,7 molaren CsCl-Polster aufgetragen und in einem SW40-Rotor zentrifuiert (35 krpm, 20 Stunden), um den Extrakt von DNA und verbleibenden Proteinen zu befreien. Das erhaltene RNA-Pellet wird in 2 ml TE pH = 8,0 resuspendiert und mit Äthanol ausgefällt. Die ausgefällte RNA wird anschließend mit einer Konzentration von 5 mg/ml in Wasser gelöst.

c) Nachweis der Interferon-omega mRNA

0,2 µl der gemäß Beispiel 7b hergestellten Hybridisierungsprobe werden zusammen mit 20-50 µg der

gemäß Beispiel 7c hergestellten RNA durch Zugabe von Äthanol ausgefällt. Im Kontrollexperiment wird anstatt der zellulären RNA transfer RNA (tRNA) oder RNA, welche aus mit dem Plasmid pRHW 12 (Beispiel 5) transformierten E. coli stammt, zugegeben.

Die erhaltenen Pellets werden mit 70%igem Äthanol salzfrei gewaschen, getrocknet und im 25 µl 80%igem Formamid (100 mM PIPES pH = 6,8, 400 mM NaCl, 10 mM EDTA) gelöst. Anschließend werden die Proben 5 Minuten lang auf 100° C erhitzt, um die Hybridisierungsprobe zu denaturieren, unmittelbar auf 52° C eingestellt und bei dieser Temperatur 24 Stunden lang inkubiert. Nach der Hybridisierung werden die Proben auf Eis gestellt und 475 µl SI-Reaktionsmischung (4 mM $Zn(Ac)_2$, 30 mM NaAc, 250 mM NaCl, 5 % Glycerin, 20 µg ss Kalb-Thymus-DNA, 100 Einheiten SI-Nuclease) zugegeben. Nach einstündiger Verdauung bei 37° C wird die Umsetzung durch Äthanol-Fällung gestoppt.

Die Pellets werden in 6 µl Formamid-Puffer gelöst und im wesentlichen wie Proben von DNA-Sequenzierungs-Reaktionen auf einem 6 % Acrylamidgel, welches 8 M Harnstoff enthält, aufgetrennt (F. Sanger et al., Proc. Hat. Acad. Sci. 74, 5463-5467 (1979)).

Zur Autoradiographie wird das getrocknete Gel einem DuPont Cronex® X-ray Film unter Verwendung des Kodak Lanex Regular® Intensivators bei -70° C ausgesetzt.

Legende zur Abbildung 10

Die Spuren A bis C stellen die Kontrollen dar.

Spur A: 20 µg tRNA
Spur B: 10 µg RNA aus pER 33 (E. coli - Expression-Stamm für Interferon-α2-Arg)
Spur C: 1 ng RNA aus pRHW 12 (E. coli-Expression-Stamm für Interferon-omega 1)
Spur D: 50 µg RNA aus unbehandelten Namalwa Zellen
Spur E: 50 µg RNA aus mit Virus infizierten Namalwa Zellen
Spur F: 50 µg RNA aus mit Dexamethason vorbehandelten und mit Virus infizierten Namalwa Zellen
Spur G: 20 µg RNA aus unbehandelten NC 37 Zellen.
Spur H: 20 µg RNA aus mit Virus infizierten NC 37 Zellen
Spur I: 20 µg RNA aus mit Dexamethason vorbehandelten und mit Virus infizierten NC 37-Zellen
Spur M: Größenmarkierung (pBR 322 geschnitten mit Hinfl).

Spuren B und C zeigen, daß das erwartete Signal nur dann nachgewiesen werden kann, wenn sich eine omegal-spezifische RNA unter den RNA-Molekülen befindet. Ferner wird gezeigt, daß selbst ein großer Überschuß der falschen RNA kein Hintergrundsignal hervorruft (siehe Spur B). Ferner ergibt auch die als Hybridisierungspartner verwendete tRNA kein Signal (siehe Spur A).

Die Spuren G bis I zeigen die Induktion der omegal-spezifischen RNA in mit Virus infizierten NC 37-Zellen. Die Vorbehandlung mit Dexamethason verstärkt hierbei diesen Effekt.

Die Spuren D bis F zeigen grundsätzlich das gleiche Ergebnis, das mit Namalwa-Zellen erhalten wird. Die Induktion mit omegal-spezifischen RNA ist jedoch nicht so stark wie bei den NC 37-Zellen. Dieses Resultat ist parallel mit den Interferon-Titern, die im jeweiligen Zellüberstand gemessen wurden.

Dieses Verhalten der Interferon-omegal-Gen Expression ist also so, wie es von einem Interferon-TypI-Gen zu erwarten war.

Beispiel 8

Isolierung des Gens kodierend für IFN-omegal bzw. dazu verwandter Gene:

a) Cosmid Screening

Eine humane Cosmidbank (menschliche DNA (männlich)) kloniert im Cosmidvektor pcos2 EMBL (A. Ponstka, H.-R. Rockwitz, A.-M. Frischauf, B. Hohn, H. Lehrach Proc. Natl. Acad. Sci. 81, 4129-4133 (1984)) mit einer Komplexität von 2 x $10^6$) wurde nach IFN-omega- bzw. dazu verwandten Genen durchsucht. E. coli DHI ($r_K$-, $m_K$+, rec.A; gyrA96, sup.E) wurde als Wirt verwendet. Es wurden zunächst $Mg^{++}$-Zellen (= "plating bacteria") hergestellt. E.coli DHI wächst über Nacht in L-Broth (10 g/l Trypton, 5 g/l Yeast-Extract, 5 g/l NaCl) supplementiert mit 0,2 % Maltose. Die Bakterien werden abzentrifugiert und in einer 10 mM $MgSO_4$ Lösung zu einer $OD_{600}$ = 2 aufgenommen. 5 ml dieser Zellsuspension werden mit 12,5 x $10^6$ colony forming units verpackter Cosmide 20 Minuten bei 37° C inkubiert. Anschließend werden 10 vol LB zugesetzt und die Suspension 1 Stunde zwecks Expression der durch das Cosmid vermittelten Kanamycinresistenz bei 37° C gehalten. Dann werden die Bakterien abzentrifugiert, in 5 ml LB resuspendiert und in 200 µl Aliqots auf Nitrozellulosefilter ausgestrichen (BA85, Schleicher und Schüll, 132 mm Durchmesser), die auf LB-Agar (1,5 % Agar in L-Broth) plus 30 µg/ml Kanamycin aufliegen. Pro Filter wachsen ca. 10.000 bis 20.000 Kolonien an. Die Kolonien werden auf weitere Nitrozellulosefilter replika-plattiert, die bei 4° C aufbewahrt werden.

Ein Satz der Kolonien-Filter wird wie in Beispiel 1c) beschrieben prozessiert, d.h. die Bakterien werden denaturiert, und die Einzelstrang-DNA an der Nitrozellulose fixiert. Die Filter werden 4 Stunden bei 65° C in einer 50 mM Tris/HCl, pH=8,0, 1 M NaCl, 1 mM EDTA, 0,1 % SDS Lösung vorgewaschen. Anschließend werden die Filter in einer 5 x Denhardt's (siehe Beispiel 1c), 6 x SSC, 0,1 % SDS-Lösung 2 Stunden bei 65° C inkubiert und mit ca. 50 x $10^6$ cpm nicktranslatierter, denaturierter IFN-omegal DNA (HindIII-BamHI Insert des Klons pRHW12, siehe Abbildung 6) 24 Stunden bei 65° C in derselben Lösung hybridisiert. Nach erfolgter Hybridisierung werden die Filter zunächst 3 x 10 Minuten bei Raumtemperatur in einer 2 x SSC, 0,01 % SDS-Lösung und anschließend 3 x 45 Minuten bei 65° C in einer 0,2 x SSC, 0,01 % SDS-Lösung gewaschen. Die Filter werden getrocknet und auf Kodak X-Omat S Film unter Verwendung einer Verstärkerfolie bei -70° C exponiert. Positiv reagierende Kolonien werden auf den Replika-Filter lokalisiert, abgekratzt und in L-Broth + Kanamycin (30 µg/ml) resuspendiert. Von dieser Suspension werden einige µl auf LB-Agar + 30 µg/ml Kanamycin ausplattiert. Die resultierenden Kolonien werden auf Nitrozellulosefilter replika-plattiert. Diese Filter werden wie oben beschrieben mit $^{32}$P-markierter IFN-omegal-DNA hybridisiert. Von jeweils einer hybridisierenden Kolonie wurde mittels der von Birnboim & Doly (Nucl. Acids Res. 7, 1513 (1979)) beschriebenen Methode das Cosmid isoliert. Mit dieser Cosmid DNA Präparation wurde E. coli DHI transformiert und die Transformanten auf LB-Agar + 30 µg/ml Kanamycin selektioniert. Die Transformanten wurden wieder mit $^{32}$P-radioaktiv markierter IFN-omegal DNA auf positiv reagierende Klone geprüft. Jeweils ein Klon ausgehend vom ursprünglichen Isolat wird ausgewählt und dessen Cosmid in größerem Maßstab hergestellt (Clewell, D.B. und Helinski, D.R., Biochemistry 9, 4428 (1970)). Drei der isolierten Cosmide tragen die Namen cos9, cos10 und cosB.

b) Subklonieren hybridisierender Fragmente in PUC8

Jeweils 1 µg der Cosmide cos9, cos10 und cosB wurden mit HindIII unter den vom Hersteller (New England Biolabs) empfohlenen Bedindungen geschnitten. Die Fragmente werden auf 1 % Agarosegelen in TBE-Puffer elektrophoretisch aufgetrennt und nach Southern auf Nitrozellulosefilter transferiert (Beispiel 4c). Beide Filter werden wie in Beispiel 4d) beschrieben mit nicktranslatierter omegal-DNA hybridisiert, gewaschen und exponiert. Etwa 20 µg von jedem Cosmid werden mit HindIII geschnitten, und die entstehenden Fragmente gelelektrophoretisch aufgetrennt. Die im Vorversuch mit omegal-DNA hybridisierenden Fragmente werden elektroeluiert und über Elutip®-Säulen (Schleicher + Schüll) gereinigt. Diese Fragmente werden mit HindIII-linearisiertem, dephosphoryliertem pUC8 (Messing, J., Vieira, J., Gene 19, 269-276 (1982)) ligiert. E. coli JM101 (supE, thi, (lac-proAB), [F', traD36, pro AB, lac q Z M15] (z.B. Fa. P.L. Biochemicals) wird mit der Ligasereaktionslösung transformiert. Die Bakterien werden auf LB-Agar, enthaltend 50 µg/ml Ampicillin, 250 µg/ml 5-Brom-4-chlor-3-indolyl-β-D-galaktopyranosid (BCIG, Sigma) und 250 µg/ml Isopropyl-β-D-thiogalakto-pyranosid (IPTG, Sigma), ausgestrichen. Eine Blaufärbung der entstehenden Kolonien zeigt das Fehlen eines Inserts in pUC8 an. Von einigen weißen Klonen wurden die Plasmid DNA's in kleinem Maßstab isoliert, mit HindIII geschnitten und auf 1 % Agarosegelen aufgetrennt. Die DNA Fragmente wurden auf Nitrozellulosefilter übertragen und wie oben mit $^{32}$P-omegal-DNA hybridisiert. Ausgehend von cos9 und cos10 wurde jeweils ein Subklon ausgewählt und mit pRHW22 bzw. pRH57 bezeichnet. Von cosB wurden zwei mit der omegal-Probe gut hybridisierende DNA Fragmente subkloniert und mit pRH51 bzw. pRH52 bezeichnet.

c) Sequenzanalyse

Die in PUC8 insertierte DNA wird durch Schnitte mit HindIII und anschließende Gelelektrophorese vom Vektorteil getrennt. Diese DNA, ca. 10 µg, wird unter Verwendung von $T_4$ DNA Ligase in 50 µl Reaktionslösung ligiert, das Volumen auf 250 µl mit Nicktranslationspuffer gebracht (Beispiel 4d) und anschließend unter Eiskühlung mittels Ultraschall zerbrochen (MSE 100 Watt Ultrasonic Disintegrator, max. Abgabe bei 20 kHz, fünfmal 30 Sekunden). Hernach werden die Enden der Bruchstücke durch Zugabe von 1/100 vol je 0,5 mM dATP, dGTP, dCTP und dTTP sowie 10 units Klenowfragment der DNA-Polymerase I zwei Stunden bei 14° C repariert. Die resultierenden, gerade Enden aufweisenden DNA-Fragmente werden ihrer Größe nach auf einem 1 % Agarosegel aufgetrennt. Fragmente der Größen zwischen 500 und 1000 bp wurden isoliert und in den mit SmaI geschnittenen, dephosphorylierten Phagenvektor M13 mp8 subkloniert. Die Einzelstrang DNA rekombinanter Phagen wird isoliert und nach der von Sanger entwickelten Methode (Sanger, F. et al., Proc. Natl. Acad. Sci 74, 5463-5467 (1976)) sequenziert. Das Zusammensetzen der Einzelsequenzen zu der Gesamtsequenz erfolgt mittels Computer (Staden, R., Nucl. Acids Res. 10, 4731-4751 (1982)).

d) Sequenz des Subklons pRH57 (IFN-omegal)

Die Sequenz ist in Abbildung 11 wiedergegeben. Dieses 1933 bp lange Fragment enthält das Gen für Interferon-omegal. Die für Protein kodierende Region umfaßt die Nukleotide 576 bis 1163. Die Sequenz ist völlig identisch mit der des cDNA Klons P9A2. Der Nukleotidabschnitt 576 bis 674 kodiert für ein 23 Aminosäure langes Signalpeptid. Die TATA-Box liegt in einem für Interferon Typ I Gene charakteristischen Abstand vor dem Startcodon ATG (Positionen 476-482). Das Gen weist einige Signalsequenzen für die Polyadenylierung bei der Transkription auf (ATTAAA auf Positionen 1497-1502, bzw. 1764-1796; AATAAA auf Positionen 1729-1734 bzw. 1798-1803), deren erste im Klon P9A2 verwendet wurde.

e) Sequenz des Subklons pRHW22 (IFN-pseudo-omega2)

In Abbildung 12 ist die 2132 bp lange Sequenz des mit der omegal-DNA Probe hybridisierenden HindIII Fragments aus dem Cosmid cos 9 wiedergegeben. Es ergibt sich ein offener Leserahmen von Nukleotid 905 bis zu Nukleotid 1366. Die daraus abzuleitende Aminosäuresequenz ist wiedergegeben. Die ersten 23 Aminosäuren sind denen des Signalpeptids eines typtischen Typ I Interferons ähnlich. Die nachfolgenden 131 Aminosäuren zeigen bis zur Aminosäure 65 Ähnlichkeit zum omegal-Interferon, wobei Tyrosin als erste Aminosäure des reifen Proteins bemerkenswert ist.

Auf Aminosäureposition 66 folgt die Sequenz einer potentiellen N-Glykosylierungsstelle (Asn-Phe-Ser). Ab dieser Stelle wird die Aminosäuresequenz von der eines Typ 1 Interferons verschieden. Es läßt sich jedoch zeigen, daß durch geeignete Insertionen und daraus resultierendem Verschieben der Proteinleserahmen Ähnlichkeit zum IFN-omegal herstellen läßt (siehe Beispiel 9). Es handelt sich bei dem isolierten Gen daher vom Standpunkt der Typ I Interferone her gesehen um ein Pseudogen: IFN-pseudo-omega2.

f) Sequenz des Subklons pRH51 (IFN-pseudo-omega3)

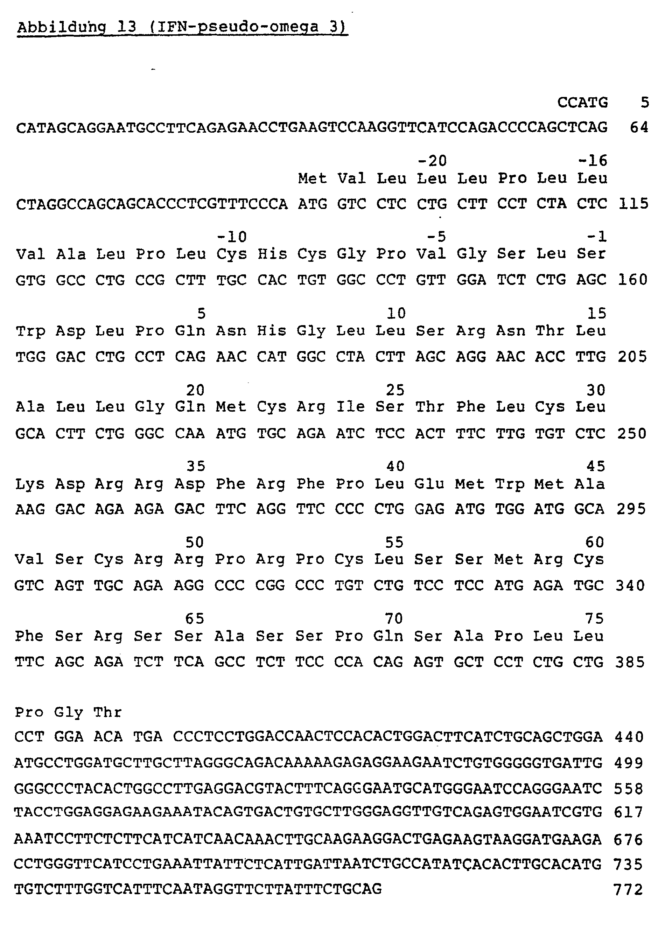

Das eine, aus dem Cosmid B stammende, etwa 3.500 bp lange und mit der omegal-Probe hybridisierende HindIII Fragment wurde teilweise sequenziert (Abbildung 13). Es ergibt sich ein offener Leserahmen von Nukleotidposition 92 bis 394. Die ersten 23 Aminosäuren zeigen die Merkmale eines Signalpeptids. Die daran anschließende Sequenz beginnt mit Tryptophan und zeigt Ähnlichkeit zu IFN-omegal bis Aminosäure 42. Danach wird die abgeleitete Sequenz unterschiedlich zu IFN-omegal und endet nach der Aminosäure 78. Die Sequenz läßt sich durch Insertionen so verändern, daß sie dann große Homologie zu IFN-omegal aufweist (Beispiel 9). Das Gen wird mit IFN-pseudo-omega3 bezeichnet.

g) Sequenz des Inserts von pRH52 (IFN-pseudo-omega4)

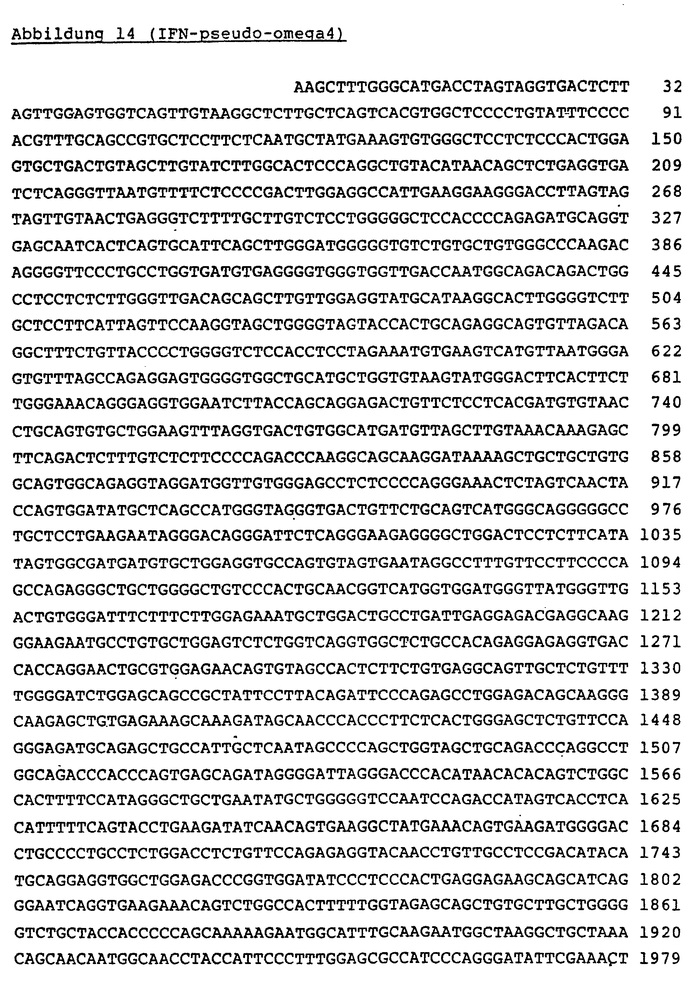

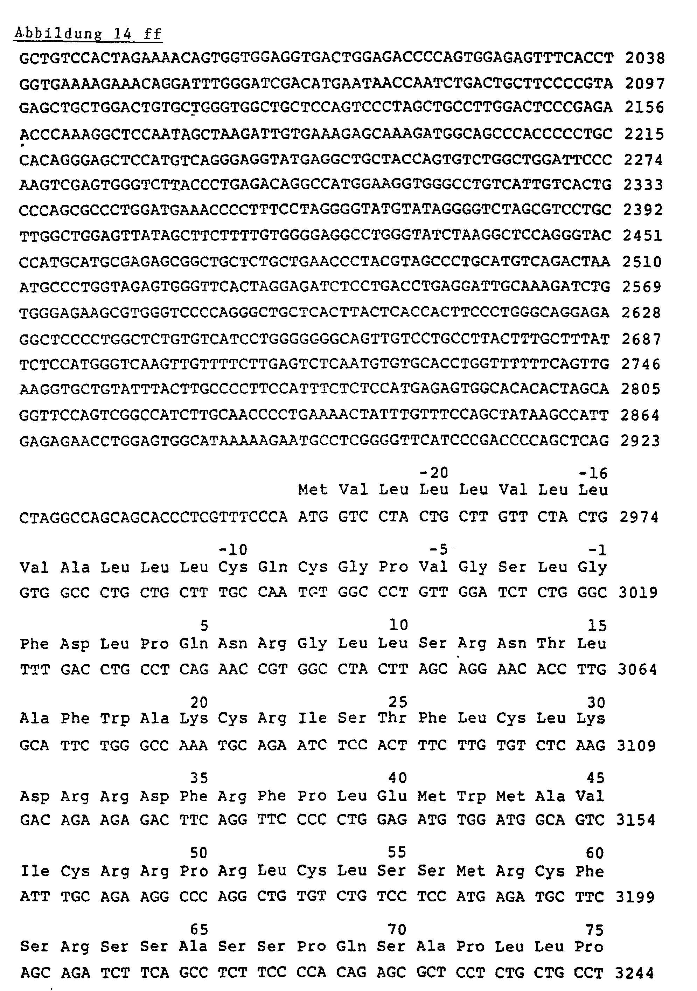

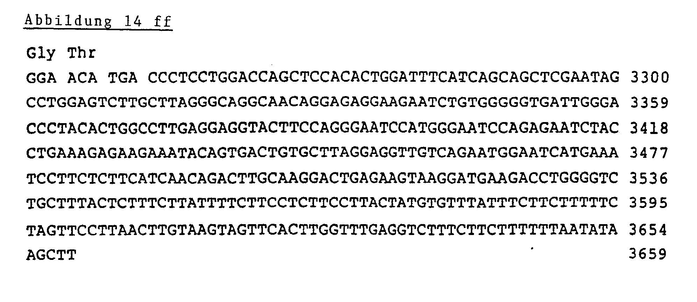

Die 3659 lange, aus dem Cosmid B isolierten und mit omegal-DNA hybridisierenden Sequenz des HindIII Fragments ist in Abbildung 14 dargestellt. Ein offener Leserahmen, dessen Translationsprodukt teilweise Homologie zu IFN-omegal zeigt, befindet sich zwischen Nukleotidposition 2951 und 3250. Nach einem 23 Aminosäure langen Signalpeptid beginnt die weitere Aminosäuresequenz mit Phenylalanin. Die Homologie zu IFN-1 wird bereits nach der 16. Aminosäure unterbrochen, setzt sich bei der 22. Aminosäure fort und endet mit der 41. Aminosäure. Eine eventuelle Translation wäre bis zur, Aminosäure 77 möglich. Analog zu Beispiel 8f) und 8g) läßt sich auch hier eine gute Homologie zu IFN-omegal durch Einführen von Insertionen herstellen (Beispiel 9). Das hier isolierte Pseudogen wird als IFN-pseudo-omega4 bezeichnet.

Beispiel 9

Auswertung der Gene für 4 Mitglieder der IFN-omega-Familie

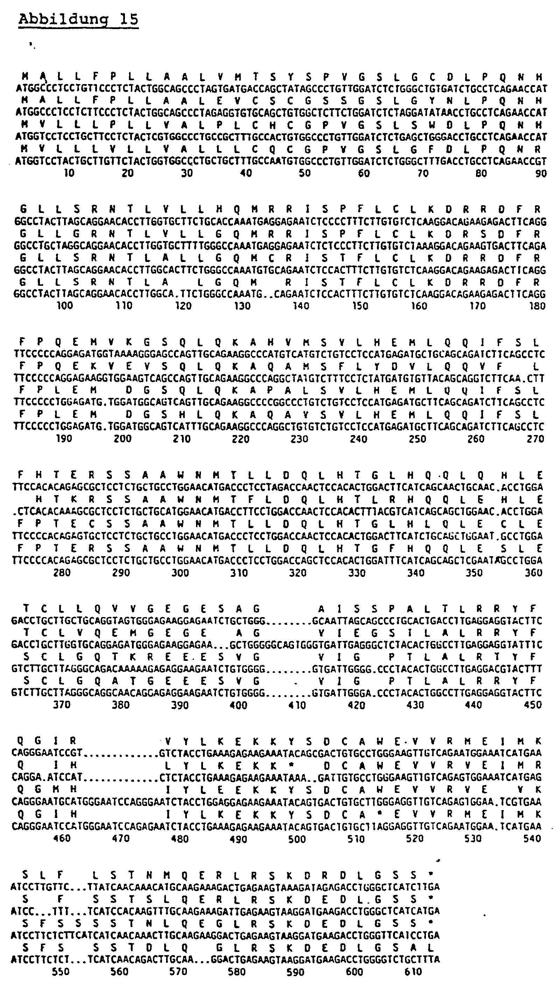

In Abbildung 15 sind die Gene für IFN-omegal bis IFN-pseudo-omega4 untereinander zusammen mit der Aminosäureübersetzung aufgelistet. Zur besseren Übereinstimmung werden Lücken in die einzelnen Gene eingefügt, die durch Punkte dargestellt sind. Es werden keine Basen weggelassen. Die Basennummerierung schließt die Lücken mit ein. Die Aminosäureübersetzung von IFN-omegal wird beibehalten (z.B. auf Position 352-355: "C.AC" kodiert für His). Bei den Pseudogenen erfolgt Übersetzung in eine Aminosäure nur dort, wo sie eindeutig möglich ist. Auf Grund dieser Auflistung ist sofort erkennbar, daß die 4 isolierten Gene untereinander verwandt sind. So ist z.B. die potentielle N-Glykosylierungsstelle (Nukleotidpositionen 301 bis 309) in allen 4 Genen erhalten. Ebenso findet sich außer bei IFN-pseudo-omega4 an den Nukleotidpositionen 611 bis 614 ein Triplett, daß ein Stopcodon darstellt, und welches bei IFN-omegal ein reifes Protein der Länge von 172 Aminosäuren beendet. Allerdings existieren in dieser Anordnung bei IFN-pseudo-omega2 (Nukleotidpositionen 497 bis 499) und bei IFN-pseudo-omega4 (Nukleotidpositionen 512 bis 514) vorzeitige Stopcodons.

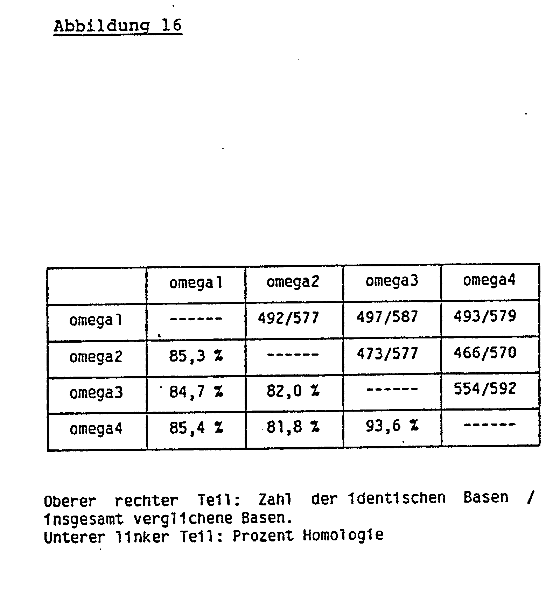

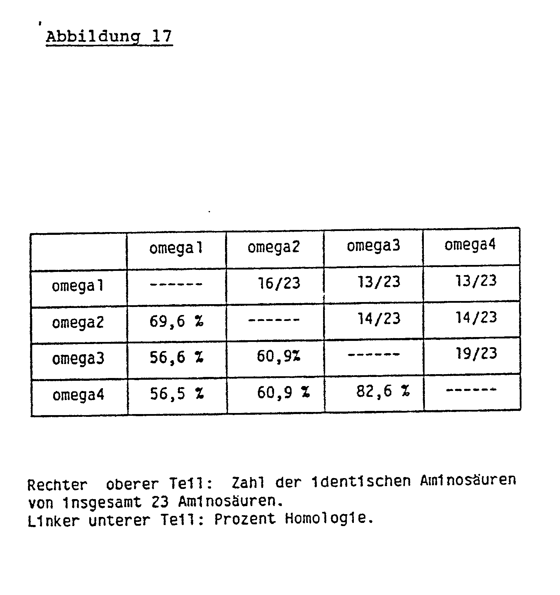

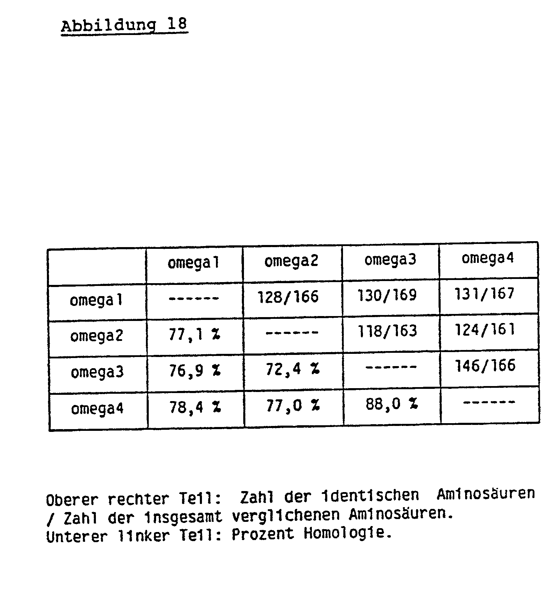

Der Verwandtschaftsgrad zwischen den Genen bzw. den Aminosäuretranslationen läßt sich aus der in Abbildung 15 getroffenen Anordnung berechnen. Abbildung 16 gibt die DNA Homologien zwischen den Mitgliedern der IFN-omega-Familie wieder. Dabei werden beim paarweisen Vergleich jene Positionen nicht mitgezählt, an denen einer der beiden Partner oder beide, eine Lücke aufweisen. Der Vergleich ergibt eine Homologie von rund 85 % zwischen IFN-omegal-DNA und den Sequenzen der Pseudogene. IFN-pseudo-omega2-DNA ist zu den DNA's von IFN-pseudo-omega3 bzw. IFN-pseudo-omega4 zu etwa 82 % homolog. Abbildung 17 zeigt die Ergebnisse der Vergleiche der Signalsequenzen und Abbildung 18 jene der Vergleiche der "reifen" Proteine. Die letzteren bewegen sich zwischen 72 und 88 %. Diese Homologie ist jedoch wesentlich größer als jene zwischen IFN-omegal und den IFN-α's und IFN-β (Beispiel 6). Der Umstand, daß die einzelnen Mitglieder der IFN-omega-Familie weiter voneinander entfernt sind als die der IFN-α-Familie untereinander, läßt sich dadurch erklären, daß drei der vier isolierten IFN-omega-Gene Pseudogene sind und offenbar nicht mehr jenem Selektionsdruck wie funktionelle Gene unterliegen.

Beispiel 10

Fermentation

Stammhaltung:

Eine Einzelkolonie des Stammes HB101/pRHW12 auf LB-Agar (mit 25 mg/ml Ampicillin) wird in Trypton-Soya-Broth (OXDID CM129) mit 25 mg/ml Ampicillin überimpft und bei 250 Umdrehungen pro Minute (U/min.) und 37° C geschüttelt bis eine optische Dichte (546 nm) von ca. 5 erreicht ist (logarithmische Wachstumsphase). Daraufhin werden 10 Gewichtsprozent steriles Glycerin zugesetzt, die Kultur in 1,5 ml Portionen in sterile Ampullen abgefüllt und bei -70° C eingefroren.

Vorkultur:

Das Kulturmedium enthält 15 g/l $Na_2HPO_4$ x $12H_2O$; 0,5 g/l NaCl; 1,0 g/l $NH_4Cl$; 3,0 g/l $KH_2PO_4$; 0,25 g/l $MgSO_4$ x $7H_2O$; 0,011 g/l $CaCl_2$; 5 g/l Caseinhydrolysat (Merck 2238); 6,6 g/l Glucose-Monohydrat; 0,1 g/l Ampicillin; 20 mg/l Cystein und 1 mg/l Thiamin-hydrochlorid. 4 x 200 ml dieses Mediums in je einem 1000 ml Erlenmeyerkolben werden mit je 1 ml einer Stammkultur von HB101/pRHW14 beimpft und 16 bis 18 Stunden bei 37° C und 250 Umdrehungen pro Minute geschüttelt.

Hauptkultur:

Das Medium für die Fermentation enthält 10 g/l $(NH_4)_2HPO_4$; 4,6 g/l $K_2HPO_4$ x $3H_2O$; 0,5 g/l NaCl; 0,25 g/l $MgSO_4$ x $7H_2O$; 0,011 g/l $CaCl_2$; 11 g/l Glucose-Monohydrat; 21 g/l Caseinhydrolysat (Merck 2238); 20 mg/l Cystein, 1 mg/l Thiamin-hydrochlorid und 20 mg/l 3-β-Indolacrylsäure. 8 Liter steriles Medium in einem Fermenter mit 14 Litern Totalvolumen (Höhe: Radius = 3:1) werden mit 800 ml der Vorkultur inoculiert.

Die Fermentation erfolgt bei 28° C, 1000 U/min. (Effigas-Turbine), einer Belüftungsrate von 1vvm (Volume/Volume/Minute) und einem Start-pH von 6,9. Während der Fermentation sinkt der pH-Wert ab und wird dann bei 6,0 mit 3N NaOH konstant gehalten. Ist eine optische Dichte (546 nm) von 18 bis 20 erreicht (gewöhnlich nach 8,5 bis 9,5 Stunden Fermentationszeit), wird unter sonst gleichen Bedingungen auf 20° C abgekühlt und anschließend mit 6N $H_2SO_4$ (ohne Belüftung) der pH-Wert auf 2,2 eingestellt und 1 Stunde bei 20° C und 800 U/min. (ohne Belüftung) gerührt. Die auf diese Weise inaktivierte Biomasse wird daraufhin in einer CEPA-Laboratoriumszentrifuge Typ GLE bei 30 000 U/min. abzentrifugiert und bei -20° C eingefroren und aufbewahrt.

Beispiel 11

Reinigung des Interferon-omega-Gly

a) Teilweise Reinigung

Alle Stufen werden bei 4° C durchgeführt.

140 g Biomasse (E.coli HB101 transformiert mit dem Expressionsklon pRHW12) werden in 1150 ml 1 %iger Essigesäure (vorgekühlt auf 4° C) resuspendiert und 30 Minuten gerührt. Die Suspension wird mit 5 M NaOH auf pH = 10,0 gestellt und weitere 2 Stunden gerührt. Nach dem Einstellen mit 5 M HCl auf pH = 7,5 wird weitere 15 Minuten gerührt und dann zentrifugiert (4° C, 1 Stunde 10.000 U/min, J21 Zentrifuge (Beckman), JA10-Rotor).

Die klare, überstehende Lösung wird auf eine 150 ml CPG(controlled pore glass)-Säule (CPG 10-350, Mesh Size 120/200) mit 50 ml/h aufgetragen, mit 1000 ml 25 mM Tris/ 1M NaCl, pH 7,5 nachgewaschen und mit 25 mM Tris/1M KCNS/50% Äthylenglykol, pH = 7,5 eluiert (50 ml/h).

Der Interferonaktivität enthaltende Proteinpeak wurde gesammelt, gegen 0,1 M Na-Phosphat, pH = 6,0 und 10 % Polyäthylenglykol 40.000 über Nacht dialysiert, und das entstandene Präzipitat abzentrifugiert (4° C, 1 Stunde, 10.000 U/min, J21-Zentrifuge, JA20-Rotor) (siehe Tabelle 1).

b) Weitere Reinigung

Das dialysierte und konzentrierte CPG-Eluat wird mit Puffer A (0,1 M Na-Phosphat pH = 6,25/25 % 1,2-Propylenglykol) 1:5 verdünnt und über ein "Superloop"-Injektionsgerät (Pharmacia) mit 0,5 ml/min auf die mit Puffer A äquilibrierte MonoS® 5/5(Pharmacia, Kationenaustauscher)-Säule aufgetragen. Die Elution erfolgt durch 20 ml eines linearen Gradienten von 0 auf 1 M NaCl in Puffer A ebenfalls bei einer Flußrate von 0,5 ml/min. Der Durchlauf und die 1 ml Fraktionen wurden gesammelt und mittels eines CPE-Reduktionstests auf Interferonaktivität getestet. Die aktiven Fraktionen wurden gesammelt.

Tabelle 1

| | Volumen (ml) | Biologischer Test U/ml+ | U total | Protein (mg/ml) | total (mg) | U/mg | Ausbeute % |
|---|---|---|---|---|---|---|---|
| roh | 1150 | 15000 | $17,3 \times 10^6$ | 3,6 | 4140 | 4180 | 100 |
| DL | 2200 | < 600 | $< 1,3 \times 10^6$ | 0,74 | 1628 | <600 | < 5 |
| Eluat | 124,3 | 170000 | $21,0 \times 10^6$ | 16,8 | 2088 | 10000 | 121 |
| nach Dialyse | 41 | 300000 | $12,3 \times 10^6$ | 12,6 | 516,6 | 23800 | 71 |

\+ CPE-Reduktionstest: A549 Zellen, EMC-Virus

U: Einheiten bezogen auf Interferon-α2 (siehe Beispiel 2 der EP-A-0.115.613: E. Coli HB101 transformiert mit dem Expressionsklon pER 33) als Standard

DL: Durchlauf

Beispiel 12

Charakterisierung von HuIFN-omega1

A. Antivirale Aktivität auf Humanzellen
B. Antivirale Aktivität auf Affenzellen
C. Antiproliferative Aktivität auf humanen Burkitt's Lymphom-Zellen (Zellinie Daudi)
D. Antiproliferative Aktivität auf humanen Cervixcarcinom-Zellen (Zellinie HeLa)
E. Säurestabilität
F. Serologische Charakterisierung

A. Antivirale Aktivität auf Humanzellen

Zellinie: Humane Lungenkarzinom-Zellinie A549 (ATCC CCL 185)
Virus: Maus Encephalomyocarditis-Virus (EMCV)
Testsystem: Hemmung des cytopathischen Effekts (alle Titrationen wurden vierfach durchgeführt)
Ein partiell gereinigtes Präparat von HuIFN-omega1 mit einem Proteingehalt von 9.4 mg/ml wurde in einer geeigneten Verdünnung im genannten Testsystem titriert. Das Präparat zeigte eine antivirale Wirkung mit einer spezifischen Aktivität von 8300 IE/mg bezogen auf den Referenzstandard Go-23-901-527.

B. Antivirale Aktivität auf Affenzellen

Zellinie: GL-V3 Affennierenzellen (Christofinis G.J., J. Med. Microbiol. 3, 251-258, 1970)
Virus: Vesicular Stomatitis Virus (VSV)
Testsystem: Plaque-Reduktions-Test (alle Titrationen vierfach)
Das in Beispiel 12A beschriebene Präparat zeigte eine spezifische antivirale Aktivität von 580 E/mg im genannten Testsystem.

C. Antiproliferative Aktivität auf humanen Burkitt's Lymphom-Zellen (Zellinie Daudi)

Die humae Burkitt's Lymphom-Zellinie Daudl wurde in Gegenwart verschiedener Konzentrationen von HuIFN-omega1 gezüchtet. Nach zwei, vier und sechs Tagen Inkubation bei 37°C wurde die Zelldichte bestimmt; unbehandelte Kulturen dienten als Kontrollen. Alle Kulturen wurden dreifach parallel angesetzt. Aus der nachfolgenden Abbildung geht hervor, daß IFN-omega eine ausgeprägte Hemmung der Zellproliferation bei einer Konzentration von 100 antiviralen Einheiten (IE, siehe Beispiel 12A) pro ml zeigt; bei einer Konzentration von 10 IE/ml war eine partielle, transiente Hemmung zu beobachten (Folgende Symbole werden in der nachfolgenden Abbildung verwendet: ○ Kontolle, □ 1 IE/ml, ▫ 10 IE/ml, ▾ 100 IE/ml):

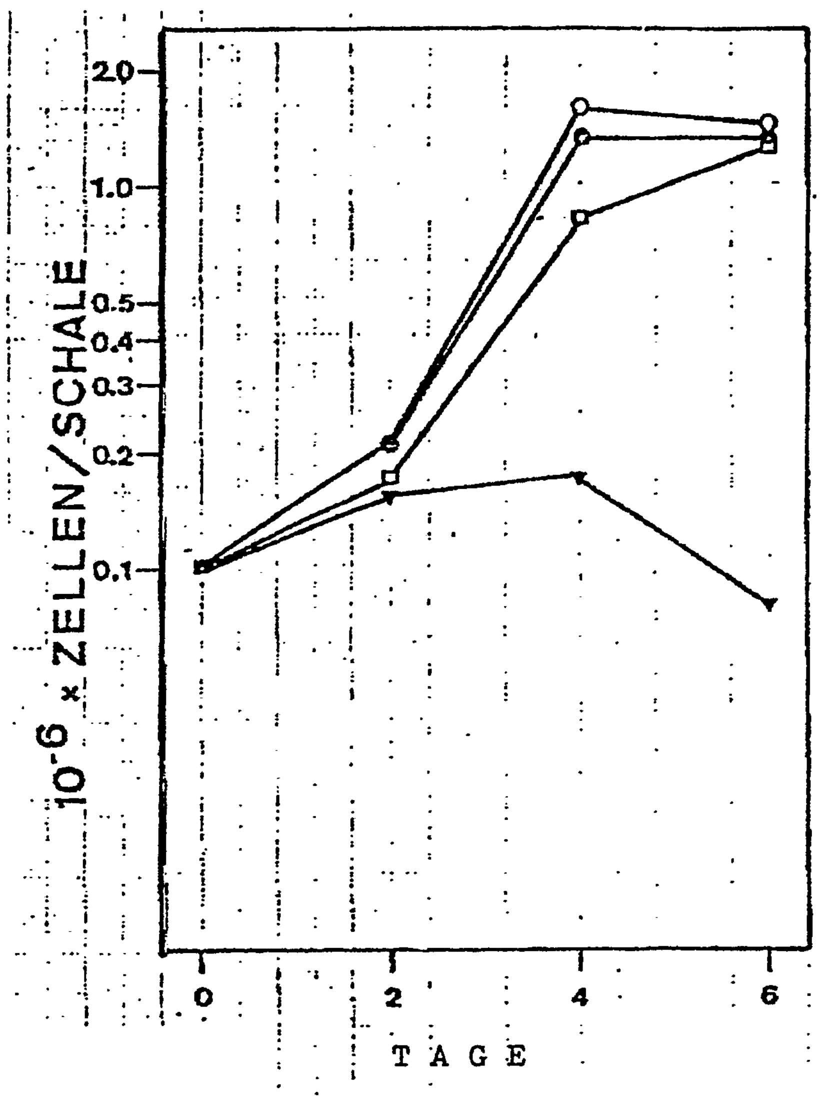


D. Antiproliferative Aktivität auf humanen Cervixcarcinom-Zellen (Zellinie HeLa)

Die humane Cervixcarcinom-Zellinie HeLa wurde in Gegenwart folgender Proteine bzw. Proteinmischungen gezüchtet:
HuIFN-omegal (siehe Beispiel 12A) 100 IE/ml
HuIFN-gamma (siehe Beispiel 12A) 100 IE/ml
Humaner Tumor-Nekrose-Faktor (HuTNF),≧98 % rein, hergestellt von Genentech Inc., San Franzisco, USA (siehe Pennica D. et al., Nature 312, 724-729; 1984) 100 ng/ml
Alle binären Kombinationen der genannten Proteine Konzentrationen wie oben
Je 2 Kulturen wurden in 3 cm-Kunststoff-Gewebekulturschalen angesetzt (50 000 Zellen/Schale) und sechs Tage bei 37° C inkubiert; anschließend wurde die Zelldichte bestimmt. Behandlung der Zellen mit HuIFN-omegal oder HuINF-gamma hatte nur geringen Einfluß auf das Zellwachstum, während HuIFN-gamma einen deutlichen cytostatischen Effekt zeigte. Kombinationen von IFN-gamma mit IFN-omegal zeigten synergistische cytostatische/cytotoxische Wirkung.

Die nachfolgende Abbildung zeigt das Ergebnis des Versuchs:

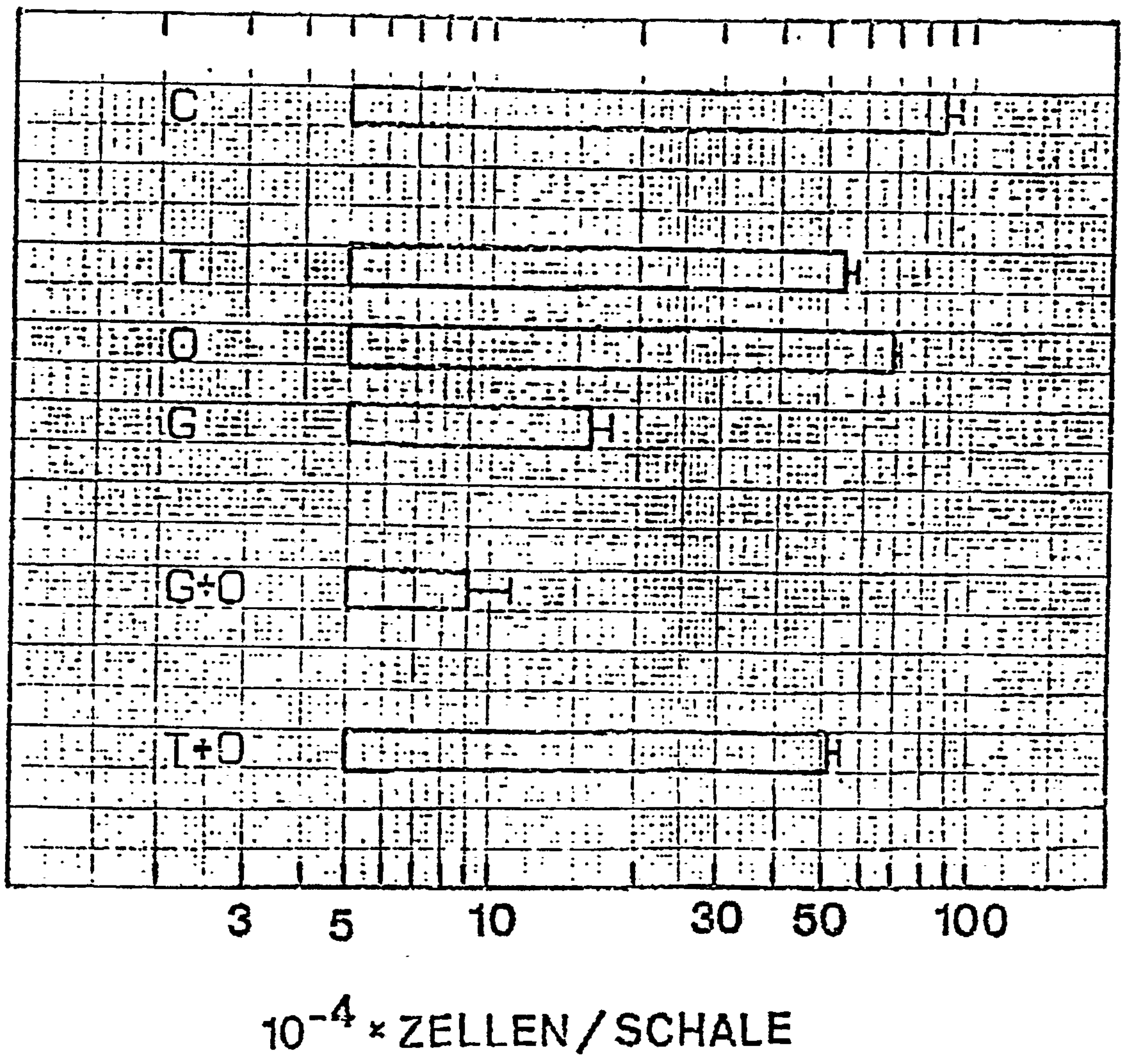


Folgende Symbole werden in der obigen Abbildung verwendet:
C unbehandelte Kontrolle, T HuTNF, O HuIFN-omega1, G HuIFN-gamma.

E. Säurestabilität

Zur Untersuchung der Säurestabilität von HuIFN-omegal wurde eine Verdünnung des in Beispiel 12A genannten Präparates in Zellkulturmedium (U/min.I 1640 mit 10 % fötalem Kalbsserum) mit Hilfe von HCl auf pH 2 eingestellt, 24 Stunden bei 4° C inkubiert und anschließend mit NaOH neutralisiert. Diese Probe wurde im antiviralen Test titriert (siehe Beispiel 12A); sie zeigte 75 % der biologischen Aktivität einer bei neutralem pH inkubierten Kontrolle. IFN-omegal ist somit als säurestabil zu bezeichnen.

F. Serologische Charakterisierung

Zur Bestimmung der serologischen Eigenschaften von HuIFN-omegal im Vergleich zu HuIFN-α2 wurden Proben (verdünnt auf 100 IE/ml) mit gleichen Volumina von Lösungen monoklonaler Antikörper oder polyklonaler Antiseren 90 Minuten bei 37° C vorinkubiert; anschließend wurde die antivirale Aktivität der Proben bestimmt. Die nachfolgende Tabelle zeigt, daß HuIFN-omegal nur durch ein Antiserum gegen Leukocyteninterferon in relativ hoher Konzentration neutralisiert werden kann, nicht aber durch Antikörper, die gegen HuIFN-α2 oder HuIFN-beta gerichtet sind. HuIFN-omegal ist somit weder mit HuIFN-α2 noch mit HuIFN-beta serologisch verwandt:

| Antiserum monokl. Antikörper | Verdünnung µg/ml | Neutralisierung von HuIFN-α2 | HuIFN-omega1 |
|---|---|---|---|
| EBI-1[1)] | 1 | + | - |
| | 10 | +++ | - |
| | 100 | +++ | - |
| | 1000 | - | O |
| EBI-3[1)] | 1 | +++ | - |
| | 10 | +++ | - |
| | 100 | +++ | - |
| | 1000 | +++ | O |
| L3B7[2)] | 100 | +++ | O |
| | 1000 | +++ | O |
| Schaf-anti-Leukocyten-IFN[3)] | 1:50 000 | +++ | - |
| | 1: 5 000 | +++ | O |
| | 1: 500 | +++ | + |
| | 1: 50 | - | +++ |
| Kaninchen-anti-HuIFN-α2 | 1: 1 000 | +++ | - |
| | 1: 100 | +++ | O |
| | 1: 10 | - | O |
| Schaf-anti-HuIFN-ß[4)] | 1: 50 | - | O |

1) = siehe EP-A-0.119.476
2) = A. Berthold et al. in Arzneimittelforschung 35, 364-369 (1985)
3) = Research Reference Reagent Catalog No. G-026-502-568
4) = Research Reference Reagent Catalog No. G-028-501-568
Research Resources Branch, National Institute of Allergy and Infectious Diseases, Bethesda, Maryland, USA.

Symbole: - nicht getestet, O keine Neutralisation, + partielle Neutralisation, +++ vollständige Neutralisation

## Patentansprüche

1. Rekombinante DNA-Moleküle, enthaltend eine codierende Sequenz für neue menschliche Interferon-Proteine vom Typ I, welche 168 - 174 Aminosäuren enthalten, für Interferon-pseudo-omega2 (siehe Abbildung 12), Interferon-pseudo-omega3 (siehe Abbildung 13) oder Interferon-pseudo-omega4 (siehe Abbildung 14), dadurch gekennzeichnet, daß die codierende Sequenz unter stringenten Bedingungen, die es nur erlauben, eine Homologie, die größer als 85 % beträgt, zu erkennen, mit den Inserts in der Pst-1 Schnittstelle a) des Plasmids E76E9 in E. coli HB 101, hinterlegt bei der DSM unter der Nummer DSM 3003 oder b) des Plasmids P9A2 in E. coli HB 101, hinterlegt bei der DSM unter der Nummer DSM 3004 hybridisiert.

2. DNA-Molekül gemäß Anspruch 1, dadurch gekennzeichnet, daß die stringenten Bedingungen nur eine Homologie von mehr als 90 % nachweisen lassen.

3. DNA-Molekül gemäß den Ansprüchen 1 und 2, dadurch gekennzeichnet, daß die codierende Sequenz als Insert in der Pst-1 Schnittstelle a) des Plasmids E76E9 in E. coli HB 101, hinterlegt bei der DSM unter der Nummer DSM 3003 oder b) des Plasmids P9A2 in E. coli HB 101, hinterlegt bei der DSM unter der Nummer DSM 3004 vorhanden ist, oder eine degenerierte Variation dieser Inserts ist.

4. DNA-Molekül gemäß den Ansprüchen 1 bis 3, dadurch gekennzeichnet, daß das Vehikel ein Plasmid ist, replizierbar in Prokaryoten oder in Eukaryoten.

5. DNA-Molekül gemäß Anspruch 4, dadurch gekennzeichnet, daß es ein Expressionsvehikel ist, replizierbar in Mikroorganismen oder in Säugetierzellen.

6. DNA-Molekül gemäß Anspruch 5, enthaltend die Sequenz

```
TGT GAT CTG CCT CAG AAC CAT GGC CTA CTT AGC AGG AAC ACC TTG  45
GTG CTT CTG CAC CAA ATG AGG AGA ATC TCC CCT TTC TTG TGT CTC  90
AAG GAC AGA AGA GAC TTC AGG TTC CCC CAG GAG ATG GTA AAA GGG 135
AGC CAG TTG CAG AAG GCC CAT GTC ATG TCT GTC CTC CAT GAG ATG 180
CTG CAG CAG ATC TTC AGC CTC TTC CAC ACA GAG CGC TCC TCT GCT 225
GCC TGG AAC ATG ACC CTC CTA GAC CAA CTC CAC ACT GGA CTT CAT 270
CAG CAA CTG CAA CAC CTG GAG ACC TGC TTG CTG CAG GTA GTG GGA 315
GAA GGA GAA TCT GCT GGG GCA ATT AGC AGC CCT GCA CTG ACC TTG 360
AGG AGG TAC TTC CAG GGA ATC CGT GTC TAC CTG AAA GAG AAG AAA 405
TAC AGC GAC TGT GCC TGG GAA GTT GTC AGA ATG GAA ATC ATG AAA 450
TCC TTG TTC TTA TCA ACA AAC ATG CAA GAA AGA CTG AGA AGT AAA 495
GAT AGA GAC CTG GGC TCA TCT                                 516
```

7. DNA-Molekül gemäß Anspruch 5, dadurch gekennzeichnet, daß das DNA-Molekül gemäß Anspruch 6 anstelle des Nukleotids G in Position 332 das Nukleotid A enthält.

8. DNA-Molekül gemäß Anspruch 5, dadurch gekennzeichnet, daß es a) das Plasmid E76E9 in E. coli HB 101 hinterlegt bei der DSM unter der Nummer DSM 3003 oder b) das Plasmid P9A2 in E. coli HB 101, hinterlegt bei der DSM unter der Nummer DSM 3004 ist.

9. DNA-Molekül gemäß den Ansprüchen 6 und 7, dadurch gekennzeichnet, daß dieses zusätzlich die für das Leader-Peptid codierende DNA-Sequenz der Formel

ATG GCC CTC CTG TTC CCT CTA CTG GCA GCC CTA GTG ATG ACC AGC TAT AGC CCT GTT GGA TCT CTG GGC

enthält.

**10.** Interferon-omegal gene of formula

```
                                               GATCTGGTAAACCTGAA   17
GCAAATATAGAAACCTATAGGGCCTGACTTCCTACATAAAGTAAGGAGGGTAAAAATGG   76
AGGCTAGAATAAGGGTTAAAATTTTGCTTCTAGAACAGAGAAAATGATTTTTTTCATAT  135
ATATATGAATATATATTATATATACACATATATACATATATTCACTATAGTGTGTATAC  194
ATAAATATATAATATATATATATTGTTAGTGTAGTGTGTGTCTGATTATTTACATGCATAT  253
AGTATATACACTTATGACTTTAGTACCCAGACGTTTTTCATTTGATTAAGCATTCATTT  312
GTATTGACACAGCTGAAGTTTACTGGAGTTTAGCTGAAGTCTAATGCAAAATTAATAGA  371
TTGTTGTCATCCTCTTAAGGTCATAGGGAGAACACACAAATGAAAACAGTAAAAGAAAC  430
TGAAAGTACAGAGAAATGTTCAGAAAATGAAAACCATGTGTTTCCTATTAAAAGCCATG  489
CATACAAGCAATGTCTTCAGAAAACCTAGGGTCCAAGGTTAAGCCATATCCCAGCTCAG  548
TAAAGCCAGGAGCATCCTCATTTCCCA ATG GCC CTC CTG TTC CCT CTA CTG  599
GCA GCC CTA GTG ATG ACC AGC TAT AGC CCT GTT GGA TCT CTG GGC  644
TGT GAT CTG CCT CAG AAC CAT GGC CTA CTT AGC AGG AAC ACC TTG  689
GTG CTT CTG CAC CAA ATG AGG AGA ATC TCC CCT TTC TTG TGT CTC  734
AAG GAC AGA AGA GAC TTC AGG TTC CCC CAG GAG ATG GTA AAA GGG  779
AGC CAG TTG CAG AAG GCC CAT GTC ATG TCT GTC CTC CAT GAG ATG  824
CTG CAG CAG ATC TTC AGC CTC TTC CAC ACA GAG CGC TCC TCT GCT  869
GCC TGG AAC ATG ACC CTC CTA GAC CAA CTC CAC ACT GGA CTT CAT  914
CAG CAA CTG CAA CAC CTG GAG ACC TGC TTG CTG CAG GTA GTG GGA  959
GAA GGA GAA TCT GCT GGG GCA ATT AGC AGC CCT GCA CTG ACC TTG 1004
AGG AGG TAC TTC CAG GGA ATC CGT GTC TAC CTG AAA GAG AAG AAA 1045
TAC AGC GAC TGT GCC TGG GAA GTT GTC AGA ATG GAA ATC ATG AAA 1094
TCC TTG TTC TTA TCA ACA AAC ATG CAA GAA AGA CTG AGA AGT AAA 1139
GAT AGA GAC CTG GGC TCA TCT TGA AATGATTCTCATTGATTAATTTGCCAT 1190
ATAACACTTGCACATGTGACTCTGGTCAATTCAAAAGACTCTTATTTCGGCTTTAATCA 1249
CAGAATTGACTGAATTAGTTCTGCAAATACTTTGTCGGTATATTAAGCCAGTATATGTT 1308
AAAAAGACTTAGGTTCAGGGGCATCAGTCCCTAAGATGTTATTTATTTTTACTCATTTA 1367
TTTATTCTTACATTTTATCATATTTATACTATTTATATTCTTATATAACAAATGTTTGC 1426
CTTTACATTGTATTAAGATAACAAAACATGTTCAGCTTTCCATTTGGTTAAATATTGTA 1485
TTTTGTTATTTATTAAATTATTTTCAAACAAAACTTCTTGAAGTTATTTATTCGAAAAC 1544
CAAAATCCAAACACTAGTTTTCTGAACCAAATCAAGGAATGGACGGTAATATACACTTA 1603
CCTATTCATTCATTCCATTTACATAATATGTATAAAGTGAGTATCAAAGTGGCATATTT 1662
TGGAATTGATGTCAAGCAATGCAGGTGTACTCATTGCATGACTGTATCAAAATATCTCA 1721
TGTAACCAATAAATATATACACTTACTATGTATCCCACAAAAATTAAAAAGTTATTTTA 1780
AAAAAGAAATACAGGTGAATAAACACAGTTTCTTTCCGTGTTGAAGAGCTTTCATTCTT 1839
ACAGGAAAAGAAACAGTAAAGATGTACCAATTTCGCTTATATGAAACACTACAAAGATA 1898
AGTAAAAGAAAATGATGTTCTCATACTAGAAGCTT                         1933
```

**11.** Interferon-pseudo-omega2-Gen der Formel

```
        AAGCTTGAGCCCCCAGGGAAGCATAACCACATGAACCTGAATGAATATATT   51
CTAGAAGGAGGGAAGCACCAGAGAAGTTCTTTCACTAATAACCATCAACGTCTTCTGTG  110
AATCAAATATCAAACAAAGATAGTCCTAAAAAGTTTAATTTCCAGAGATAGGTAATTTC  169
CTAACTGAATACAGAAACCCATAGGGCCCAGGGATCCTGATTTCCTATGCAAAATGGAG  228
GGTAAAACTGGAGGCTAGGATCTGGGCTAAAAGTATATACTTCTAACAGTAGCACAAAG  287
ATGTTTCTCATCTGATTGATCAATATTCATTTGGATTGATATATCTTAAGTTTACTGGG  346
AATATTGAACATCCATTGCAAAAATCAAGAGTGTAGAGTGATGACCTCCTTTTAGGTCA  405
TATAGAACAAGGTTTTTCAACCCCCATCCATGGACCGGGGTACTGGTCCTGGCCTGGTA  464
GGAACAGGGCCGCACAGCAGGAGGCAAGCAGGCCAACCAACAAGCATTAACGCCTGAGC  523
TCTGCCTCCTGTCAGATCAGCAGTGGCATTGGATTCTCAAAAGAGCAGGAACCCTATTG  582
TGAAGTGCAGATGCGAAGGATCTAGGTTGTGGTCTCCTAATGAGAATCTAATGCCTCTG  641
AAAGCATTCCCTCCCTGACCCCATTTTTCGTGGAAAAATTATCTTCCACCAAACTGGTG  700
GCCAAAAGGTTGTGGATGCTGATATAGAAGACATGTAAATGAAAACAATAAATGGAATT  759
AAAAATTTAGAGAAATGCTCAGAAAAATGAAAACTATTTGTGCTCCATTAAAGCCATGC  818
ATAGATAGAATGTCTTCATAGAACCTAGGATCCAAGGTTCTATGAAGACCTCAGCTCAA  877
CCAGGCCAAAAGCATCCTGATTTCTCA ATG GCC CTC CTC TTC CCT CTA CTG  928
GCA GCC CTA GAG GTG TGC AGC TGT GGC TCT TCT GGA TCT CTA GGA  973
TAT AAC CTG CCT CAG AAC CAT GGC CTG CTA GGC AGG AAC ACC TTG 1018
GTG CTT TTG GGC CAA ATG AGG AGA ATC TCT CCC TTC TTG TGT CTA 1063
AAG GAC AGA AGT GAC TTC AGA TTC CCC CAG GAG AAG GTG GAA GTC 1108
AGC CAG TTG CAG AAG GCC CAG GCT ATG TCT TTC CTC TAT GAT GTG 1153
TTA CAG CAG GTC TTC AAC TTC TCA CAC AAA GCG CTC CTC TGC TGC 1198
ATG GAA CAT GAC CTT CCT GGA CCA ACT CCA CAC TTT ACG TCA TCA 1243
GCA GCT GGA ACA CCT GGA GAC CTG CTT GGT GCA GGA GAT GGG AGA 1288
AGG AGA AGC TGG GGG CAG TGG GTG ATT GAG GGC TCT ACA CTG GCC 1333
TTG AGG AGG TAT TTC CAG GAA TCC ATC TCT ACC TGA AAGAGAAGAAA 1380
TAAAGATTGTGCCTGGGAAGTTGTCAGAGTGGAAATCATGAGATCCTTTTCATCCACAA 1439
GTTTGCAAGAAAGATTGAGAAGTAAGGATGAAGACCTGGGCTCATCATGAAATGATTCT 1498
CATTGACTAATCTGCCATATCACACTTGTACATGTGACTTTGGATATTCAAAAAGCTCA 1557
TTTCTGTTTCATCAGAAATTATTGAATTAGTTTTAGCAAATACTTTATTAATAGCATAA 1619
AGCAAGTTTATGTCAAAAACATTCAGCTCCTGGGGCATCCGTAACTCAGAGATAACTGC 1675
CCTGATGCTGTTTATTTATCTTCCTTCTTTTTTTTCATGCCTTGTATTTATGATATTTA 1734
TATATTTTATATTTTCATCTTCACATCGTATTAAAATTTATAAAACATTCACTTTTTCA 1793
TATTAAGTTTGCATTTTGTTTTATTAAATTCATATCAAAGAAAACTCTGTAAATGTTTC 1852
```

```
TATTCTAAAAACAATGTCTACTTTCTCTTTTTGTAAACCAAATTGAAAATATGGTAAAA 1911
TGTATTAACTCATTCATTTCATTCCTATTATATGTATAAATTGAGTAAATGGCAAACTG 1970
TGGGGTTTTCTTAAAGAAATACAGGTGAATAAAGCAAACACAGTTTCTCTCAGTCTAAG 2029
AGGGAAAGAGACGTAAAAACAGGACAAATATTTATATTATTTCAATTATGTTAAATGCT 2088
ACAAAGAGAAGTAAAGAAAAGTGATGTTCTCACATCAGAAGCTT                2132
```

**12.** Interferon-pseudo-omega3-Gen der Formel

```
                                                      CCATG   5
CATAGCAGGAATGCCTTCAGAGAACCTGAAGTCCAAGGTTCATCCAGACCCCAGCTCAG  64
CTAGGCCAGCAGCACCCTCGTTTCCCA ATG GTC CTC CTG CTT CCT CTA CTC 115
GTG GCC CTG CCG CTT TGC CAC TGT GGC CCT GTT GGA TCT CTG AGC 160
TGG GAC CTG CCT CAG AAC CAT GGC CTA CTT AGC AGG AAC ACC TTG 205
GCA CTT CTG GGC CAA ATG TGC AGA ATC TCC ACT TTC TTG TGT CTC 250
AAG GAC AGA AGA GAC TTC AGG TTC CCC CTG GAG ATG TGG ATG GCA 295
GTC AGT TGC AGA AGG CCC CGG CCC TGT CTG TCC TCC ATG AGA TGC 340
TTC AGC AGA TCT TCA GCC TCT TCC CCA CAG AGT GCT CCT CTG CTG 385
CCT GGA ACA TGA CCCTCCTGGACCAACTCCACACTGGACTTCATCTGCAGCTGGA 440
ATGCCTGGATGCTTGCTTAGGGCAGACAAAAAGAGAGGAAGAATCTGTGGGGGTGATTG 499
GGGCCCTACACTGGCCTTGAGGACGTACTTTCAGGGAATGCATGGGAATCCAGGGAATC 558
TACCTGGAGGAGAAGAAATACAGTGACTGTGCTTGGGAGGTTGTCAGAGTGGAATCGTG 617
AAATCCTTCTCTTCATCATCAACAAACTTGCAAGAAGGACTGAGAAGTAAGGATGAAGA 676
CCTGGGTTCATCCTGAAATTATTCTCATTGATTAATCTGCCATATCACACTTGCACATG 735
TGTCTTTGGTCATTTCAATAGGTTCTTATTTCTGCAG                       772
```

**13.** Interferon-pseudo-omega4-Gen der Formel

```
                            AAGCTTTGGGCATGACCTAGTAGGTGACTCTT   32
AGTTGGAGTGGTCAGTTGTAAGGCTCTTGCTCAGTCACGTGGCTCCCCTGTATTTCCCC   91
ACGTTTGCAGCCGTGCTCCTTCTCAATGCTATGAAAGTGTGGGCTCCTCTCCCACTGGA  150
GTGCTGACTGTAGCTTGTATCTTGGCACTCCCAGGCTGTACATAACAGCTCTGAGGTGA  209
TCTCAGGGTTAATGTTTTCTCCCCGACTTGGAGGCCATTGAAGGAAGGGACCTTAGTAG  268
TAGTTGTAACTGAGGGTCTTTTGCTTGTCTCCTGGGGGCTCCACCCCAGAGATGCAGGT  327
GAGCAATCACTCAGTGCATTCAGCTTGGGATGGGGGTGTCTGTGCTGTGGGCCCAAGAC  386
AGGGGTTCCCTGCCTGGTGATGTGAGGGGTGGGTGGTTGACCAATGGCAGACAGACTGG  445
CCTCCTCTCTTGGGTTGACAGCAGCTTGTTGGAGGTATGCATAAGGCACTTGGGGTCTT  504
GCTCCTTCATTAGTTCCAAGGTAGCTGGGGTAGTACCACTGCAGAGGCAGTGTTAGACA  563
GGCTTTCTGTTACCCCTGGGGTCTCCACCTCCTAGAAATGTGAAGTCATGTTAATGGGA  622
```

GTGTTTAGCCAGAGGAGTGGGGTGGCTGCATGCTGGTGTAAGTATGGGACTTCACTTCT 681
TGGGAAACAGGGAGGTGGAATCTTACCAGCAGGAGACTGTTCTCCTCACGATGTGTAAC 740
CTGCAGTGTGCTGGAAGTTTAGGTGACTGTGGCATGATGTTAGCTTGTAAACAAAGAGC 799
TTCAGACTCTTTGTCTCTTCCCCAGACCCAAGGCAGCAAGGATAAAAGCTGCTGCTGTG 858
GCAGTGGCAGAGGTAGGATGGTTGTGGAGCCTCTCCCCAGGGAAACTCTAGTCAACTA 917
CCAGTGGATATGCTCAGCCATGGGTAGGGTGACTGTTCTGCAGTCATGGGCAGGGGGCC 976
TGCTCCTGAAGAATAGGGACAGGGATTCTCAGGGAAGAGGGGCTGGACTCCTCTTCATA 1035
TAGTGGCGATGATGTGCTGGAGGTGCCAGTGTAGTGAATAGGCCTTTGTTCCTTCCCCA 1094
GCCAGAGGGCTGCTGGGGCTGTCCCACTGCAACGGTCATGGTGGATGGGTTATGGGTTG 1153
ACTGTGGGATTTCTTTCTTGGAGAAATGCTGGACTGCCTGATTGAGGAGACGAGGCAAG 1212
GGAAGAATGCCTGTGCTGGAGTCTCTGGTCAGGTGGCTCTGCCACAGAGGAGAGGTGAC 1271
CACCAGGAACTGCGTGGAGAACAGTGTAGCCACTCTTCTGTGAGGCAGTTGCTCTGTTT 1330
TGGGGATCTGGAGCAGCCGCTATTCCTTACAGATTCCCAGAGCCTGGAGACAGCAAGGG 1389
CAAGAGCTGTGAGAAAGCAAAGATAGCAACCCACCCTTCTCACTGGGAGCTCTGTTCCA 1448
GGGAGATGCAGAGCTGCCATTGCTCAATAGCCCCAGCTGGTAGCTGCAGACCCAGGCCT 1507
GGCAGACCCACCCAGTGAGCAGATAGGGGATTAGGGACCCACATAACACACAGTCTGGC 1566
CACTTTTCCATAGGGCTGCTGAATATGCTGGGGGTCCAATCCAGACCATAGTCACCTCA 1625
CATTTTTCAGTACCTGAAGATATCAACAGTGAAGGCTATGAAACAGTGAAGATGGGGAC 1684
CTGCCCCTGCCTCTGGACCTCTGTTCCAGAGAGGTACAACCTGTTGCCTCCGACATACA 1743
TGCAGGAGGTGGCTGGAGACCCGGTGGATATCCCTCCCACTGAGGAGAAGCAGCATCAG 1802
GGAATCAGGTGAAGAAACAGTCTGGCCACTTTTTGGTAGAGCAGCTGTGCTTGCTGGGG 1861
GTCTGCTACCACCCCCAGCAAAAAGAATGGCATTTGCAAGAATGGCTAAGGCTGCTAAA 1920
CAGCAACAATGGCAACCTACCATTCCCTTTGGAGCGCCATCCCAGGGATATTCGAAACT 1979
GCTGTCCACTAGAAAACAGTGGTGGAGGTGACTGGAGACCCCAGTGGAGAGTTTCACCT 2038
GGTGAAAAGAAACAGGATTTGGGATCGACATGAATAACCAATCTGACTGCTTCCCCGTA 2097
GAGCTGCTGGACTGTGCTGGGTGGCTGCTCCAGTCCCTAGCTGCCTTGGACTCCCGAGA 2156
ACCCAAAGGCTCCAATAGCTAAGATTGTGAAAGAGCAAAGATGGCAGCCCACCCCCTGC 2215
CACAGGGAGCTCCATGTCAGGGAGGTATGAGGCTGCTACCAGTGTCTGGCTGGATTCCC 2274
AAGTCGAGTGGGTCTTACCCTGAGACAGGCCATGGAAGGTGGGCCTGTCATTGTCACTG 2333
CCCAGCGCCCTGGATGAAACCCCTTTCCTAGGGGTATGTATAGGGGTCTAGCGTCCTGC 2392
TTGGCTGGAGTTATAGCTTCTTTTGTGGGGAGGCCTGGGTATCTAAGGCTCCAGGGTAC 2451
CCATGCATGCGAGAGCGGCTGCTCTGCTGAACCCTACGTAGCCCTGCATGTCAGACTAA 2510
ATGCCCTGGTAGAGTGGGTTCACTAGGAGATCTCCTGACCTGAGGATTGCAAAGATCTG 2569
TGGGAGAAGCGTGGGTCCCCAGGGCTGCTCACTTACTCACCACTTCCCTGGGCAGGAGA 2628
GGCTCCCCTGGCTCTGTGTCATCCTGGGGGGGCAGTTGTCCTGCCTTACTTTGCTTTAT 2687
TCTCCATGGGTCAAGTTGTTTTCTTGAGTCTCAATGTGTGCACCTGGTTTTTTCAGTTG 2746
AAGGTGCTGTATTTACTTGCCCCTTCCATTTCTCTCCATGAGAGTGGCACACACTAGCA 2805
GGTTCCAGTCGGCCATCTTGCAACCCCTGAAAACTATTTGTTTCCAGCTATAAGCCATT 2864

GAGAGAACCTGGAGTGGCATAAAAAGAATGCCTCGGGGTTCATCCCGACCCCAGCTCAG 2923
CTAGGCCAGCAGCACCCTCGTTTCCCA ATG GTC CTA CTG CTT GTT CTA CTG 2974
GTG GCC CTG CTG CTT TGC CAA TGT GGC CCT GTT GGA TCT CTG GGC 3019
TTT GAC CTG CCT CAG AAC CGT GGC CTA CTT AGC AGG AAC ACC TTG 3064
GCA TTC TGG GCC AAA TGC AGA ATC TCC ACT TTC TTG TGT CTC AAG 3109
GAC AGA AGA GAC TTC AGG TTC CCC CTG GAG ATG TGG ATG GCA GTC 3154
ATT TGC AGA AGG CCC AGG CTG TGT CTG TCC TCC ATG AGA TGC TTC 3199
AGC AGA TCT TCA GCC TCT TCC CCA CAG AGC GCT CCT CTG CTG CCT 3244
GGA ACA TGA CCCTCCTGGACCAGCTCCACACTGGATTTCATCAGCAGCTCGAATAG 3300
CCTGGAGTCTTGCTTAGGGCAGGCAACAGGAGAGGAAGAATCTGTGGGGGTGATTGGGA 3359
CCCTACACTGGCCTTGAGGAGGTACTTCCAGGGAATCCATGGGAATCCAGAGAATCTAC 3418
CTGAAAGAGAAGAAATACAGTGACTGTGCTTAGGAGGTTGTCAGAATGGAATCATGAAA 3477
TCCTTCTCTTCATCAACAGACTTGCAAGGACTGAGAAGTAAGGATGAAGACCTGGGGTC 3536
TGCTTTACTCTTTCTTATTTTCTTCCTCTTCCTTACTATGTGTTTATTTCTTCTTTTTC 3595
TAGTTCCTTAACTTGTAAGTAGTTCACTTGGTTTGAGGTCTTTCTTCTTTTTTAATATA 3654
AGCTT 3659

14. Transformierter Wirtsorganismus, der die genetische Information gemäß den Ansprüchen 1 bis 10 enthält.

15. Wirtsorganismus gemäß Anspruch 14, dadurch gekennzeichnet, daß die genetische Sequenz in einem Vehikel enthalten ist, das in dem Wirtsorganismus replizierbar ist.

16. Wirtsorganismus gemäß Anspruch 14, dadurch gekennzeichnet, daß er eine Säugetierzellinie oder E. coli ist.

17. Wirtsorganismus gemäß Anspruch 14, dadurch gekennzeichnet, daß er E. coli mit der DSM Nr. 3003 oder E. coli mit der DSM Nr. 3004 ist.

18. Wirtsorganismus gemäß Anspruch 17, dadurch gekennzeichnet, daß das Vehikel zusätzlich die für die Expression erforderlichen Replikon- und Kontrollsequenzen enthält.

19. Wirtsorganismus gemäß Anspruch 18, dadurch gekennzeichnet, daß das Vehikel die für die Expression erforderlichen Replikon- und Kontrollsequenzen des Plasmids pER103 mit der DSM Nr. 2773 (siehe Abbildung 6) enthält.

20. Wirtsorganismus gemäß Anspruch 19, transformiert mit einem Expressionsplasmid gemäß Anspruch 21, 22 oder 23.

21. Expressionsplasmid für Interferon omega, das ein Derivat des plasmids pBR322 ist, dadurch gekennzeichnet, daß das Plasmid pBR322 anstelle des plasmideigenen kürzeren EcoRI/BamHI-Fragmentes die DNA Sequenz der Formel

```
EcoRI        Sau3A
gaattcacgctGATCGCTAAAACATTGTGCAAAAAGAGGGTTGACTTTGCCTTCGCGA  59

                       ↓mRNA-Start                               Met
ACCAGTTAACTAGTACACAAGTTCACGGCAACGGTAAGGAGGTTTAAGCTTAAAG ATG 116
                                        RBS      HindIII
Cys Asp
TGT GAT C ——— IFN-omega-Gen ———→
    Sau3A
```

enthält.

22. Expressionsplasmid pRHW12 gemäß Anspruch 21, dadurch gekennzeichnet, daß das Interferon-omega-Gen die DNA-Sequenz der Formel

```
TGT GAT CTG CCT CAG AAC CAT GGC CTA CTT AGC AGG AAC ACC TTG  28
                        NcoI
GTG CTT CTG CAC CAA ATG AGG AGA ATC TCC CCT TTC TTG TGT CTC  73
AAG GAC AGA AGA GAC TTC AGG TTC CCC CAG GAG ATG GTA AAA GGG 118
AGC CAG TTG CAG AAG GCC CAT GTC ATG TCT GTC CTC CAT GAG ATG 163
CTG CAG CAG ATC TTC AGC CTC TTC CAC ACA GAG CGC TCC TCT GCT 208
GCC TGG AAC ATG ACC CTC CTA GAC CAA CTC CAC ACT GGA CTT CAT 253
CAG CAA CTG CAA CAC CTG GAG ACC TGC TTG CTG CAG GTA GTG GGA 298
GAA GGA GAA TCT GCT GAG GCA ATT AGC AGC CCT GCA CTG ACC TTG 343
AGG AGG TAC TTC CAG GGA ATC CGT GTC TAC CTG AAA GAG AAG AAA 388
TAC AGC GAC TGT GCC TGG GAA GTT GTC AGA ATG GAA ATC ATG AAA 433

TCC TTG TTC TTA TCA ACA AAC ATG CAA GAA AGA CTG AGA AGT AAA 478
GAT AGA GAC CTG GGC TCA TCT TGAAATGATTCTCATTGATTAATTTGCCATA 530
TAACACTTGCACATGTGACTCTGGTCAATTCAAAAGACTCTTATTTCGGCTTTAATCAC 589
AGAATTGACTGAATTAGTTCTGCAAATACTTTGTCGGTATATTAAGCCAGTATATGTTA 648
AAAAGACTTAGGTTCAGGGGCATCAGTCCCTAAGATGTTATTTATTTTTACTCATTTAT 707
TTATTCTTACATTTTATCATATTTATACTATTTATATTCTTATATAACAAATGTTTGCC 766
TTTACATTGTATTAAGATAACAAAACATGTTCAGct                        802
                                AluI
```

aufweist.

23. Expressionsplasmid pRHW11 gemäß Anspruch 21, dadurch gekennzeichnet, daß das Interferon-omega-Gen die DNA-Sequenz der Formel

```
TGT GAT CTG CCT CAG AAC CAT GGC CTA CTT AGC AGG AAC ACC TTG  28
                        NcoI
GTG CTT CTG CAC CAA ATG AGG AGA ATC TCC CCT TTC TTG TGT CTC  73
AAG GAC AGA AGA GAC TTC AGG TTC CCC CAG GAG ATG GTA AAA GGG 118
AGC CAG TTG CAG AAG GCC CAT GTC ATG TCT GTC CTC CAT GAG ATG 163
CTG CAG CAG ATC TTC AGC CTC TTC CAC ACA GAG CGC TCC TCT GCT 208
GCC TGG AAC ATG ACC CTC CTA GAC CAA CTC CAC ACT GGA CTT CAT 253
CAG CAA CTG CAA CAC CTG GAG ACC TGC TTG CTG CAG GTA GTG GGA 298
GAA GGA GAA TCT GCT GAG GCA ATT AGC AGC CCT GCA CTG ACC TTG 343
AGG AGG TAC TTC CAG GGA ATC CGT GTC TAC CTG AAA GAG AAG AAA 388
TAC AGC GAC TGT GCC TGG GAA GTT GTC AGA ATG GAA ATC ATG AAA 433
TCC TTG TTC TTA TCA ACA AAC ATG CAA GAA AGA CTG AGA AGT AAA 478
GAT AGA GAC CTG GGC TCA TCT TGAAATGATTCTCATTGATTAATTTGCCATA 530
TAACACTTGCACATGTGACTCTGGTCAATTCAAAAGACTCTTATTTCGGCTTTAATCAC 589
AGAATTGACTGAATTAGTTCTGCAAATACTTTGTCGGTATATTAAGCCAGTATATGTTA 648
AAAAGACTTAGGTTCAGGGGCATCAGTCCCTAAGATGTTATTTATTTTTACTCATTTAT 707
TTATTCTTACATTTTATCATATTTATACTATTTATATTCTTATATAACAAATGTTTGCC 766
TTTACATTGTATTAAGATAACAAAACATGTTCAGct                        802
                                AluI
```

aufweist.

**24.** Plasmid PRHW10, das ein Derivat des Plasmids pBR322 ist, dadurch gekennzeichnet, daß in das Plasmid pER103 mit der DSM Nr. 2773 (siehe Abbildung 6) in die HindIII-Stelle das DNA-Fragment der Formel

```
HindIII           Sau3A           NcoI
aAGCTTAAAG ATGTGTGATC TGCCTCAGAA CCATGGCCTA CTTAGCAGGA    50

ACACCTTGGT GCTTCTGCAC CAAATGAGGA GAATCTCCCC TTTCTTGTGT   100

CTCAAGGACA GAAGAGACTT CAGGTTCCCC CAGGAGATGG TAAAAGGGAG   150

CCAGTTGCAG AAGGCCCATG TCATGTCTGT CCTCCATGAG ATGCTGCAGC   200

AGATCACACA TCTTTAagct t
 Sau3A          HindIII
```

eingefügt ist.

**25.** E. coli HB 101 transformiert mit einem Plasmid gemäß Anspruch 21, 22, 23 oder 24.

**26.** Neue menschliche Interferon-Proteine vom Typ I, dadurch gekennzeichnet, daß sie von einem DNA-Molekül nach einem der Ansprüche 1 bis 10 codiert werden.

**27.** Neue menschliche Interferone gemäß Anspruch 26, dadurch gekennzeichnet, daß die reifen Interferone eine Divergenz von 30 bis 50 % gegenüber den menschlichen α-Inteferonen aufweisen.

**28.** Neue menschliche Interferone vom Typ I gemäß Anspruch 26, dadurch gekennzeichnet, daß

a) die reifen Interferone eine Divergenz von 30 bis 50% gegenüber den menschlichen α-Interferonen und eine Divergenz von ungefähr 70 % gegenüber β-Interferon aufweisen und

b) 168 bis 174 Aminosäuren lang sind, und deren N-glycosylierte Derivate.

**29.** Interferone gemäß Anspruch 28, dadurch gekennzeichnet, daß diese eine Divergenz von 40 bis 48 % gegenüber den menschlichen α-Interferonen aufweisen, und deren N-glycosylierte Derivate.

**30.** Interferone gemäß den Ansprüchen 26 bis 29, dadurch gekennzeichnet, daß diese ein Leader-Peptid enthalten.

**31.** Interferone gemäß den Ansprüchen 26 bis 29, dadurch gekennzeichnet, daß diese 172 Aminosäuren enthalten.

**32.** Interferon gemäß Anspruch 31, dadurch gekennzeichnet, daß es die Aminosäuresequenz

| | | | | | | | | | | | | | | |
|---|---|---|---|---|---|---|---|---|---|---|---|---|---|---|
| | | | | 5 | | | | | 10 | | | | | 15 |
| Cys | Asp | Leu | Pro | Gln | Asn | His | Gly | Leu | Leu | Ser | Arg | Asn | Thr | Leu |
| | | | | 20 | | | | | 25 | | | | | 30 |
| Val | Leu | Leu | His | Gln | Met | Arg | Arg | Ile | Ser | Pro | Phe | Leu | Cys | Leu |
| | | | | 35 | | | | | 40 | | | | | 45 |
| Lys | Asp | Arg | Arg | Asp | Phe | Arg | Phe | Pro | Gln | Glu | Met | Val | Lys | Gly |
| | | | | 50 | | | | | 55 | | | | | 60 |
| Ser | Gln | Leu | Gln | Lys | Ala | His | Val | Met | Ser | Val | Leu | His | Glu | Met |
| | | | | 65 | | | | | 70 | | | | | 75 |
| Leu | Gln | Gln | Ile | Phe | Ser | Leu | Phe | His | Thr | Glu | Arg | Ser | Ser | Ala |
| | | | | 80 | | | | | 85 | | | | | 90 |
| Ala | Trp | Asn | Met | Thr | Leu | Leu | Asp | Gln | Leu | His | Thr | Gly | Leu | His |
| | | | | 95 | | | | | 100 | | | | | 105 |
| Gln | Gln | Leu | Gln | His | Leu | Glu | Thr | Cys | Leu | Leu | Gln | Val | Val | Gly |
| | | | | 110 | | | | | 115 | | | | | 120 |
| Glu | Gly | Glu | Ser | Ala | Gly | Ala | Ile | Ser | Ser | Pro | Ala | Leu | Thr | Leu |
| | | | | 125 | | | | | 130 | | | | | 135 |
| Arg | Arg | Tyr | Phe | Gln | Gly | Ile | Arg | Val | Tyr | Leu | Lys | Glu | Lys | Lys |
| | | | | 140 | | | | | 145 | | | | | 150 |
| Tyr | Ser | Asp | Cys | Ala | Trp | Glu | Val | Val | Arg | Met | Glu | Ile | Met | Lys |

155 160 165
Ser Leu Phe Leu Ser Thr Asn Met Gln Glu Arg Leu Arg Ser Lys

170
Asp Arg Asp Leu Gly Ser Ser

enthält, und dessen in Aminosäureposition 78 N-glycolysierte Derivate.

**33.** Interferon gemäß Anspruch 31, dadurch gekennzeichnet, daß das Interferon gemäß Anspruch 32 in Position 111 die Aminosäure Glu anstelle von Gly enthält, und dessen in Aminosäureposition 78 N-glycolysierte Derivate.

**34.** Interferone gemäß den Ansprüchen 30 bis 33 dadurch gekennzeichnet, daß diese das Leader-Peptid der Formel

Met Ala Leu Leu Phe Pro Leu Leu Ala Ala Leu Val Met Thr Ser Tyr Ser Pro Val Gly Ser Leu Gly

enthalten.

**35.** Verfahren zur Herstellung der Interferon-Proteine gemäß den Ansprüchen 26 bis 34, dadurch gekennzeichnet, daß

ein geeigneter Wirtsorganismus mit genetischen Informationen, codierend für die Interferon-Proteine gemäß den Ansprüchen 26 bis 34, transformiert wird,

diese Information exprimiert wird, um das entsprechende Interferon in dem Wirtsorganismus zu produzieren und

das so erhaltene Interferon-Peptid aus diesem Wirtsorganismus isoliert wird.

**36.** Verfahren gemäß Anspruch 35, dadurch gekennzeichnet, daß die Information in jedem rekombinanten DNA-Molekül gemäß den Ansprüchen 1 bis 10 enthalten ist.

**37.** Verfahren gemäß Anspruch 36, dadurch gekennzeichnet, daß der Wirtsorganismus, nach der Transformation, gemäß einem der Ansprüche 15 bis 20 definiert ist.

**38.** Verfahren gemäß Anspruch 35, dadurch gekennzeichnet, daß das Interferon gemäß den Ansprüchen 32 bis 34 definiert ist.

**39.** Verfahren gemäß Anspruch 35, dadurch gekennzeichnet, daß als Wirt E. coli HB 101 und als Plasmid ein Plasmid gemäß Anspruch 21, 22 oder 23 verwendet wird.

**40.** Omega-Interferon herstellbar durch das Verfahren gemäß Anspruch 35 oder 39.

**41.** Mischung geeignet für die antitumor- oder antivirale Behandlung, enthaltend neben einem oder mehreren inerten Trägerstoffen und/oder Verdünnungsmitteln eine wirksame Menge an Interferon-Peptid gemäß einem der Ansprüche 26 bis 34.

**42.** Verwendung von Omega-Interferon gemäß den Ansprüchen 30 bis 34 zur Herstellung eines zur antitumor- oder antiviraler Behandlung geeigneten Mischung.

**43.** Verfahren zur Herstellung des Plasmids pRHW10, dadurch gekennzeichnet, daß in das Plasmid pER103 mit der DSM Nr. 2773 (Abbildung 6) in die HindIII-Stelle mittels Ligasereaktion das DNA-Fragment der Formel

```
HindIII              Sau3A               NcoI
aAGCTTAAAG ATGTGTGATC TGCCTCAGAA CCATGGCCTA CTTAGCAGGA     50

ACACCTTGGT GCTTCTGCAC CAAATGAGGA GAATCTCCCC TTTCTTGTGT    100

CTCAAGGACA GAAGAGACTT CAGGTTCCCC CAGGAGATGG TAAAAGGGAG    150

CCAGTTGCAG AAGGCCCATG TCATGTCTGT CCTCCATGAG ATGCTGCAGC    200

                      AGATCACACA TCTTTAagct t
                        Sau3A          HindIII
```

eingefügt wird,
und das so erhaltene Plasmid zur Replikation in E. coli HB 101 transformiert und kultiviert wird.

**44.** Verfahren zur Herstellung des Expressionsplasmids pRHW12, dadurch gekennzeichnet, daß in das durch Restrictionsendonucleaseverdauung mit BamHI, Auffüllen der Schnittstellen mit Hilfe des Klenow-Fragmentes der DNA-Polymerase I und den 4 Deoxynucleosidtriphosphaten sowie anschließendem Schneiden mit NcoI erhaltene größere Fragment des Plasmids pRHW10, welches in der HindIII-Stelle des Plasmids pER103 mit der DSM Nr. 2773 (siehe Abbildung 6) das DNA-Fragment der Formel

```
HindIII              Sau3A               NcoI
aAGCTTAAAG ATGTGTGATC TGCCTCAGAA CCATGGCCTA CTTAGCAGGA     50

ACACCTTGGT GCTTCTGCAC CAAATGAGGA GAATCTCCCC TTTCTTGTGT    100

CTCAAGGACA GAAGAGACTT CAGGTTCCCC CAGGAGATGG TAAAAGGGAG    150

CCAGTTGCAG AAGGCCCATG TCATGTCTGT CCTCCATGAG ATGCTGCAGC    200

AGATCACACA TCTTTAagct t
 Sau3A          HindIII
```

enthält,
das durch Verdauen des AvaII-Fragmentes des Klones P9A2 (DSM-Nr. 3004) mit NcoI und AluI erhaltene Fragment der Formel

```
                    c CAT GGC CTA CTT AGC AGG AAC ACC TTG  28
                      NcoI
GTG CTT CTG CAC CAA ATG AGG AGA ATC TCC CCT TTC TTG TGT CTC  73
AAG GAC AGA AGA GAC TTC AGG TTC CCC CAG GAG ATG GTA AAA GGG 118
AGC CAG TTG CAG AAG GCC CAT GTC ATG TCT GTC CTC CAT GAG ATG 163
CTG CAG CAG ATC TTC AGC CTC TTC CAC ACA GAG CGC TCC TCT GCT 208
```

```
GCC TGG AAC ATG ACC CTC CTA GAC CAA CTC CAC ACT GGA CTT CAT 253
CAG CAA CTG CAA CAC CTG GAG ACC TGC TTG CTG CAG GTA GTG GGA 298
GAA GGA GAA TCT GCT GGG GCA ATT AGC AGC CCT GCA CTG ACC TTG 343
AGG AGG TAC TTC CAG GGA ATC CGT GTC TAC CTG AAA GAG AAG AAA 388
TAC AGC GAC TGT GCC TGG GAA GTT GTC AGA ATG GAA ATC ATG AAA 433
TCC TTG TTC TTA TCA ACA AAC ATG CAA GAA AGA CTG AGA AGT AAA 478
GAT AGA GAC CTG GGC TCA TCT TGAAATGATTCTCATTGATTAATTTGCCATA 530
TAACACTTGCACATGTGACTCTGGTCAATTCAAAAGACTCTTATTTCGGCTTTAATCAC 589
AGAATTGACTGAATTAGTTCTGCAAATACTTTGTCGGTATATTAAGCCAGTATATGTTA 648
AAAAGACTTAGGTTCAGGGGCATCAGTCCCTAAGATGTTATTTATTTTTACTCATTTAT 707
TTATTCTTACATTTTATCATATTTATACTATTTATATTCTTATATAACAAATGTTTGCC 766
TTTACATTGTATTAAGATAACAAAACATGTTCAGCt                        802
                                 AluI
```

mittels Ligasereaktion eingefügt wird,
und das so erhaltene Plasmid zur Replikation in E. coli HB 101 transformiert und kultiviert wird.

**45.** Verfahren zur Herstellung des Expressionsplasmids pHWR11, dadurch gekennzeichnet, daß in das durch Restrictionsendonucleaseverdauung mit BamHI, Auffüllen der Schnittstellen mit Hilfe des Klenow-Fragmentes der DNA-Polymerase I und den 4 Deoxynucleosidtriphosphaten sowie anschließendem Schneiden mit NcoI erhaltene größere Fragment des Plasmids pRHW10, welches in der HindIII-Stelle des Plasmids pER103 mit der DSM Nr. 2773 (siehe Abbildung 6) das DNA-Fragment der Formel

```
HindIII          Sau3A           NcoI
aAGCTTAAAG ATGTGTGATC TGCCTCAGAA CCATGGCCTA CTTAGCAGGA    50

ACACCTTGGT GCTTCTGCAC CAAATGAGGA GAATCTCCCC TTTCTTGTGT   100

CTCAAGGACA GAAGAGACTT CAGGTTCCCC CAGGAGATGG TAAAAGGGAG   150

CCAGTTGCAG AAGGCCCATG TCATGTCTGT CCTCCATGAG ATGCTGCAGC   200

AGATCACACA TCTTTAagct t
 Sau3A          HindIII
```

enthält, das durch Verdauen eines AvaII-Fragmentes des Klones E76E9 (DSM Nr. 3003) mit NcoI und AluI erhaltene Fragment der Formel

```
                              c CAT GGC CTA CTT AGC AGG AAC ACC TTG  28
                                NcoI
GTG CTT CTG CAC CAA ATG AGG AGA ATC TCC CCT TTC TTG TGT CTC  73
AAG GAC AGA AGA GAC TTC AGG TTC CCC CAG GAG ATG GTA AAA GGG 118
AGC CAG TTG CAG AAG GCC CAT GTC ATG TCT GTC CTC CAT GAG ATG 163
CTG CAG CAG ATC TTC AGC CTC TTC CAC ACA GAG CGC TCC TCT GCT 208
GCC TGG AAC ATG ACC CTC CTA GAC CAA CTC CAC ACT GGA CTT CAT 253
CAG CAA CTG CAA CAC CTG GAG ACC TGC TTG CTG CAG GTA GTG GGA 298
GAA GGA GAA TCT GCT GAG GCA ATT AGC AGC CCT GCA CTG ACC TTG 343
AGG AGG TAC TTC CAG GGA ATC CGT GTC TAC CTG AAA GAG AAG AAA 388
TAC AGC GAC TGT GCC TGG GAA GTT GTC AGA ATG GAA ATC ATG AAA 433
TCC TTG TTC TTA TCA ACA AAC ATG CAA GAA AGA CTG AGA AGT AAA 478
GAT AGA GAC CTG GGC TCA TCT TGAAATGATTCTCATTGATTAATTTGCCATA 530
TAACACTTGCACATGTGACTCTGGTCAATTCAAAAGACTCTTATTTCGGCTTTAATCAC 589
AGAATTGACTGAATTAGTTCTGCAAATACTTTGTCGGTATATTAAGCCAGTATATGTTA 648
AAAAGACTTAGGTTCAGGGGCATCAGTCCCTAAGATGTTATTTATTTTTACTCATTTAT 707
TTATTCTTACATTTTATCATATTTATACTATTTATATTCTTATATAACAAATGTTTGCC 766
TTTACATTGTATTAAGATAACAAAACATGTTCAGct                        802
                                  AluI
```

mittels Ligasereaktion eingefügt wird,
und das so erhaltene Plasmid zur Replikation in E. coli HB 101 transformiert und kultiviert wird.

**46.** Synergistische Mischung gemäß Anspruch 41, dadurch gekennzeichnet, daß diese zusätzlich $\gamma$-Interferon enthält.

**47.** Synergistische Mischung gemäß Anspruch 41, dadurch gekennzeichnet, daß diese zusätzlich humanen Tumor-Nekrose-Faktor enthält.

## Claims

**1.** Recombinant DNA molecule containing a coding sequence for new human interferon proteins of type I, containing 168-174 amino acids, for interferon-pseudo-omega2 (see Figure 12), interferon-pseudo-omega3 (see Figure 13) or interferon-pseudo-omega4 (see Figure 14), characterised in that the coding sequence is hybridised under stringent conditions which enable a homology greater than 85% to be recognised, with the inserts in the Pst-1 cutting site a) of the plasmid E76E9 in E. coli HB 101, filed at the DSM under the number DSM 3003 or b) of the plasmid P9A2 in E. coli HB 101, filed at the DSM under the number DSM 3004.

**2.** DNA molecule as claimed in claim 1, characterised in that the stringent conditions only enable a homology of more than 90% to be detected.

**3.** DNA molecule as claimed in claims 1 and 2, characterised in that the coding sequence is present as an insert in the Pst-1 cutting site a) of the plasmid E76E9 in E. coli HB 101, filed at the DSM under the number DSM 3003 or b) of the plasmid P9A2 in E. coli HB 101, filed at the DSM under the number DSM 3004, or a degenerate variation of this insert.

**4.** DNA molecule as claimed in claims 1 to 3, characterised in that the vehicle is a plasmid which is replicatable in procaryotes or eucaryotes.

5. DNA molecule according to claim 4, characterised in that it is an expression vehicle which is replicatable in microorganisms or in mammalian cells.

6. DNA molecule as claimed in claim 5, containing the sequence

```
TGT GAT CTG CCT CAG AAC CAT GGC CTA CTT AGC AGG AAC ACC TTG  45
GTG CTT CTG CAC CAA ATG AGG AGA ATC TCC CCT TTC TTG TGT CTC  90
AAG GAC AGA AGA GAC TTC AGG TTC CCC CAG GAG ATG GTA AAA GGG 135
AGC CAG TTG CAG AAG GCC CAT GTC ATG TCT GTC CTC CAT GAG ATG 180
CTG CAG CAG ATC TTC AGC CTC TTC CAC ACA GAG CGC TCC TCT GCT 225
GCC TGG AAC ATG ACC CTC CTA GAC CAA CTC CAC ACT GGA CTT CAT 270
CAG CAA CTG CAA CAC CTG GAG ACC TGC TTG CTG CAG GTA GTG GGA 315
GAA GAA GAA TCT GCT GGG GCA ATT AGC AGC CCT GCA CTG ACC TTG 360
AGG AGG TAC TTC CAG GGA ATC CGT GTC TAC CTG AAA GAG AAG AAA 405
TAC AGC GAC TGT GCC TGG GAA GTT GTC AGA ATG GAA ATC ATG AAA 450
TCC TTG TTC TTA TCA ACA AAC ATG CAA GAA AGA CTG AGA AGT AAA 495
GAT AGA GAC CTG GGC TCA TCT                                 516
```

7. DNA molecule as claimed in claim 5, characterised in that the DNA molecule as claimed in claim 6 contains the nucleotide A instead of the nucleotide G in position 332.

8. DNA molecule as claimed in claim 5, characterised in that it is a) the plasmid E76E9 in E. coli HB 101 filed at the DSM under the number DSM 3003 or b) the plasmid P9A2 in E. coli HB 101, filed at the DSM under the number DSM 3004.

9. DNA molecule as claimed in claims 6 and 7, characterised in that it additionally contains the DNA sequence of formula

ATG GCC CTC CTG TTC CCT CTA CTG GCA GCC CTA GTG ATG ACC AGC TAT AGC CCT GTT GGA TCT CTG GGC
coding for the leader peptide.

10. Interferon-omegal gene of formula

GATCTGGTAAACCTGAA 17
GCAAATATAGAAACCTATAGGGCCTGACTTCCTACATAAAGTAAGGAGGGTAAAAATGG 76
AGGCTAGAATAAGGGTTAAAATTTTGCTTCTAGAACAGAGAAAATGATTTTTTTCATAT 135
ATATATGAATATATATTATATATACACATATATACATATATTCACTATAGTGTGTATAC 194
ATAAATATATAATATATATATATTGTTAGTGTAGTGTGTGTCTGATTATTTACATGCATAT 253
AGTATATACACTTATGACTTTAGTACCCAGACGTTTTTCATTTGATTAAGCATTCATTT 312
GTATTGACACAGCTGAAGTTTACTGGAGTTTAGCTGAAGTCTAATGCAAAATTAATAGA 371
TTGTTGTCATCCTCTTAAGGTCATAGGGAGAACACACAAATGAAAACAGTAAAAGAAAC 430
TGAAAGTACAGAGAAATGTTCAGAAAATGAAAACCATGTGTTTCCTATTAAAAGCCATG 489
CATACAAGCAATGTCTTCAGAAAACCTAGGGTCCAAGGTTAAGCCATATCCCAGCTCAG 548
TAAAGCCAGGAGCATCCTCATTTCCCA ATG GCC CTC CTG TTC CCT CTA CTG 599
GCA GCC CTA GTG ATG ACC AGC TAT AGC CCT GTT GGA TCT CTG GGC 644
TGT GAT CTG CCT CAG AAC CAT GGC CTA CTT AGC AGG AAC ACC TTG 689
GTG CTT CTG CAC CAA ATG AGG AGA ATC TCC CCT TTC TTG TGT CTC 734
AAG GAC AGA AGA GAC TTC AGG TTC CCC CAG GAG ATG GTA AAA GGG 779
AGC CAG TTG CAG AAG GCC CAT GTC ATG TCT GTC CTC CAT GAG ATG 824
CTG CAG CAG ATC TTC AGC CTC TTC CAC ACA GAG CGC TCC TCT GCT 869
GCC TGG AAC ATG ACC CTC CTA GAC CAA CTC CAC ACT GGA CTT CAT 914
CAG CAA CTG CAA CAC CTG GAG ACC TGC TTG CTG CAG GTA GTG GGA 959
GAA GGA GAA TCT GCT GGG GCA ATT AGC AGC CCT GCA CTG ACC TTG 1004
AGG AGG TAC TTC CAG GGA ATC CGT GTC TAC CTG AAA GAG AAG AAA 1045
TAC AGC GAC TGT GCC TGG GAA GTT GTC AGA ATG GAA ATC ATG AAA 1094
TCC TTG TTC TTA TCA ACA AAC ATG CAA GAA AGA CTG AGA AGT AAA 1139
GAT AGA GAC CTG GGC TCA TCT TGA AATGATTCTCATTGATTAATTTGCCAT 1190
ATAACACTTGCACATGTGACTCTGGTCAATTCAAAAGACTCTTATTTCGGCTTTAATCA 1249
CAGAATTGACTGAATTAGTTCTGCAAATACTTTGTCGGTATATTAAGCCAGTATATGTT 1308
AAAAAGACTTAGGTTCAGGGGCATCAGTCCCTAAGATGTTATTTATTTTTACTCATTTA 1367
TTTATTCTTACATTTTATCATATTTATACTATTTATATTCTTATATAACAAATGTTTGC 1426
CTTTACATTGTATTAAGATAACAAAACATGTTCAGCTTTCCATTTGGTTAAATATTGTA 1485
TTTTGTTATTTATTAAATTATTTTCAAACAAAACTTCTTGAAGTTATTTATTCGAAAAC 1544
CAAAATCCAAACACTAGTTTTCTGAACCAAATCAAGGAATGGACGGTAATATACACTTA 1603
CCTATTCATTCATTCCATTTACATAATATGTATAAAGTGAGTATCAAAGTGGCATATTT 1662
TGGAATTGATGTCAAGCAATGCAGGTGTACTCATTGCATGACTGTATCAAAATATCTCA 1721
TGTAACCAATAAATATATACACTTACTATGTATCCCACAAAAATTAAAAAGTTATTTTA 1780
AAAAAGAAATACAGGTGAATAAACACAGTTTCTTTCCGTGTTGAAGAGCTTTCATTCTT 1839
ACAGGAAAAGAAACAGTAAAGATGTACCAATTTCGCTTATATGAAACACTACAAAGATA 1898
AGTAAAAGAAAATGATGTTCTCATACTAGAAGCTT 1933

**11.** Interferon-pseudo-omega2-gene of formula

AAGCTTGAGCCCCCAGGGAAGCATAACCACATGAACCTGAATGAATATATT 51
CTAGAAGGAGGGAAGCACCAGAGAAGTTCTTTCACTAATAACCATCAACGTCTTCTGTG 110
AATCAAATATCAAACAAAGATAGTCCTAAAAAGTTTAATTTCCAGAGATAGGTAATTTC 169
CTAACTGAATACAGAAACCCATAGGGCCCAGGGATCCTGATTTCCTATGCAAAATGGAG 228
GGTAAAACTGGAGGCTAGGATCTGGGCTAAAAGTATATACTTCTAACAGTAGCACAAAG 287
ATGTTTCTCATCTGATTGATCAATATTCATTTGGATTGATATATCTTAAGTTTACTGGG 346
AATATTGAACATCCATTGCAAAAATCAAGAGTGTAGAGTGATGACCTCCTTTTAGGTCA 405
TATAGAACAAGGTTTTTCAACCCCCATCCATGGACCGGGGTACTGGTCCTGGCCTGGTA 464
GGAACAGGGCCGCACAGCAGGAGGCAAGCAGGCCAACCAACAAGCATTAACGCCTGAGC 523
TCTGCCTCCTGTCAGATCAGCAGTGGCATTGGATTCTCAAAAGAGCAGGAACCCTATTG 582
TGAAGTGCAGATGCGAAGGATCTAGGTTGTGGTCTCCTAATGAGAATCTAATGCCTCTG 641
AAAGCATTCCCTCCCTGACCCCATTTTTCGTGGAAAAATTATCTTCCACCAAACTGGTG 700
GCCAAAAGGTTGTGGATGCTGATATAGAAGACATGTAAATGAAAACAATAAATGGAATT 759
AAAAATTTAGAGAAATGCTCAGAAAAATGAAAACTATTTGTGCTCCATTAAAGCCATGC 818
ATAGATAGAATGTCTTCATAGAACCTAGGATCCAAGGTTCTATGAAGACCTCAGCTCAA 877
CCAGGCCAAAAGCATCCTGATTTCTCA ATG GCC CTC CTC TTC CCT CTA CTG 928
GCA GCC CTA GAG GTG TGC AGC TGT GGC TCT TCT GGA TCT CTA GGA 973
TAT AAC CTG CCT CAG AAC CAT GGC CTG CTA GGC AGG AAC ACC TTG 1018
GTG CTT TTG GGC CAA ATG AGG AGA ATC TCT CCC TTC TTG TGT CTA 1063
AAG GAC AGA AGT GAC TTC AGA TTC CCC CAG GAG AAG GTG GAA GTC 1108
AGC CAG TTG CAG AAG GCC CAG GCT ATG TCT TTC CTC TAT GAT GTG 1153
TTA CAG CAG GTC TTC AAC TTC TCA CAC AAA GCG CTC CTC TGC TGC 1198
ATG GAA CAT GAC CTT CCT GGA CCA ACT CCA CAC TTT ACG TCA TCA 1243
GCA GCT GGA ACA CCT GGA GAC CTG CTT GGT GCA GGA GAT GGG AGA 1288
AGG AGA AGC TGG GGG CAG TGG GTG ATT GAG GGC TCT ACA CTG GCC 1333
TTG AGG AGG TAT TTC CAG GAA TCC ATC TCT ACC TGA AAGAGAAGAAA 1380
TAAAGATTGTGCCTGGGAAGTTGTCAGAGTGGAAATCATGAGATCCTTTTCATCCACAA 1439
GTTTGCAAGAAAGATTGAGAAGTAAGGATGAAGACCTGGGCTCATCATGAAATGATTCT 1498
CATTGACTAATCTGCCATATCACACTTGTACATGTGACTTTGGATATTCAAAAAGCTCA 1557
TTTCTGTTTCATCAGAAATTATTGAATTAGTTTTAGCAAATACTTTATTAATAGCATAA 1619
AGCAAGTTTATGTCAAAAACATTCAGCTCCTGGGGCATCCGTAACTCAGAGATAACTGC 1675
CCTGATGCTGTTTATTTATCTTCCTTCTTTTTTTTCATGCCTTGTATTTATGATATTTA 1734
TATATTTTATATTTTCATCTTCACATCGTATTAAAATTTATAAAACATTCACTTTTTCA 1793

TATTAAGTTTGCATTTTGTTTTATTAAATTCATATCAAAGAAAACTCTGTAAATGTTTC 1852
TATTCTAAAAACAATGTCTACTTTCTCTTTTTGTAAACCAAATTGAAAATATGGTAAAA 1911
TGTATTAACTCATTCATTTCATTCCTATTATATGTATAAATTGAGTAAATGGCAAACTG 1970
TGGGGTTTTCTTAAAGAAATACAGGTGAATAAAGCAAACACAGTTTCTCTCAGTCTAAG 2029
AGGGAAAGAGACGTAAAAACAGGACAAATATTTATATTATTTCAATTATGTTAAATGCT 2088
ACAAAGAGAAGTAAAGAAAAGTGATGTTCTCACATCAGAAGCTT 2132

**12.** Interferon-pseudo-omega3-gene of formula

```
                                                       CCATG     5
CATAGCAGGAATGCCTTCAGAGAACCTGAAGTCCAAGGTTCATCCAGACCCCAGCTCAG     64
CTAGGCCAGCAGCACCCTCGTTTCCCA ATG GTC CTC CTG CTT CCT CTA CTC    115
GTG GCC CTG CCG CTT TGC CAC TGT GGC CCT GTT GGA TCT CTG AGC    160
TGG GAC CTG CCT CAG AAC CAT GGC CTA CTT AGC AGG AAC ACC TTG    205
GCA CTT CTG GGC CAA ATG TGC AGA ATC TCC ACT TTC TTG TGT CTC    250
AAG GAC AGA AGA GAC TTC AGG TTC CCC CTG GAG ATG TGG ATG GCA    295
GTC AGT TGC AGA AGG CCC CGG CCC TGT CTG TCC TCC ATG AGA TGC    340
TTC AGC AGA TCT TCA GCC TCT TCC CCA CAG AGT GCT CCT CTG CTG    385
CCT GGA ACA TGA CCCTCCTGGACCAACTCCACACTGGACTTCATCTGCAGCTGGA    440
ATGCCTGGATGCTTGCTTAGGGCAGACAAAAAGAGAGGAAGAATCTGTGGGGGTGATTG    499
GGGCCCTACACTGGCCTTGAGGACGTACTTTCAGGGAATGCATGGGAATCCAGGGAATC    558
TACCTGGAGGAGAAGAAATACAGTGACTGTGCTTGGGAGGTTGTCAGAGTGGAATCGTG    617
AAATCCTTCTCTTCATCATCAACAAACTTGCAAGAAGGACTGAGAAGTAAGGATGAAGA    676
CCTGGGTTCATCCTGAAATTATTCTCATTGATTAATCTGCCATATCACACTTGCACATG    735
TGTCTTTGGTCATTTCAATAGGTTCTTATTTCTGCAG                          772
```

**13.** Interferon-pseudo-omega4 gene of formula

```
                             AAGCTTTGGGCATGACCTAGTAGGTGACTCTT     32
AGTTGGAGTGGTCAGTTGTAAGGCTCTTGCTCAGTCACGTGGCTCCCCTGTATTTCCCC     91
ACGTTTGCAGCCGTGCTCCTTCTCAATGCTATGAAAGTGTGGGCTCCTCTCCCACTGGA    150
GTGCTGACTGTAGCTTGTATCTTGGCACTCCCAGGCTGTACATAACAGCTCTGAGGTGA    209
TCTCAGGGTTAATGTTTTCTCCCCGACTTGGAGGCCATTGAAGGAAGGGACCTTAGTAG    268
TAGTTGTAACTGAGGGTCTTTTGCTTGTCTCCTGGGGGCTCCACCCCAGAGATGCAGGT    327
```

GAGCAATCACTCAGTGCATTCAGCTTGGGATGGGGGTGTCTGTGCTGTGGGCCCAAGAC 386
AGGGGTTCCCTGCCTGGTGATGTGAGGGGTGGGTGGTTGACCAATGGCAGACAGACTGG 445
CCTCCTCTCTTGGGTTGACAGCAGCTTGTTGGAGGTATGCATAAGGCACTTGGGGTCTT 504
GCTCCTTCATTAGTTCCAAGGTAGCTGGGGTAGTACCACTGCAGAGGCAGTGTTAGACA 563
GGCTTTCTGTTACCCCTGGGGTCTCCACCTCCTAGAAATGTGAAGTCATGTTAATGGGA 622
GTGTTTAGCCAGAGGAGTGGGGTGGCTGCATGCTGGTGTAAGTATGGGACTTCACTTCT 681
TGGGAAACAGGGAGGTGGAATCTTACCAGCAGGAGACTGTTCTCCTCACGATGTGTAAC 740
CTGCAGTGTGCTGGAAGTTTAGGTGACTGTGGCATGATGTTAGCTTGTAAACAAAGAGC 799
TTCAGACTCTTTGTCTCTTCCCCAGACCCAAGGCAGCAAGGATAAAAGCTGCTGCTGTG 858
GCAGTGGCAGAGGTAGGATGGTTGTGGAGCCTCTCCCCAGGGAAACTCTAGTCAACTA 917
CCAGTGGATATGCTCAGCCATGGGTAGGGTGACTGTTCTGCAGTCATGGGCAGGGGGCC 976
TGCTCCTGAAGAATAGGGACAGGGATTCTCAGGGAAGAGGGGCTGGACTCCTCTTCATA 1035
TAGTGGCGATGATGTGCTGGAGGTGCCAGTGTAGTGAATAGGCCTTTGTTCCTTCCCCA 1094
GCCAGAGGGCTGCTGGGGCTGTCCCACTGCAACGGTCATGGTGGATGGGTTATGGGTTG 1153
ACTGTGGGATTTCTTTCTTGGAGAAATGCTGGACTGCCTGATTGAGGAGACGAGGCAAG 1212
GGAAGAATGCCTGTGCTGGAGTCTCTGGTCAGGTGGCTCTGCCACAGAGGAGAGGTGAC 1271
CACCAGGAACTGCGTGGAGAACAGTGTAGCCACTCTTCTGTGAGGCAGTTGCTCTGTTT 1330
TGGGGATCTGGAGCAGCCGCTATTCCTTACAGATTCCCAGAGCCTGGAGACAGCAAGGG 1389
CAAGAGCTGTGAGAAAGCAAAGATAGCAACCCACCCTTCTCACTGGGAGCTCTGTTCCA 1448
GGGAGATGCAGAGCTGCCATTGCTCAATAGCCCCAGCTGGTAGCTGCAGACCCAGGCCT 1507
GGCAGACCCACCCAGTGAGCAGATAGGGGATTAGGGACCCACATAACACACAGTCTGGC 1566
CACTTTTCCATAGGGCTGCTGAATATGCTGGGGGTCCAATCCAGACCATAGTCACCTCA 1625
CATTTTTCAGTACCTGAAGATATCAACAGTGAAGGCTATGAAACAGTGAAGATGGGGAC 1684
CTGCCCCTGCCTCTGGACCTCTGTTCCAGAGAGGTACAACCTGTTGCCTCCGACATACA 1743
TGCAGGAGGTGGCTGGAGACCCGGTGGATATCCCTCCCACTGAGGAGAAGCAGCATCAG 1802
GGAATCAGGTGAAGAAACAGTCTGGCCACTTTTTGGTAGAGCAGCTGTGCTTGCTGGGG 1861
GTCTGCTACCACCCCCAGCAAAAAGAATGGCATTTGCAAGAATGGCTAAGGCTGCTAAA 1920
CAGCAACAATGGCAACCTACCATTCCCTTTGGAGCGCCATCCCAGGGATATTCGAAACT 1979
GCTGTCCACTAGAAAACAGTGGTGGAGGTGACTGGAGACCCCAGTGGAGAGTTTCACCT 2038
GGTGAAAAGAAACAGGATTTGGGATCGACATGAATAACCAATCTGACTGCTTCCCCGTA 2097
GAGCTGCTGGACTGTGCTGGGTGGCTGCTCCAGTCCCTAGCTGCCTTGGACTCCCGAGA 2156
ACCCAAAGGCTCCAATAGCTAAGATTGTGAAAGAGCAAAGATGGCAGCCCACCCCCTGC 2215
CACAGGGAGCTCCATGTCAGGGAGGTATGAGGCTGCTACCAGTGTCTGGCTGGATTCCC 2274
AAGTCGAGTGGGTCTTACCCTGAGACAGGCCATGGAAGGTGGGCCTGTCATTGTCACTG 2333
CCCAGCGCCCTGGATGAAACCCCTTTCCTAGGGGTATGTATAGGGGTCTAGCGTCCTGC 2392
TTGGCTGGAGTTATAGCTTCTTTTGTGGGGAGGCCTGGGTATCTAAGGCTCCAGGGTAC 2451

CCATGCATGCGAGAGCGGCTGCTCTGCTGAACCCTACGTAGCCCTGCATGTCAGACTAA 2510
ATGCCCTGGTAGAGTGGGTTCACTAGGAGATCTCCTGACCTGAGGATTGCAAAGATCTG 2569
TGGGAGAAGCGTGGGTCCCCAGGGCTGCTCACTTACTCACCACTTCCCTGGGCAGGAGA 2628
GGCTCCCCTGGCTCTGTGTCATCCTGGGGGGCAGTTGTCCTGCCTTACTTTGCTTTAT 2687
TCTCCATGGGTCAAGTTGTTTCTTGAGTCTCAATGTGTGCACCTGGTTTTTCAGTTG 2746
AAGGTGCTGTATTTACTTGCCCCTTCCATTTCTCTCCATGAGAGTGGCACACACTAGCA 2805
GGTTCCAGTCGGCCATCTTGCAACCCCTGAAAACTATTTGTTTCCAGCTATAAGCCATT 2864
GAGAGAACCTGGAGTGGCATAAAAAGAATGCCTCGGGGTTCATCCCGACCCCAGCTCAG 2923
CTAGGCCAGCAGCACCCTCGTTTCCCA ATG GTC CTA CTG CTT GTT CTA CTG 2974
GTG GCC CTG CTG CTT TGC CAA TGT GGC CCT GTT GGA TCT CTG GGC 3019
TTT GAC CTG CCT CAG AAC CGT GGC CTA CTT AGC AGG AAC ACC TTG 3064
GCA TTC TGG GCC AAA TGC AGA ATC TCC ACT TTC TTG TGT CTC AAG 3109
GAC AGA AGA GAC TTC AGG TTC CCC CTG GAG ATG TGG ATG GCA GTC 3154
ATT TGC AGA AGG CCC AGG CTG TGT CTG TCC TCC ATG AGA TGC TTC 3199
AGC AGA TCT TCA GCC TCT TCC CCA CAG AGC GCT CCT CTG CTG CCT 3244
GGA ACA TGA CCCTCCTGGACCAGCTCCACACTGGATTTCATCAGCAGCTCGAATAG 3300
CCTGGAGTCTTGCTTAGGGCAGGCAACAGGAGAGGAAGAATCTGTGGGGGTGATTGGGA 3359
CCCTACACTGGCCTTGAGGAGGTACTTCCAGGGAATCCATGGGAATCCAGAGAATCTAC 3418
CTGAAAGAGAAGAAATACAGTGACTGTGCTTAGGAGGTTGTCAGAATGGAATCATGAAA 3477
TCCTTCTCTTCATCAACAGACTTGCAAGGACTGAGAAGTAAGGATGAAGACCTGGGGTC 3536
TGCTTTACTCTTTCTTATTTCTTCCTCTTCCTTACTATGTGTTTATTTCTTCTTTTTC 3595
TAGTTCCTTAACTTGTAAGTAGTTCACTTGGTTTGAGGTCTTTCTTCTTTTTTAATATA 3654
AGCTT 3659

**14.** Transformed host organism which contains the genetic information as claimed in claims 1 to 10.

**15.** Host organism as claimed in claim 14, characterised in that the genetic sequence is contained in a vehicle which is replicatable in the host organism.

**16.** Host organism as claimed in claim 14, characterised in that it is a mammalian cell line or E. coli.

**17.** Host organism as claimed in claim 14, characterised in that it is E. coli with the DSM number 3003 or E. coli with the DSM number 3004.

**18.** Host organism as claimed in claim 17, characterised in that the vehicle additionally contains the replicon and control sequences required for expression.

**19.** Host organism as claimed in claim 18, characterised in that the vehicle contains the replicon and control sequences of the plasmid pER103 with DSM no. 2773 (see Fig. 6) required for expression.

**20.** Host organism as claimed in claim 20, transformed with an expression plasmid as claimed in claims 21, 22 or 24.

**21.** Expression plasmid for interferon-omega which is a derivative of plasmid pBR 322, characterised in that instead of the shorter EcoRI/BamHI fragment inherent in the plasmid, the plasmid pBR322 contains the DNA sequence of formula

```
EcoRI        Sau3A
gaattcacgctGATCGCTAAAACATTGTGCAAAAAGAGGGTTGACTTTGCCTTCGCGA  59

                 ↓mRNA-Start                                  Met
ACCAGTTAACTAGTACACAAGTTCACGGCAACGGTAAGGAGGTTTAAGCTTAAAG ATG 116
                                      RBS       HindIII

Cys Asp
TGT GAT C —— interferon-omega gene——→
    Sau3A
```

22. Expression plasmid pRHW12 as claimed in claim 21, characterised in that the interferon-omega-gene contains the DNA sequence of formula

```
TGT GAT CTG CCT CAG AAC CAT GGC CTA CTT AGC AGG AAC ACC TTG  28
                        NcoI
GTG CTT CTG CAC CAA ATG AGG AGA ATC TCC CCT TTC TTG TGT CTC  73
AAG GAC AGA AGA GAC TTC AGG TTC CCC CAG GAG ATG GTA AAA GGG 118
AGC CAG TTG CAG AAG GCC CAT GTC ATG TCT GTC CTC CAT GAG ATG 163
CTG CAG CAG ATC TTC AGC CTC TTC CAC ACA GAG CGC TCC TCT GCT 208
GCC TGG AAC ATG ACC CTC CTA GAC CAA CTC CAC ACT GGA CTT CAT 253
CAG CAA CTG CAA CAC CTG GAG ACC TGC TTG CTG CAG GTA GTG GGA 298
GAA GGA GAA TCT GCT GAG GCA ATT AGC AGC CCT GCA CTG ACC TTG 343
AGG AGG TAC TTC CAG GGA ATC CGT GTC TAC CTG AAA GAG AAG AAA 388
TAC AGC GAC TGT GCC TGG GAA GTT GTC AGA ATG GAA ATC ATG AAA 433
TCC TTG TTC TTA TCA ACA AAC ATG CAA GAA AGA CTG AGA AGT AAA 478
GAT AGA GAC CTG GGC TCA TCT TGAAATGATTCTCATTGATTAATTTGCCATA 530
TAACACTTGCACATGTGACTCTGGTCAATTCAAAAGACTCTTATTTCGGCTTTAATCAC 589
AGAATTGACTGAATTAGTTCTGCAAATACTTTGTCGGTATATTAAGCCAGTATATGTTA 648
AAAAGACTTAGGTTCAGGGGCATCAGTCCCTAAGATGTTATTTATTTTTACTCATTTAT 707
TTATTCTTACATTTTATCATATTTATACTATTTATATTCTTATATAACAAATGTTTGCC 766
TTTACATTGTATTAAGATAACAAAACATGTTCAGCt                        802
                                 AluI
```

23. Expression plasmid pRHW11 as claimed in claim 21, characterised in that the interferon-omega-gene contains the DNA sequence of formula

TGT GAT CTG CCT CAG AAC CAT GGC CTA CTT AGC AGG AAC ACC TTG 28
NcoI
GTG CTT CTG CAC CAA ATG AGG AGA ATC TCC CCT TTC TTG TGT CTC 73
AAG GAC AGA AGA GAC TTC AGG TTC CCC CAG GAG ATG GTA AAA GGG 118
AGC CAG TTG CAG AAG GCC CAT GTC ATG TCT GTC CTC CAT GAG ATG 163
CTG CAG CAG ATC TTC AGC CTC TTC CAC ACA GAG CGC TCC TCT GCT 208
GCC TGG AAC ATG ACC CTC CTA GAC CAA CTC CAC ACT GGA CTT CAT 253
CAG CAA CTG CAA CAC CTG GAG ACC TGC TTG CTG CAG GTA GTG GGA 298

GAA GGA GAA TCT GCT GAG GCA ATT AGC AGC CCT GCA CTG ACC TTG 343
AGG AGG TAC TTC CAG GGA ATC CGT GTC TAC CTG AAA GAG AAG AAA 388
TAC AGC GAC TGT GCC TGG GAA GTT GTC AGA ATG GAA ATC ATG AAA 433
TCC TTG TTC TTA TCA ACA AAC ATG CAA GAA AGA CTG AGA AGT AAA 478
GAT AGA GAC CTG GGC TCA TCT TGAAATGATTCTCATTGATTAATTTGCCATA 530
TAACACTTGCACATGTGACTCTGGTCAATTCAAAAGACTCTTATTTCGGCTTTAATCAC 589
AGAATTGACTGAATTAGTTCTGCAAATACTTTGTCGGTATATTAAGCCAGTATATGTTA 648
AAAAGACTTAGGTTCAGGGGCATCAGTCCCTAAGATGTTATTTATTTTTACTCATTTAT 707
TTATTCTTACATTTTATCATATTTATACTATTTATATTCTTATATAACAAATGTTTGCC 766
TTTACATTGTATTAAGATAACAAAACATGTTCAGct 802
AluI

24. Plasmid PRHW10 which is a derivative of plasmid pBR 322, characterised in that the DNA fragment of formula

HindIII Sau3A NcoI
aAGCTTAAAG ATGTGTGATC TGCCTCAGAA CCATGGCCTA CTTAGCAGGA 50

ACACCTTGGT GCTTCTGCAC CAAATGAGGA GAATCTCCCC TTTCTTGTGT 100

CTCAAGGACA GAAGAGACTT CAGGTTCCCC CAGGAGATGG TAAAAGGGAG 150

CCAGTTGCAG AAGGCCCATG TCATGTCTGT CCTCCATGAG ATGCTGCAGC 200

AGATCACACA TCTTTAagct t
Sau3A HindIII

is inserted in the plasmid pER103, DSM no. 2773 (see Fig. 6), in the HindIII site.

25. E. coli HB 101 transformed with a plasmid as claimed in claim 21, 22, 23 or 24.

26. New human type I interferon proteins, characterised in that they are coded by a DNA molecule as claimed in one of claims 1 to 10.

**27.** New human interferons as claimed in claim 26, characterised in that the mature interferons have a divergence of 30 to 50% compared with human α-interferons.

**28.** New human interferons of type I as claimed in claim 26, characterised in that
a) the mature interferons have a divergence of 30 to 50% compared with human α-interferons and a divergence of approximately 70% compared with β-interferon and
b) they are 168 to 174 amino acids long, and the N-glycosylated derivatives thereof.

**29.** Interferons as claimed in claim 28, characterised in that they have a divergence of from 40 to 48% compared with human α-interferons, and the N-glycosylated derivatives thereof.

**30.** Interferons as claimed in claims 26 to 29, characterised in that they contain a leader peptide.

**31.** Interferons as claimed in claims 26 to 29, characterised in that they contain 172 amino acids.

**32.** Interferon as claimed in claim 31, characterised in that it contains the amino acid sequence

```
                    5                   10                  15
Cys Asp Leu Pro Gln Asn His Gly Leu Leu Ser Arg Asn Thr Leu

                    20                  25                  30
Val Leu Leu His Gln Met Arg Arg Ile Ser Pro Phe Leu Cys Leu

                    35                  40                  45
Lys Asp Arg Arg Asp Phe Arg Phe Pro Gln Glu Met Val Lys Gly

                    50                  55                  60
Ser Gln Leu Gln Lys Ala His Val Met Ser Val Leu His Glu Met

                    65                  70                  75
Leu Gln Gln Ile Phe Ser Leu Phe His Thr Glu Arg Ser Ser Ala

                    80                  85                  90
Ala Trp Asn Met Thr Leu Leu Asp Gln Leu His Thr Gly Leu His

                    95                  100                 105
Gln Gln Leu Gln His Leu Glu Thr Cys Leu Leu Gln Val Val Gly

                    110                 115                 120
Glu Gly Glu Ser Ala Gly Ala Ile Ser Ser Pro Ala Leu Thr Leu

                    125                 130                 135
Arg Arg Tyr Phe Gln Gly Ile Arg Val Tyr Leu Lys Glu Lys Lys

                    140                 145                 150
Tyr Ser Asp Cys Ala Trp Glu Val Val Arg Met Glu Ile Met Lys

                    155                 160                 165
Ser Leu Phe Leu Ser Thr Asn Met Gln Glu Arg Leu Arg Ser Lys

                    170
Asp Arg Asp Leu Gly Ser Ser
```

and the derivatives thereof N-glycolysed in amino acid position 78.

**33.** Interferon as claimed in claim 31, characterised in that the interferon as claimed in claim 32 contains in position 111 the amino acid Glu instead of Gly, and the derivatives thereof N-glycolysed in amino acid position 78.

**34.** Interferons as claimed in claims 30 to 33, characterised in that they contain the leader peptide of formula

Met Ala Leu Leu Phe Pro Leu Leu Ala Ala Leu Val Met Thr Ser Tyr Ser Pro Val Gly Ser Leu Gly.

**35.** Process for preparing the interferon peptides as claimed in claims 26 to 34, characterised in that

a suitable host organism is transformed with genetic information coding for the interferon proteins as claimed in claims 26 to 34,

this information is expressed in order to produce the corresponding interferon in the host organism and

the interferon peptide thus obtained is isolated from this host organism.

**36.** Process as claimed in claim 35, characterised in that the information is contained in each recombinant DNA molecule as claimed in claims 1 to 10.

**37.** Process as claimed in claim 36, characterised in that after transformation the host organism is defined according to one of the claims 15 to 20.

**38.** Process as claimed in claim 35, characterised in that the interferon is defined according to claims 32 to 34.

**39.** Process as claimed in claim 35, characterised in that E. coli HB101 is used as the host and a plasmid as claimed in claim 21, 22 or 23 is used as the plasmid.

**40.** omega-interferon which can be prepared by the process as claimed in claim 35 or 39.

**41.** Mixture suitable for antitumour or antiviral treatment, containing in addition to one or more inert carriers and/or diluents an effective quantity of interferon peptide as claimed in one of claims 26 to 34.

**42.** Use of omega-interferon as claimed in claims 30 to 34 for preparing a mixture suitable for antitumour or antiviral treatment.

**43.** Process for preparing the plasmid pRHW10, characterised in that the DNA fragment of formula

```
HindIII              Sau3A               NcoI
aAGCTTAAAG ATGTGTGATC TGCCTCAGAA CCATGGCCTA CTTAGCAGGA     50

ACACCTTGGT GCTTCTGCAC CAAATGAGGA GAATCTCCCC TTTCTTGTGT    100

CTCAAGGACA GAAGAGACTT CAGGTTCCCC CAGGAGATGG TAAAAGGGAG    150
```

```
CCAGTTGCAG AAGGCCCATG TCATGTCTGT CCTCCATGAG ATGCTGCAGC     200

AGATCACACA TCTTTAagct t
 Sau3A            HindIII
```

is inserted into the plasmid pER103, DSM no. 2773 (Fig. 6), at the HindIII site by ligase reaction, and the resulting plasmid is transformed for replication in E. coli HB 101 and cultivated.

**44.** Process for preparing the expression plasmid pRHW12, characterised in that the fragment of formula

```
                          c CAT GGC CTA CTT AGC AGG AAC ACC TTG  28
                            NcoI
GTG CTT CTG CAC CAA ATG AGG AGA ATC TCC CCT TTC TTG TGT CTC  73
AAG GAC AGA AGA GAC TTC AGG TTC CCC CAG GAG ATG GTA AAA GGG 118
AGC CAG TTG CAG AAG GCC CAT GTC ATG TCT GTC CTC CAT GAG ATG 163
CTG CAG CAG ATC TTC AGC CTC TTC CAC ACA GAG CGC TCC TCT GCT 208
GCC TGG AAC ATG ACC CTC CTA GAC CAA CTC CAC ACT GGA CTT CAT 253
CAG CAA CTG CAA CAC CTG GAG ACC TGC TTG CTG CAG GTA GTG GGA 298
GAA GGA GAA TCT GCT GGG GCA ATT AGC AGC CCT GCA CTG ACC TTG 343
AGG AGG TAC TTC CAG GGA ATC CGT GTC TAC CTG AAA GAG AAG AAA 388
TAC AGC GAC TGT GCC TGG GAA GTT GTC AGA ATG GAA ATC ATG AAA 433
TCC TTG TTC TTA TCA ACA AAC ATG CAA GAA AGA CTG AGA AGT AAA 478
GAT AGA GAC CTG GGC TCA TCT TGAAATGATTCTCATTGATTAATTTGCCATA 530
TAACACTTGCACATGTGACTCTGGTCAATTCAAAAGACTCTTATTTCGGCTTTAATCAC 589
AGAATTGACTGAATTAGTTCTGCAAATACTTTGTCGGTATATTAAGCCAGTATATGTTA 648
AAAAGACTTAGGTTCAGGGGCATCAGTCCCTAAGATGTTATTTATTTTTACTCATTTAT 707
TTATTCTTACATTTTATCATATTTATACTATTTATATTCTTATATAACAAATGTTTGCC 766
TTTACATTGTATTAAGATAACAAAACATGTTCAGct                        802
                                AluI
```

obtained by digesting the AvaII fragment of clone P9A2 (DSM no. 3004) with NcoI and AluI is inserted by ligase reaction into the larger fragment of plasmid pRHW10, which contains in the HindIII site of the plasmid pER103, DSM no. 2773, (Fig. 6), the DNA fragment of formula

```
HindIII              Sau3A               NcoI
aAGCTTAAAG ATGTGTGATC TGCCTCAGAA CCATGGCCTA CTTAGCAGGA    50

ACACCTTGGT GCTTCTGCAC CAAATGAGGA GAATCTCCCC TTTCTTGTGT   100

CTCAAGGACA GAAGAGACTT CAGGTTCCCC CAGGAGATGG TAAAAGGGAG   150

CCAGTTGCAG AAGGCCCATG TCATGTCTGT CCTCCATGAG ATGCTGCAGC   200

AGATCACACA TCTTTAagct t
 Sau3A          HindIII
```

this larger fragment of the plasmid pRHW10 having been obtained by restriction endonuclease digestion with BamHI, filling the cutting sites with the aid of the Klenow fragment of DNA polymerase I and the 4 deoxynucleoside triphosphates and subsequent cutting with NcoI, and the resulting plasmid is transformed and cultivated in E. coli HB 101 for replication.

45. Process for preparing the expression plasmid pHWR11, characterised in that the fragment of formula

```
                          c CAT GGC CTA CTT AGC AGG AAC ACC TTG   28
                            NcoI
GTG CTT CTG CAC CAA ATG AGG AGA ATC TCC CCT TTC TTG TGT CTC   73
AAG GAC AGA AGA GAC TTC AGG TTC CCC CAG GAG ATG GTA AAA GGG 118
AGC CAG TTG CAG AAG GCC CAT GTC ATG TCT GTC CTC CAT GAG ATG 163
CTG CAG CAG ATC TTC AGC CTC TTC CAC ACA GAG CGC TCC TCT GCT 208
GCC TGG AAC ATG ACC CTC CTA GAC CAA CTC CAC ACT GGA CTT CAT 253
CAG CAA CTG CAA CAC CTG GAG ACC TGC TTG CTG CAG GTA GTG GGA 298
GAA GGA GAA TCT GCT GAG GCA ATT AGC AGC CCT GCA CTG ACC TTG 343
AGG AGG TAC TTC CAG GGA ATC CGT GTC TAC CTG AAA GAG AAG AAA 388
TAC AGC GAC TGT GCC TGG GAA GTT GTC AGA ATG GAA ATC ATG AAA 433
TCC TTG TTC TTA TCA ACA AAC ATG CAA GAA AGA CTG AGA AGT AAA 478
GAT AGA GAC CTG GGC TCA TCT TGAAATGATTCTCATTGATTAATTTGCCATA 530
TAACACTTGCACATGTGACTCTGGTCAATTCAAAAGACTCTTATTTCGGCTTTAATCAC 589
AGAATTGACTGAATTAGTTCTGCAAATACTTTGTCGGTATATTAAGCCAGTATATGTTA 648

AAAAGACTTAGGTTCAGGGGCATCAGTCCCTAAGATGTTATTTATTTTTACTCATTTAT 707
TTATTCTTACATTTTATCATATTTATACTATTTATATTCTTATATAACAAATGTTTGCC 766
TTTACATTGTATTAAGATAACAAAACATGTTCAGct                        802
                                  AluI
```

obtained by digesting the AvaII fragment of clone E76E9 (DSM no. 3003) with NcoI and AluI is inserted by ligase reaction into the larger fragment of plasmid pRHW10, which contains in the HindIII site of the plasmid pER103 (DSM no. 2773, see Fig. 6) the DNA fragment of formula

```
HindIII              Sau3A                NcoI
aAGCTTAAAG ATGTGTGATC TGCCTCAGAA CCATGGCCTA CTTAGCAGGA     50

ACACCTTGGT GCTTCTGCAC CAAATGAGGA GAATCTCCCC TTTCTTGTGT    100

CTCAAGGACA GAAGAGACTT CAGGTTCCCC CAGGAGATGG TAAAAGGGAG    150

CCAGTTGCAG AAGGCCCATG TCATGTCTGT CCTCCATGAG ATGCTGCAGC    200

AGATCACACA TCTTTAagct t
 Sau3A          HindIII
```

this larger fragment of the plasmid pRHW10 having been obtained by restriction endonuclease digestion with BamHI, filling the cutting sites with the aid of the Klenow fragment of DNA polymerase I and the 4 deoxynucleoside triphosphates and subsequent cutting with NcoI, and the resulting plasmid is transformed and cultivated in E. coli HB 101 for replication.

**46.** Synergistic mixture according to claim 41, characterised in that it additionally contains $\gamma$-interferon.

**47.** Synergistic mixture according to claim 41, characterised in that it additionally contains human tumour necrosis factor.

**Revendications**

**1.** Molécules d'ADN recombiné, contenant une séquence codante pour de nouvelles protéines d'interféron humaines de type I, qui contiennent 168-174 acides aminés, pour interféron pseudo-oméga 2 (voir figure 12), interféron pseudo oméga 3 (voir figure 13) ou pour interféron pseudo-oméga 4 (voir figure 14), caractérisées en ce que la séquence codante s'hybride dans des conditions sévères, qui ne permettent de reconnaître qu'une homologie supérieure à 85%, avec l'insert du site de coupure Pst-1 a) du plasmide E76E9 dans E. coli HB 101, déposé auprès de la DSM sous le numéro DSM 3003 ou b) du plasmide P9A2 dans E. coli HB 101, déposé auprès de la DSM sous le numéro DSM 3004.

**2.** Molécule d'ADN selon la revendication 1, caractérisée en ce que les conditions sévères ne permettent la mise en évidence que d'une homologie supérieure à 90%.

**3.** Molécule d'ADN selon les revendications 1 et 2, caractérisée en ce que la séquence codante est présente sous forme d'insert dans le site de coupure Pst-1 a) du plasmide E76E9 dans E. coli HB 101, déposé auprès de la DSM sous le numéro DSM 3003 ou b) du plasmide P9A2 dans E. coli HB 101, déposé auprès de la DSM sous le numéro DSM 3004, ou est un variant dégénéré de cet insert.

**4.** Molécule d'ADN selon les revendications 1 à 3, caractérisée en ce que le vecteur est un plasmide qui est réplicable chez les procaryotes ou les eucaryotes.

**5.** Molécule d'ADN selon la revendication 4, caractérisée en ce qu'elle est un vecteur d'expression réplicable dans les micro-organismes ou les cellules de mammifères.

**6.** Molécule d'ADN selon la revendication 5, contenant la séquence

```
TGT GAT CTG CCT CAG AAC CAT GGC CTA CTT AGC AGG AAC ACC TTG  45
GTG CTT CTG CAC CAA ATG AGG AGA ATC TCC CCT TTC TTG TGT CTC  90
AAG GAC AGA AGA GAC TTC AGG TTC CCC CAG GAG ATG GTA AAA GGG 135
AGC CAG TTG CAG AAG GCC CAT GTC ATG TCT GTC CTC CAT GAG ATG 180
CTG CAG CAG ATC TTC AGC CTC TTC CAC ACA GAG CGC TCC TCT GCT 225
GCC TGG AAC ATG ACC CTC CTA GAC CAA CTC CAC ACT GGA CTT CAT 270
CAG CAA CTG CAA CAC CTG GAG ACC TGC TTG CTG CAG GTA GTG GGA 315
GAA GGA GAA TCT GCT GGG GCA ATT AGC AGC CCT GCA CTG ACC TTG 360
AGG AGG TAC TTC CAG GGA ATC CGT GTC TAC CTG AAA GAG AAG AAA 405
TAC AGC GAC TGT GCC TGG GAA GTT GTC AGA ATG GAA ATC ATG AAA 450
TCC TTG TTC TTA TCA ACA AAC ATG CAA GAA AGA CTG AGA AGT AAA 495
GAT AGA GAC CTG GGC TCA TCT                                 516
```

7. Molécule d'ADN selon la revendication 5, caractérisée en ce que la molécule d'ADN selon la revendication 6 contient en position 332 le nucléotide A à la place du nucléotide G.

8. Molécule d'ADN selon la revendication 5, caractérisée en ce qu'elle est a) le plasmide E76E9 dans E. coli HB 101 déposé auprès de la DSM sous le numéro DSM 3003 ou b) le plasmide P9A2 dans E. coli HB 101, déposé auprès de la DSM sous le numéro DSM 3004.

9. Molécule d'ADN selon les revendications 6 et 7, caractérisée en ce qu'elle contient en outre la séquence d'ADN de formule ATG GCC CTC CTG TTC CCT CTA CTG GCA GCC CTA GTG ATG ACC AGC TAT AGC CCT GTT GGA TCT CTG GGC codant pour le peptide de tête.

10. Gène de l'interféron oméga 1 de formule

```
                                             GATCTGGTAAACCTGAA   17
GCAAATATAGAAACCTATAGGGCCTGACTTCCTACATAAAGTAAGGAGGGTAAAAATGG   76
AGGCTAGAATAAGGGTTAAAATTTTGCTTCTAGAACAGAGAAAATGATTTTTTTCATAT  135
ATATATGAATATATATTATATATACACATATATACATATATTCACTATAGTGTGTATAC  194
```

ATAAATATATAATATATATATTGTTAGTGTAGTGTGTGTCTGATTATTTACATGCATAT 253
AGTATATACACTTATGACTTTAGTACCCAGACGTTTTTCATTTGATTAAGCATTCATTT 312
GTATTGACACAGCTGAAGTTTACTGGAGTTTAGCTGAAGTCTAATGCAAAATTAATAGA 371
TTGTTGTCATCCTCTTAAGGTCATAGGGAGAACACACAAATGAAAACAGTAAAAGAAAC 430
TGAAAGTACAGAGAAATGTTCAGAAAATGAAAACCATGTGTTTCCTATTAAAAGCCATG 489
CATACAAGCAATGTCTTCAGAAAACCTAGGGTCCAAGGTTAAGCCATATCCCAGCTCAG 548
TAAAGCCAGGAGCATCCTCATTTCCCA ATG GCC CTC CTG TTC CCT CTA CTG 599
GCA GCC CTA GTG ATG ACC AGC TAT AGC CCT GTT GGA TCT CTG GGC 644
TGT GAT CTG CCT CAG AAC CAT GGC CTA CTT AGC AGG AAC ACC TTG 689
GTG CTT CTG CAC CAA ATG AGG AGA ATC TCC CCT TTC TTG TGT CTC 734
AAG GAC AGA AGA GAC TTC AGG TTC CCC CAG GAG ATG GTA AAA GGG 779
AGC CAG TTG CAG AAG GCC CAT GTC ATG TCT GTC CTC CAT GAG ATG 824
CTG CAG CAG ATC TTC AGC CTC TTC CAC ACA GAG CGC TCC TCT GCT 869
GCC TGG AAC ATG ACC CTC CTA GAC CAA CTC CAC ACT GGA CTT CAT 914
CAG CAA CTG CAA CAC CTG GAG ACC TGC TTG CTG CAG GTA GTG GGA 959
GAA GGA GAA TCT GCT GGG GCA ATT AGC AGC CCT GCA CTG ACC TTG 1004
AGG AGG TAC TTC CAG GGA ATC CGT GTC TAC CTG AAA GAG AAG AAA 1045
TAC AGC GAC TGT GCC TGG GAA GTT GTC AGA ATG GAA ATC ATG AAA 1094
TCC TTG TTC TTA TCA ACA AAC ATG CAA GAA AGA CTG AGA AGT AAA 1139
GAT AGA GAC CTG GGC TCA TCT TGA AATGATTCTCATTGATTAATTTGCCAT 1190
ATAACACTTGCACATGTGACTCTGGTCAATTCAAAAGACTCTTATTTCGGCTTTAATCA 1249
CAGAATTGACTGAATTAGTTCTGCAAATACTTTGTCGGTATATTAAGCCAGTATATGTT 1308
AAAAAGACTTAGGTTCAGGGGCATCAGTCCCTAAGATGTTATTTATTTTTACTCATTTA 1367
TTTATTCTTACATTTTATCATATTTATACTATTTATATTCTTATATAACAAATGTTTGC 1426
CTTTACATTGTATTAAGATAACAAAACATGTTCAGCTTTCCATTTGGTTAAATATTGTA 1485
TTTTGTTATTTATTAAATTATTTTCAAACAAAACTTCTTGAAGTTATTTATTCGAAAAC 1544
CAAAATCCAAACACTAGTTTTCTGAACCAAATCAAGGAATGGACGGTAATATACACTTA 1603
CCTATTCATTCATTCCATTTACATAATATGTATAAAGTGAGTATCAAAGTGGCATATTT 1662
TGGAATTGATGTCAAGCAATGCAGGTGTACTCATTGCATGACTGTATCAAAATATCTCA 1721
TGTAACCAATAAATATATACACTTACTATGTATCCCACAAAAATTAAAAAGTTATTTTA 1780
AAAAAGAAATACAGGTGAATAAACACAGTTTCTTTCCGTGTTGAAGAGCTTTCATTCTT 1839
ACAGGAAAAGAAACAGTAAAGATGTACCAATTTCGCTTATATGAAACACTACAAAGATA 1898
AGTAAAAGAAAATGATGTTCTCATACTAGAAGCTT 1933

**11.** Gène de l'interféron pseudo-oméga 2 de formule

```
          AAGCTTGAGCCCCCAGGGAAGCATAACCACATGAACCTGAATGAATATATT   51
CTAGAAGGAGGGAAGCACCAGAGAAGTTCTTTCACTAATAACCATCAACGTCTTCTGTG  110
AATCAAATATCAAACAAAGATAGTCCTAAAAAGTTTAATTTCCAGAGATAGGTAATTTC  169
CTAACTGAATACAGAAACCCATAGGGCCCAGGGATCCTGATTTCCTATGCAAAATGGAG  228
GGTAAAACTGGAGGCTAGGATCTGGGCTAAAAGTATATACTTCTAACAGTAGCACAAAG  287
ATGTTTCTCATCTGATTGATCAATATTCATTTGGATTGATATATCTTAAGTTTACTGGG  346
AATATTGAACATCCATTGCAAAAATCAAGAGTGTAGAGTGATGACCTCCTTTTAGGTCA  405
TATAGAACAAGGTTTTTCAACCCCCATCCATGGACCGGGGTACTGGTCCTGGCCTGGTA  464
GGAACAGGGCCGCACAGCAGGAGGCAAGCAGGCCAACCAACAAGCATTAACGCCTGAGC  523
TCTGCCTCCTGTCAGATCAGCAGTGGCATTGGATTCTCAAAAGAGCAGGAACCCTATTG  582
TGAAGTGCAGATGCGAAGGATCTAGGTTGTGGTCTCCTAATGAGAATCTAATGCCTCTG  641
AAAGCATTCCCTCCCTGACCCCATTTTTCGTGGAAAAATTATCTTCCACCAAACTGGTG  700
GCCAAAAGGTTGTGGATGCTGATATAGAAGACATGTAAATGAAAACAATAAATGGAATT  759
AAAAATTTAGAGAAATGCTCAGAAAAATGAAAACTATTTGTGCTCCATTAAAGCCATGC  818
ATAGATAGAATGTCTTCATAGAACCTAGGATCCAAGGTTCTATGAAGACCTCAGCTCAA  877
CCAGGCCAAAAGCATCCTGATTTCTCA ATG GCC CTC CTC TTC CCT CTA CTG  928
GCA GCC CTA GAG GTG TGC AGC TGT GGC TCT TCT GGA TCT CTA GGA  973
TAT AAC CTG CCT CAG AAC CAT GGC CTG CTA GGC AGG AAC ACC TTG 1018
GTG CTT TTG GGC CAA ATG AGG AGA ATC TCT CCC TTC TTG TGT CTA 1063
AAG GAC AGA AGT GAC TTC AGA TTC CCC CAG GAG AAG GTG GAA GTC 1108
AGC CAG TTG CAG AAG GCC CAG GCT ATG TCT TTC CTC TAT GAT GTG 1153
TTA CAG CAG GTC TTC AAC TTC TCA CAC AAA GCG CTC CTC TGC TGC 1198
ATG GAA CAT GAC CTT CCT GGA CCA ACT CCA CAC TTT ACG TCA TCA 1243
GCA GCT GGA ACA CCT GGA GAC CTG CTT GGT GCA GGA GAT GGG AGA 1288
AGG AGA AGC TGG GGG CAG TGG GTG ATT GAG GGC TCT ACA CTG GCC 1333
TTG AGG AGG TAT TTC CAG GAA TCC ATC TCT ACC TGA AAGAGAAGAAA 1380
TAAAGATTGTGCCTGGGAAGTTGTCAGAGTGGAAATCATGAGATCCTTTTCATCCACAA 1439
GTTTGCAAGAAAGATTGAGAAGTAAGGATGAAGACCTGGGCTCATCATGAAATGATTCT 1498
CATTGACTAATCTGCCATATCACACTTGTACATGTGACTTTGGATATTCAAAAAGCTCA 1557
TTTCTGTTTCATCAGAAATTATTGAATTAGTTTTAGCAAATACTTTATTAATAGCATAA 1619
AGCAAGTTTATGTCAAAAACATTCAGCTCCTGGGGCATCCGTAACTCAGAGATAACTGC 1675
CCTGATGCTGTTTATTTATCTTCCTTCTTTTTTTTCATGCCTTGTATTTATGATATTTA 1734
TATATTTTATATTTTCATCTTCACATCGTATTAAAATTTATAAAACATTCACTTTTTCA 1793
TATTAAGTTTGCATTTTGTTTTATTAAATTCATATCAAAGAAAACTCTGTAAATGTTTC 1852

TATTCTAAAAACAATGTCTACTTTCTCTTTTTGTAAACCAAATTGAAAATATGGTAAAA 1911
TGTATTAACTCATTCATTTCATTCCTATTATATGTATAAATTGAGTAAATGGCAAACTG 1970
TGGGGTTTTCTTAAAGAAATACAGGTGAATAAAGCAAACACAGTTTCTCTCAGTCTAAG 2029
AGGGAAAGAGACGTAAAAACAGGACAAATATTTATATTATTTCAATTATGTTAAATGCT 2088
ACAAAGAGAAGTAAAGAAAAGTGATGTTCTCACATCAGAAGCTT                2132
```

**12.** Gène de l'interféron pseudo-oméga 3 de formule

```
                                                        CCATG   5
CATAGCAGGAATGCCTTCAGAGAACCTGAAGTCCAAGGTTCATCCAGACCCCAGCTCAG  64
CTAGGCCAGCAGCACCCTCGTTTCCCA ATG GTC CTC CTG CTT CCT CTA CTC 115
GTG GCC CTG CCG CTT TGC CAC TGT GGC CCT GTT GGA TCT CTG AGC 160
TGG GAC CTG CCT CAG AAC CAT GGC CTA CTT AGC AGG AAC ACC TTG 205
GCA CTT CTG GGC CAA ATG TGC AGA ATC TCC ACT TTC TTG TGT CTC 250
AAG GAC AGA AGA GAC TTC AGG TTC CCC CTG GAG ATG TGG ATG GCA 295
GTC AGT TGC AGA AGG CCC CGG CCC TGT CTG TCC TCC ATG AGA TGC 340
TTC AGC AGA TCT TCA GCC TCT TCC CCA CAG AGT GCT CCT CTG CTG 385
CCT GGA ACA TGA CCCTCCTGGACCAACTCCACACTGGACTTCATCTGCAGCTGGA 440
ATGCCTGGATGCTTGCTTAGGGCAGACAAAAAGAGAGGAAGAATCTGTGGGGGTGATTG 499
GGGCCCTACACTGGCCTTGAGGACGTACTTTCAGGGAATGCATGGGAATCCAGGGAATC 558
TACCTGGAGGAGAAGAAATACAGTGACTGTGCTTGGGAGGTTGTCAGAGTGGAATCGTG 617
AAATCCTTCTCTTCATCATCAACAAACTTGCAAGAAGGACTGAGAAGTAAGGATGAAGA 676
CCTGGGTTCATCCTGAAATTATTCTCATTGATTAATCTGCCATATCACACTTGCACATG 735
TGTCTTTGGTCATTTCAATAGGTTCTTATTTCTGCAG                       772
```

**13.** Gène de l'interféron pseudo-oméga 4 de formule

```
                              AAGCTTTGGGCATGACCTAGTAGGTGACTCTT  32
AGTTGGAGTGGTCAGTTGTAAGGCTCTTGCTCAGTCACGTGGCTCCCCTGTATTTCCCC  91
ACGTTTGCAGCCGTGCTCCTTCTCAATGCTATGAAAGTGTGGGCTCCTCTCCCACTGGA 150
GTGCTGACTGTAGCTTGTATCTTGGCACTCCCAGGCTGTACATAACAGCTCTGAGGTGA 209
TCTCAGGGTTAATGTTTTCTCCCCGACTTGGAGGCCATTGAAGGAAGGGACCTTAGTAG 268
TAGTTGTAACTGAGGGTCTTTTGCTTGTCTCCTGGGGGCTCCACCCCAGAGATGCAGGT 327
GAGCAATCACTCAGTGCATTCAGCTTGGGATGGGGGTGTCTGTGCTGTGGGCCCAAGAC 386
AGGGGTTCCCTGCCTGGTGATGTGAGGGGTGGGTGGTTGACCAATGGCAGACAGACTGG 445
CCTCCTCTCTTGGGTTGACAGCAGCTTGTTGGAGGTATGCATAAGGCACTTGGGGTCTT 504
GCTCCTTCATTAGTTCCAAGGTAGCTGGGGTAGTACCACTGCAGAGGCAGTGTTAGACA 563
GGCTTTCTGTTACCCCTGGGGTCTCCACCTCCTAGAAATGTGAAGTCATGTTAATGGGA 622
```

GTGTTTAGCCAGAGGAGTGGGGTGGCTGCATGCTGGTGTAAGTATGGGACTTCACTTCT 681
TGGGAAACAGGGAGGTGGAATCTTACCAGCAGGAGACTGTTCTCCTCACGATGTGTAAC 740
CTGCAGTGTGCTGGAAGTTTAGGTGACTGTGGCATGATGTTAGCTTGTAAACAAAGAGC 799
TTCAGACTCTTTGTCTCTTCCCCAGACCCAAGGCAGCAAGGATAAAAGCTGCTGCTGTG 858
GCAGTGGCAGAGGTAGGATGGTTGTGGGAGCCTCTCCCCAGGGAAACTCTAGTCAACTA 917
CCAGTGGATATGCTCAGCCATGGGTAGGGTGACTGTTCTGCAGTCATGGGCAGGGGGCC 976
TGCTCCTGAAGAATAGGGACAGGGATTCTCAGGGAAGAGGGGCTGGACTCCTCTTCATA 1035
TAGTGGCGATGATGTGCTGGAGGTGCCAGTGTAGTGAATAGGCCTTTGTTCCTTCCCCA 1094
GCCAGAGGGCTGCTGGGGCTGTCCCACTGCAACGGTCATGGTGGATGGGTTATGGGTTG 1153
ACTGTGGGATTTCTTTCTTGGAGAAATGCTGGACTGCCTGATTGAGGAGACGAGGCAAG 1212
GGAAGAATGCCTGTGCTGGAGTCTCTGGTCAGGTGGCTCTGCCACAGAGGAGAGGTGAC 1271
CACCAGGAACTGCGTGGAGAACAGTGTAGCCACTCTTCTGTGAGGCAGTTGCTCTGTTT 1330
TGGGGATCTGGAGCAGCCGCTATTCCTTACAGATTCCCAGAGCCTGGAGACAGCAAGGG 1389
CAAGAGCTGTGAGAAAGCAAAGATAGCAACCCACCCTTCTCACTGGGAGCTCTGTTCCA 1448
GGGAGATGCAGAGCTGCCATTGCTCAATAGCCCCAGCTGGTAGCTGCAGACCCAGGCCT 1507
GGCAGACCCACCCAGTGAGCAGATAGGGGATTAGGGACCCACATAACACACAGTCTGGC 1566
CACTTTTCCATAGGGCTGCTGAATATGCTGGGGGTCCAATCCAGACCATAGTCACCTCA 1625
CATTTTTCAGTACCTGAAGATATCAACAGTGAAGGCTATGAAACAGTGAAGATGGGGAC 1684
CTGCCCCTGCCTCTGGACCTCTGTTCCAGAGAGGTACAACCTGTTGCCTCCGACATACA 1743
TGCAGGAGGTGGCTGGAGACCCGGTGGATATCCCTCCCACTGAGGAGAAGCAGCATCAG 1802
GGAATCAGGTGAAGAAACAGTCTGGCCACTTTTTGGTAGAGCAGCTGTGCTTGCTGGGG 1861
GTCTGCTACCACCCCCAGCAAAAAGAATGGCATTTGCAAGAATGGCTAAGGCTGCTAAA 1920
CAGCAACAATGGCAACCTACCATTCCCTTTGGAGCGCCATCCCAGGGATATTCGAAACT 1979
GCTGTCCACTAGAAAACAGTGGTGGAGGTGACTGGAGACCCCAGTGGAGAGTTTCACCT 2038
GGTGAAAAGAAACAGGATTTGGGATCGACATGAATAACCAATCTGACTGCTTCCCCGTA 2097
GAGCTGCTGGACTGTGCTGGGTGGCTGCTCCAGTCCCTAGCTGCCTTGGACTCCCGAGA 2156
ACCCAAAGGCTCCAATAGCTAAGATTGTGAAAGAGCAAAGATGGCAGCCCACCCCCTGC 2215
CACAGGGAGCTCCATGTCAGGGAGGTATGAGGCTGCTACCAGTGTCTGGCTGGATTCCC 2274
AAGTCGAGTGGGTCTTACCCTGAGACAGGCCATGGAAGGTGGGCCTGTCATTGTCACTG 2333
CCCAGCGCCCTGGATGAAACCCCTTTCCTAGGGGTATGTATAGGGGTCTAGCGTCCTGC 2392
TTGGCTGGAGTTATAGCTTCTTTTGTGGGGAGGCCTGGGTATCTAAGGCTCCAGGGTAC 2451
CCATGCATGCGAGAGCGGCTGCTCTGCTGAACCCTACGTAGCCCTGCATGTCAGACTAA 2510
ATGCCCTGGTAGAGTGGGTTCACTAGGAGATCTCCTGACCTGAGGATTGCAAAGATCTG 2569
TGGGAGAAGCGTGGGTCCCCAGGGCTGCTCACTTACTCACCACTTCCCTGGGCAGGAGA 2628

```
GGCTCCCCTGGCTCTGTGTCATCCTGGGGGGCAGTTGTCCTGCCTTACTTTGCTTTAT 2687
TCTCCATGGGTCAAGTTGTTTTCTTGAGTCTCAATGTGTGCACCTGGTTTTTTCAGTTG 2746
AAGGTGCTGTATTTACTTGCCCCTTCCATTTCTCTCCATGAGAGTGGCACACACTAGCA 2805
GGTTCCAGTCGGCCATCTTGCAACCCCTGAAAACTATTTGTTTCCAGCTATAAGCCATT 2864
GAGAGAACCTGGAGTGGCATAAAAAGAATGCCTCGGGGTTCATCCCGACCCCAGCTCAG 2923
CTAGGCCAGCAGCACCCTCGTTTCCCA ATG GTC CTA CTG CTT GTT CTA CTG 2974
GTG GCC CTG CTG CTT TGC CAA TGT GGC CCT GTT GGA TCT CTG GGC 3019
TTT GAC CTG CCT CAG AAC CGT GGC CTA CTT AGC AGG AAC ACC TTG 3064
GCA TTC TGG GCC AAA TGC AGA ATC TCC ACT TTC TTG TGT CTC AAG 3109
GAC AGA AGA GAC TTC AGG TTC CCC CTG GAG ATG TGG ATG GCA GTC 3154
ATT TGC AGA AGG CCC AGG CTG TGT CTG TCC TCC ATG AGA TGC TTC 3199
AGC AGA TCT TCA GCC TCT TCC CCA CAG AGC GCT CCT CTG CTG CCT 3244
GGA ACA TGA CCCTCCTGGACCAGCTCCACACTGGATTTCATCAGCAGCTCGAATAG 3300
CCTGGAGTCTTGCTTAGGGCAGGCAACAGGAGAGGAAGAATCTGTGGGGGTGATTGGGA 3359
CCCTACACTGGCCTTGAGGAGGTACTTCCAGGGAATCCATGGGAATCCAGAGAATCTAC 3418
CTGAAAGAGAAGAAATACAGTGACTGTGCTTAGGAGGTTGTCAGAATGGAATCATGAAA 3477
TCCTTCTCTTCATCAACAGACTTGCAAGGACTGAGAAGTAAGGATGAAGACCTGGGGTC 3536
TGCTTTACTCTTTCTTATTTCTTCCTCTTCCTTACTATGTGTTTATTTCTTCTTTTTC 3595
TAGTTCCTTAACTTGTAAGTAGTTCACTTGGTTTGAGGTCTTTCTTCTTTTTTAATATA 3654
AGCTT                                                       3659
```

**14.** Organisme hôte transformé, qui contient l'information génétique selon les revendications 1 à 10.

**15.** Organisme hôte selon la revendication 14, caractérisé en ce que la séquence génétique est contenue dans un vecteur qui est réplicable dans l'organisme hôte.

**16.** Organisme hôte selon la revendication 14, caractérisé en ce qu'il s'agit d'une lignée cellulaire de mammifère ou de E. coli.

**17.** Organisme hôte selon la revendication 14, caractérisé en ce qu'il s'agit de E. coli portant le N°. DSM 3003 ou de E. coli portant le N°. DSM 3004.

**18.** Organisme hôte selon la revendication 17, caractérisé en ce que vecteur contient en outre les séquences de réplicon et de contrôle nécessaires à l'expression.

**19.** Organisme hôte selon la revendication 18, caractérisé en ce que le vecteur contient les séquences de réplicon et de contrôle du plasmide pER103 portant le N°. DSM 2773 (voir figure 6) qui sont nécessaires à l'expression.

**20.** Organisme hôte selon la revendication 19, transformé avec un plasmide d'expression selon la revendication 21, 22 ou 23.

**21.** Plasmide d'expression pour l'interféron oméga, qui est un dérivé du plasmide pBR322, caractérisé en ce que le plasmide pBR322 contient la séquence d'ADN de formule

```
EcoRI        Sau3A
gaattcacgctGATCGCTAAAACATTGTGCAAAAAGAGGGTTGACTTTGCCTTCGCGA 59
                  ↓ début d'ARNm                          Met
ACCAGTTAACTAGTACACAAGTTCACGGCAACGGTAAGGAGGTTTAAGCTAAAG ATG 116
                                       RBS      HindIII
Cys Asp
TGT GAT C— Gène d'interféron oméga ———→
    Sau3A
```

à la place du fragment plus court EcoRI/BamHI propre du plasmide.

22. Plasmide d'expression pRHW12 selon la revendication 21, caractérisé en ce que le gène de l'interféron oméga présente la séquence d'ADN de formule

```
TGT GAT CTG CCT CAG AAC CAT GGC CTA CTT AGC AGG AAC ACC TTG  28
                        NcoI
GTG CTT CTG CAC CAA ATG AGG AGA ATC TCC CCT TTC TTG TGT CTC  73
AAG GAC AGA AGA GAC TTC AGG TTC CCC CAG GAG ATG GTA AAA GGG 118
AGC CAG TTG CAG AAG GCC CAT GTC ATG TCT GTC CTC CAT GAG ATG 163
CTG CAG CAG ATC TTC AGC CTC TTC CAC ACA GAG CGC TCC TCT GCT 208
GCC TGG AAC ATG ACC CTC CTA GAC CAA CTC CAC ACT GGA CTT CAT 253
CAG CAA CTG CAA CAC CTG GAG ACC TGC TTG CTG CAG GTA GTG GGA 298

GAA GGA GAA TCT GCT GAG GCA ATT AGC AGC CCT GCA CTG ACC TTG 343
AGG AGG TAC TTC CAG GGA ATC CGT GTC TAC CTG AAA GAG AAG AAA 388
TAC AGC GAC TGT GCC TGG GAA GTT GTC AGA ATG GAA ATC ATG AAA 433
TCC TTG TTC TTA TCA ACA AAC ATG CAA GAA AGA CTG AGA AGT AAA 478
GAT AGA GAC CTG GGC TCA TCT TGAAATGATTCTCATTGATTAATTTGCCATA 530
TAACACTTGCACATGTGACTCTGGTCAATTCAAAAGACTCTTATTTCGGCTTTAATCAC 589
AGAATTGACTGAATTAGTTCTGCAAATACTTTGTCGGTATATTAAGCCAGTATATGTTA 648
AAAAGACTTAGGTTCAGGGGCATCAGTCCCTAAGATGTTATTTATTTTTACTCATTTAT 707
TTATTCTTACATTTTATCATATTTATACTATTTATATTCTTATATAACAAATGTTTGCC 766
TTTACATTGTATTAAGATAACAAAACATGTTCAGct                        802
                                AluI
```

23. Plasmide d'expression pRHW11 selon la revendication 21, caractérisé en ce que le gène de l'interféron oméga présente la séquence d'AN de formule

```
TGT GAT CTG CCT CAG AAC CAT GGC CTA CTT AGC AGG AAC ACC TTG  28
                        NcoI
GTG CTT CTG CAC CAA ATG AGG AGA ATC TCC CCT TTC TTG TGT CTC  73
AAG GAC AGA AGA GAC TTC AGG TTC CCC CAG GAG ATG GTA AAA GGG 118
AGC CAG TTG CAG AAG GCC CAT GTC ATG TCT GTC CTC CAT GAG ATG 163
CTG CAG CAG ATC TTC AGC CTC TTC CAC ACA GAG CGC TCC TCT GCT 208
GCC TGG AAC ATG ACC CTC CTA GAC CAA CTC CAC ACT GGA CTT CAT 253
CAG CAA CTG CAA CAC CTG GAG ACC TGC TTG CTG CAG GTA GTG GGA 298
GAA GGA GAA TCT GCT GAG GCA ATT AGC AGC CCT GCA CTG ACC TTG 343
AGG AGG TAC TTC CAG GGA ATC CGT GTC TAC CTG AAA GAG AAG AAA 388
TAC AGC GAC TGT GCC TGG GAA GTT GTC AGA ATG GAA ATC ATG AAA 433
TCC TTG TTC TTA TCA ACA AAC ATG CAA GAA AGA CTG AGA AGT AAA 478
GAT AGA GAC CTG GGC TCA TCT TGAAATGATTCTCATTGATTAATTTGCCATA 530
TAACACTTGCACATGTGACTCTGGTCAATTCAAAAGACTCTTATTTCGGCTTTAATCAC 589
AGAATTGACTGAATTAGTTCTGCAAATACTTTGTCGGTATATTAAGCCAGTATATGTTA 648
AAAAGACTTAGGTTCAGGGGCATCAGTCCCTAAGATGTTATTTATTTTTACTCATTTAT 707
TTATTCTTACATTTTATCATATTTATACTATTTATATTCTTATATAACAAATGTTTGCC 766
TTTACATTGTATTAAGATAACAAAACATGTTCAGct                        802
                                AluI
```

**24.** Plasmide pRHW10, qui est un dérivé du plasmide pBR322, caractérisé en ce que le fragment d'AN de formule

```
HindIII         Sau3A           NcoI
aAGCTTAAAG ATGTGTGATC TGCCTCAGAA CCATGGCCTA CTTAGCAGGA    50

ACACCTTGGT GCTTCTGCAC CAAATGAGGA GAATCTCCCC TTTCTTGTGT   100

CTCAAGGACA GAAGAGACTT CAGGTTCCCC CAGGAGATGG TAAAAGGGAG   150

CCAGTTGCAG AAGGCCCATG TCATGTCTGT CCTCCATGAG ATGCTGCAGC   200

AGATCACACA TCTTTAagct t
 Sau3A          HindIII
```

est inséré dans le plasmide pER103 portant le N°. DSM 2773 (voir la figure 6) dans le site HindIII.

**25.** E. coli HB 101 transformé avec un plasmide selon la revendication 21, 22, 23 ou 24.

**26.** Nouvelles protéines d'interféron humaines de type I, caractérisées en ce qu'elles sont codées par une molécule d'ADN selon l'une des revendications 1 à 10.

**27.** Nouveaux interférons humains selon la revendication 26, caractérisés en ce que les interférons matures présentent une divergence de 30 à 50% par rapport aux interférons $\alpha$ humains.

**28.** Nouveaux interférons humains de type I selon la revendication 26, caractérisés en ce que
a) les interférons matures présentent une divergence de 30 à 50% par rapport aux interférons α humains et une divergence d'environ 70% par rapport à l'interféron β et
b) ont une longueur de 168 à 174 acides aminés, et leurs dérivés N-glycosylés.

**29.** Interférons selon la revendication 28, caractérisés en ce qu'ils présentent une divergence de 40 à 48% par rapport aux interférons α humains, et leurs dérivés N-glycosylés.

**30.** Interférons selon les revendications 26 à 29, caractérisés en ce qu'ils contiennent un peptide de tête.

**31.** Interférons selon les revendications 26 à 29, caractérisés en ce qu'ils ccntiennent 172 acides aminés.

**32.** Interféron selon la revendication 31, caractérisé en ce qu'il contient la séquence d'acides aminés

```
                    5                   10                  15
Cys Asp Leu Pro Gln Asn His Gly Leu Leu Ser Arg Asn Thr Leu

                    20                  25                  30
Val Leu Leu His Gln Met Arg Arg Ile Ser Pro Phe Leu Cys Leu

                    35                  40                  45
Lys Asp Arg Arg Asp Phe Arg Phe Pro Gln Glu Met Val Lys Gly

                    50                  55                  60
Ser Gln Leu Gln Lys Ala His Val Met Ser Val Leu His Glu Met

                    65                  70                  75
Leu Gln Gln Ile Phe Ser Leu Phe His Thr Glu Arg Ser Ser Ala

                    80                  85                  90
Ala Trp Asn Met Thr Leu Leu Asp Gln Leu His Thr Gly Leu His

                    95                  100                 105
Gln Gln Leu Gln His Leu Glu Thr Cys Leu Leu Gln Val Val Gly

                    110                 115                 120
Glu Gly Glu Ser Ala Gly Ala Ile Ser Ser Pro Ala Leu Thr Leu

                    125                 130                 135
Arg Arg Tyr Phe Gln Gly Ile Arg Val Tyr Leu Lys Glu Lys Lys

                    140                 145                 150
Tyr Ser Asp Cys Ala Trp Glu Val Val Arg Met Glu Ile Met Lys

                    155                 160                 165
Ser Leu Phe Leu Ser Thr Asn Met Gln Glu Arg Leu Arg Ser Lys

                    170
Asp Arg Asp Leu Gly Ser Ser
```

et ses dérivés N-glycosylés en position d'acide aminé 78.

**33.** Interféron selon la revendication 31, caractérisé en ce que l'interféron selon la revendication 32 contient en position 111 l'acide aminé Glu à la place de l'acide aminé Gly, et ses dérivés N-glycosylés en position d'acide aminé 78.

**34.** Interférons selon les revendications 30 à 33, caractérisés en ce qu'ils contiennent le peptide de tête de formule
Met Ala Leu Leu Phe Pro Leu Leu Ala Ala Sein val Met Thr Ser Tyr Ser Pro val Gly Ser Leu Gly.

**35.** Procédé de préparation des protéines d'interféron selon les revendications 26 à 34, caractérisé en ce ou'un organisme hôte approprié est transformé avec des informations génétiques codant pour les protéines d'interféron selon les revendications 26 à 34, cette information est exprimée pour produire l'interféron correspondant dans l'organisme hôte et le peptide d'interféron ainsi obtenu est isolé à partir de cet organisme hôte.

**36.** Procédé selon la revendication 35, caractérisé en ce que l'information est contenue dans chaque molécule d'ADN recombiné selon les revendications 1 à 10.

**37.** Procédé selon la revendication 36, caractérisé en ce que l'organisme hôte, après la transformation, est défini selon l'une des revendications 15 à 20.

**38.** Procédé selon la revendication 35, caractérisé en ce que l'interféron est défini selon les revendications 32 à 34.

**39.** Procédé selon la revendication 35, caractérisé en ce que l'on utilise comme hôte E. coli HB 101 et comme plasmide un plasmide selon la revendication 21, 22 ou 23.

**40.** Interféron oméga qui peut être préparé par le procédé selon la revendication 35 ou 39.

**41.** Mélange convenant au traitement antitumoral ou antiviral, contenant outre une ou plusieurs substances inertes formant véhicule et/ou un ou plusieurs diluants une quantité efficace de peptide d'interféron selon l'une des revendications 26 à 34.

**42.** Utilisation de l'interféron oméga selon les revendications 30 à 34 pour la préparation d'un mélange convenant au traitement antitumoral ou antiviral.

**43.** Procédé de préparation du plasmide pRHW10, caractérisé en ce que le fragment d'ADN de formule

```
HindIII             Sau3A               NcoI
aAGCTTAAAG ATGTGTGATC TGCCTCAGAA CCATGGCCTA CTTAGCAGGA     50

ACACCTTGGT GCTTCTGCAC CAAATGAGGA GAATCTCCCC TTTCTTGTGT    100

CTCAAGGACA GAAGAGACTT CAGGTTCCCC CAGGAGATGG TAAAAGGGAG    150

CCAGTTGCAG AAGGCCCATG TCATGTCTGT CCTCCATGAG ATGCTGCAGC    200
AGATCACACA TCTTTAagct t
 Sau3A            HindIII
```

est inséré dans le plasmide pER103 portant le N°. DSM 2773 (figure 6) dans le site HindIII par réaction avec une ligase, et le plasmide ainsi obtenu est transformé dans E. coli HB 101 et cultivé en vue de sa réplication.

**44.** Procédé de préparation du plasmide d'expression pRHW12, caractérisé en ce que le fragment de formule

```
                              c CAT GGC CTA CTT AGC AGG AAC ACC TTG  28
                                NcoI
GTG CTT CTG CAC CAA ATG AGG AGA ATC TCC CCT TTC TTG TGT CTC  73
AAG GAC AGA AGA GAC TTC AGG TTC CCC CAG GAG ATG GTA AAA GGG 118
AGC CAG TTG CAG AAG GCC CAT GTC ATG TCT GTC CTC CAT GAG ATG 163
CTG CAG CAG ATC TTC AGC CTC TTC CAC ACA GAG CGC TCC TCT GCT 208
GCC TGG AAC ATG ACC CTC CTA GAC CAA CTC CAC ACT GGA CTT CAT 253
CAG CAA CTG CAA CAC CTG GAG ACC TGC TTG CTG CAG GTA GTG GGA 298

GAA GGA GAA TCT GCT GGG GCA ATT AGC AGC CCT GCA CTG ACC TTG 343
AGG AGG TAC TTC CAG GGA ATC CGT GTC TAC CTG AAA GAG AAG AAA 388
TAC AGC GAC TGT GCC TGG GAA GTT GTC AGA ATG GAA ATC ATG AAA 433
TCC TTG TTC TTA TCA ACA AAC ATG CAA GAA AGA CTG AGA AGT AAA 478
GAT AGA GAC CTG GGC TCA TCT TGAAATGATTCTCATTGATTAATTTGCCATA 530
TAACACTTGCACATGTGACTCTGGTCAATTCAAAAGACTCTTATTTCGGCTTTAATCAC 589
AGAATTGACTGAATTAGTTCTGCAAATACTTTGTCGGTATATTAAGCCAGTATATGTTA 648
AAAAGACTTAGGTTCAGGGGCATCAGTCCCTAAGATGTTATTTATTTTTACTCATTTAT 707
TTATTCTTACATTTTATCATATTTATACTATTTATATTCTTATATAACAAATGTTTGCC 766
TTTACATTGTATTAAGATAACAAAACATGTTCAGct                        802
                                AluI
```

obtenu par digestion du fragment AvaII du clone P9A2 (N°. DSM 3004) avec NcoI et AluI est inséré par réaction avec une ligase dans le plus grand fragment du plasmide pRHW10 obtenu par digestion par endonucléase de restriction avec BamHI, remplissage des sites de coupure à l'aide du fragment de Klenow de l'ADN polymérase I et des 4 désoxynucléosidetriphosphates puis coupure avec NcoI, qui contient le fragment d'ADN de formule

```
HindIII          Sau3A            NcoI
aAGCTTAAAG ATGTGTGATC TGCCTCAGAA CCATGGCCTA CTTAGCAGGA     50

ACACCTTGGT GCTTCTGCAC CAAATGAGGA GAATCTCCCC TTTCTTGTGT    100

CTCAAGGACA GAAGAGACTT CAGGTTCCCC CAGGAGATGG TAAAAGGGAG    150

CCAGTTGCAG AAGGCCCATG TCATGTCTGT CCTCCATGAG ATGCTGCAGC    200

AGATCACACA TCTTTAagct t
 Sau3A         HindIII
```

dans le site HindIII du plasmide pER103 portant le N°. DSM 2773 (voir figure 6), et en ce que le plasmide ainsi obtenu est transformé dans E. coli HB 101 et cultivé en vue de sa réplication.

**45.** Procédé de préparation du plasmide d'expression pHWR11, caractérisé en ce que le fragment de

formule

```
                              c CAT GGC CTA CTT AGC AGG AAC ACC TTG  28
                                NcoI
GTG CTT CTG CAC CAA ATG AGG AGA ATC TCC CCT TTC TTG TGT CTC  73
AAG GAC AGA AGA GAC TTC AGG TTC CCC CAG GAG ATG GTA AAA GGG 118
AGC CAG TTG CAG AAG GCC CAT GTC ATG TCT GTC CTC CAT GAG ATG 163
CTG CAG CAG ATC TTC AGC CTC TTC CAC ACA GAG CGC TCC TCT GCT 208
GCC TGG AAC ATG ACC CTC CTA GAC CAA CTC CAC ACT GGA CTT CAT 253
CAG CAA CTG CAA CAC CTG GAG ACC TGC TTG CTG CAG GTA GTG GGA 298
GAA GGA GAA TCT GCT GAG GCA ATT AGC AGC CCT GCA CTG ACC TTG 343
AGG AGG TAC TTC CAG GGA ATC CGT GTC TAC CTG AAA GAG AAG AAA 388
TAC AGC GAC TGT GCC TGG GAA GTT GTC AGA ATG GAA ATC ATG AAA 433
TCC TTG TTC TTA TCA ACA AAC ATG CAA GAA AGA CTG AGA AGT AAA 478
GAT AGA GAC CTG GGC TCA TCT TGAAATGATTCTCATTGATTAATTTGCCATA 530
TAACACTTGCACATGTGACTCTGGTCAATTCAAAAGACTCTTATTTCGGCTTTAATCAC 589
AGAATTGACTGAATTAGTTCTGCAAATACTTTGTCGGTATATTAAGCCAGTATATGTTA 648
AAAAGACTTAGGTTCAGGGGCATCAGTCCCTAAGATGTTATTTATTTTTACTCATTTAT 707
TTATTCTTACATTTTATCATATTTATACTATTTATATTCTTATATAACAAATGTTTGCC 766
TTTACATTGTATTAAGATAACAAAACATGTTCAGct                        802
                                                AluI
```

obtenu par digestion d'un fragment AvaII du clone E76E9 (N°. DSM 3003) avec NcoI et AluI est inséré par réaction avec une ligase dans le plus grand fragment du plasmide pRHW10, obtenu par digestion par endonucléase de restriction avec BamHI, remplissage des sites de coupure à l'aide du fragment de Klenow de l'ADN polymérase I et des 4 désoxynucléosidephosphates puis coupure avec NcoI, qui contient dans le site HindIII du plasmide pER103 portant le N°. DSM 2773 (voir figure 6) le fragment d'ADN de formule

```
HindIII            Sau3A           NcoI
aAGCTTAAAG ATGTGTGATC TGCCTCAGAA CCATGGCCTA CTTAGCAGGA     50

ACACCTTGGT GCTTCTGCAC CAAATGAGGA GAATCTCCCC TTTCTTGTGT    100

CTCAAGGACA GAAGAGACTT CAGGTTCCCC CAGGAGATGG TAAAAGGGAG    150

CCAGTTGCAG AAGGCCCATG TCATGTCTGT CCTCCATGAG ATGCTGCAGC    200

AGATCACACA TCTTTAagct t
 Sau3A          HindIII
```

et en ce que le plasmide ainsi obtenu est transformé dans E. coli et cultivé en vue de sa réplication.

**46.** Mélange synergique selon la revendication 41, caractérisé en ce qu'il contient en outre l'interféron $\gamma$.

**47.** Mélange synergique selon la revendication 41, caractérisé en ce qu'il contient en outre le facteur de nécrose de tumeur humain.

Abbildung 1

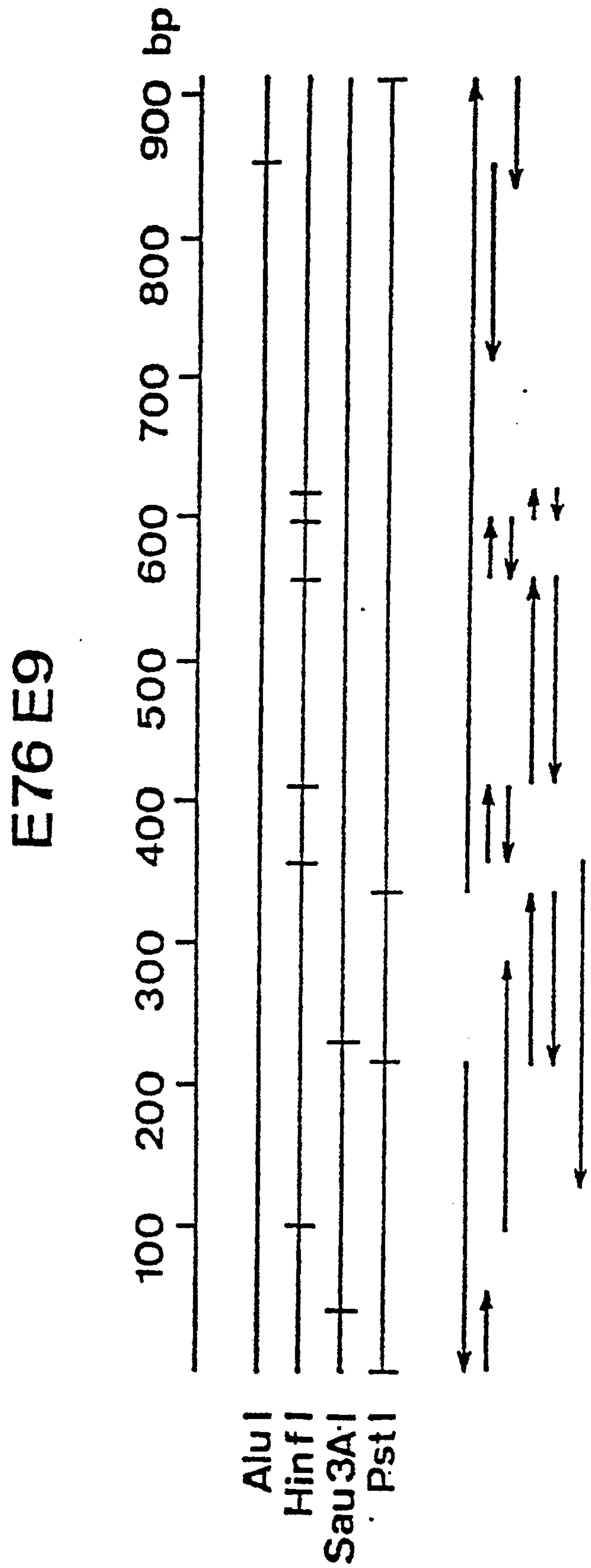
E76 E9
bp
100
200
300
400
500
600
700
800
900
Alu I
Hinf I
Sau3A I
Pst I

Abbildung 2

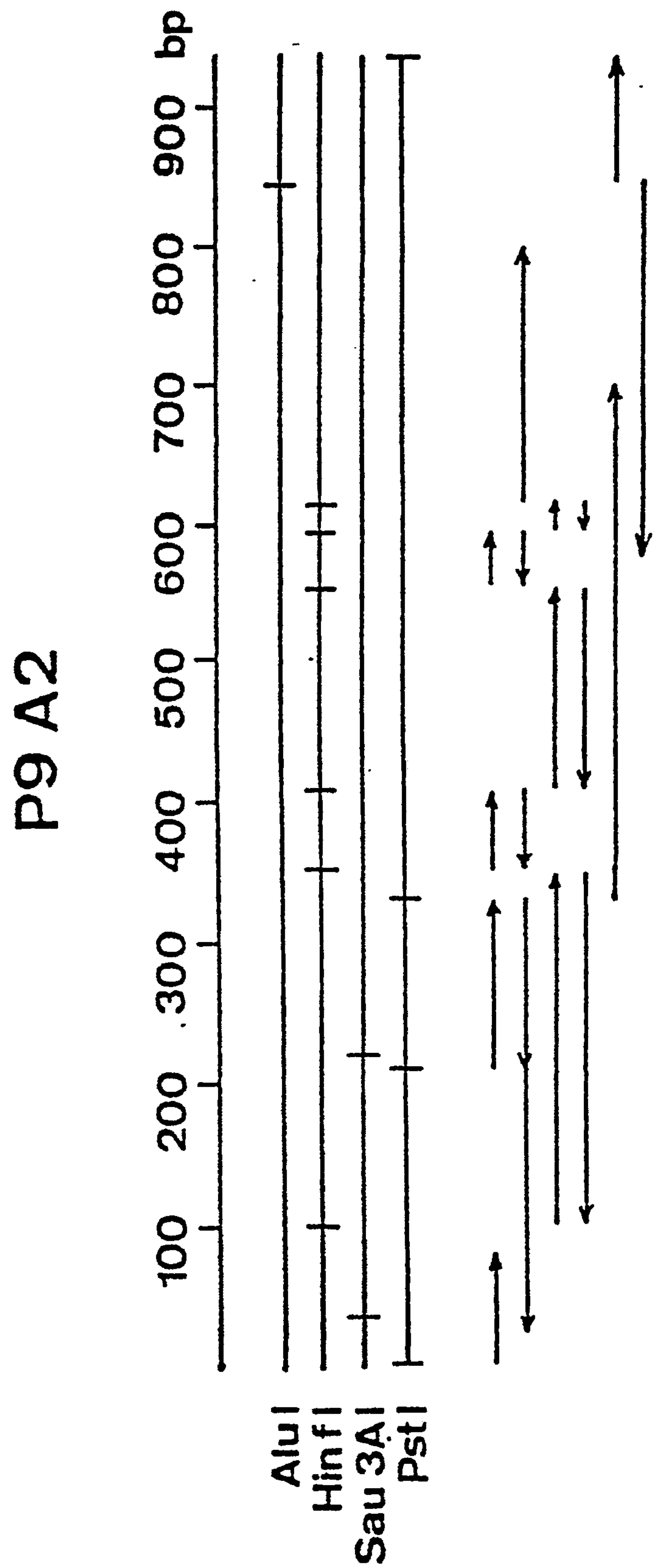
P9 A2
100
200
300
400
500
600
700
800
900
bp
Alu I
Hin f I
Sau 3A I
Pst I

Abbildung 3 (P9A2):

TCTGGGC 7

5 10 15
Cys Asp Leu Pro Gln Asn His Gly Leu Leu Ser Arg Asn Thr Leu
TGT GAT CTG CCT CAG AAC CAT GGC CTA CTT AGC AGG AAC ACC TTG 52

20 25 30
Val Leu Leu His Gln Met Arg Arg Ile Ser Pro Phe Leu Cys Leu
GTG CTT CTG CAC CAA ATG AGG AGA ATC TCC CCT TTC TTG TGT CTC 97

35 40 45
Lys Asp Arg Arg Asp Phe Arg Phe Pro Gln Glu Met Val Lys Gly
AAG GAC AGA AGA GAC TTC AGG TTC CCC CAG GAG ATG GTA AAA GGG 142

50 55 60
Ser Gln Leu Gln Lys Ala His Val Met Ser Val Leu His Glu Met
AGC CAG TTG CAG AAG GCC CAT GTC ATG TCT GTC CTC CAT GAG ATG 187

65 70 75
Leu Gln Gln Ile Phe Ser Leu Phe His Thr Glu Arg Ser Ser Ala
CTG CAG CAG ATC TTC AGC CTC TTC CAC ACA GAG CGC TCC TCT GCT 232

80 85 90
Ala Trp Asn Met Thr Leu Leu Asp Gln Leu His Thr Gly Leu His
GCC TGG AAC ATG ACC CTC CTA GAC CAA CTC CAC ACT GGA CTT CAT 277

95 100 105
Gln Gln Leu Gln His Leu Glu Thr Cys Leu Leu Gln Val Val Gly
CAG CAA CTG CAA CAC CTG GAG ACC TGC TTG CTG CAG GTA GTG GGA 322

110 115 120
Glu Gly Glu Ser Ala Gly Ala Ile Ser Ser Pro Ala Leu Thr Leu
GAA GGA GAA TCT GCT GGG GCA ATT AGC AGC CCT GCA CTG ACC TTG 367

125 130 135
Arg Arg Tyr Phe Gln Gly Ile Arg Val Tyr Leu Lys Glu Lys Lys
AGG AGG TAC TTC CAG GGA ATC CGT GTC TAC CTG AAA GAG AAG AAA 412

140 145 150
Tyr Ser Asp Cys Ala Trp Glu Val Val Arg Met Glu Ile Met Lys
TAC AGC GAC TGT GCC TGG GAA GTT GTC AGA ATG GAA ATC ATG AAA 457

Abbildung 3 ff

```
                        155                     160                     165
Ser Leu Phe Leu Ser Thr Asn Met Gln Glu Arg Leu Arg Ser Lys
TCC TTG TTC TTA TCA ACA AAC ATG CAA GAA AGA CTG AGA AGT AAA 502

                        170
Asp Arg Asp Leu Gly Ser Ser
GAT AGA GAC CTG GGC TCA TCT TGAAATGATTCTCATTGATTAATTTGCCATA 554

TAACACTTGCACATGTGACTCTGGTCAATTCAAAAGACTCTTATTTCGGCTTTAATCAC 613

AGAATTGACTGAATTAGTTCTGCAAATACTTTGTCGGTATATTAAGCCAGTATATGTTA 672

AAAAGACTTAGGTTCAGGGGCATCAGTCCCTAAGATGTTATTTATTTTTACTCATTTAT 731

TTATTCTTACATTTTATCATATTTATACTATTTATATTCTTATATAACAAATGTTTGCC 790

TTTACATTGTATTAAGATAACAAAACATGTTCAGCTTTCCATTTGGTTAAATATTGTAT 849

TTTGTTATTTATTAAATTATTTTCAAAC-poly-A                         877
```

Abbildung 4 (E76E9):

```
                                                          CTCTGGGC    8

                    5                  10                  15
Cys Asp Leu Pro Gln Asn His Gly Leu Leu Ser Arg Asn Thr Leu
TGT GAT CTG CCT CAG AAC CAT GGC CTA CTT AGC AGG AAC ACC TTG   53

                   20                  25                  30
Val Leu Leu His Gln Met Arg Arg Ile Ser Pro Phe Leu Cys Leu
GTG CTT CTG CAC CAA ATG AGG AGA ATC TCC CCT TTC TTG TGT CTC   98

                   35                  40                  45
Lys Asp Arg Arg Asp Phe Arg Phe Pro Gln Glu Met Val Lys Gly
AAG GAC AGA AGA GAC TTC AGG TTC CCC CAG GAG ATG GTA AAA GGG 143

                   50                  55                  60
Ser Gln Leu Gln Lys Ala His Val Met Ser Val Leu His Glu Met
AGC CAG TTG CAG AAG GCC CAT GTC ATG TCT GTC CTC CAT GAG ATG 118

                   65                  70                  75
Leu Gln Gln Ile Phe Ser Leu Phe His Thr Glu Arg Ser Ser Ala
CTG CAG CAG ATC TTC AGC CTC TTC CAC ACA GAG CGC TCC TCT GCT 233

                   80                  85                  90
Ala Trp Asn Met Thr Leu Leu Asp Gln Leu His Thr Gly Leu His
GCC TGG AAC ATG ACC CTC CTA GAC CAA CTC CAC ACT GGA CTT CAT 278

                   95                 100                 105
Gln Gln Leu Gln His Leu Glu Thr Cys Leu Leu Gln Val Val Gly
CAG CAA CTG CAA CAC CTG GAG ACC TGC TTG CTG CAG GTA GTG GGA 323

                  110                 115                 120
Glu Gly Glu Ser Ala Glu Ala Ile Ser Ser Pro Ala Leu Thr Leu
GAA GGA GAA TCT GCT GAG GCA ATT AGC AGC CCT GCA CTG ACC TTG 368

                  125                 130                 135
Arg Arg Tyr Phe Gln Gly Ile Arg Val Tyr Leu Lys Glu Lys Lys
AGG AGG TAC TTC CAG GGA ATC CGT GTC TAC CTG AAA GAG AAG AAA 413

                  140                 145                 150
Tyr Ser Asp Cys Ala Trp Glu Val Val Arg Met Glu Ile Met Lys
TAC AGC GAC TGT GCC TGG GAA GTT GTC AGA ATG GAA ATC ATG AAA 458
```

Abbildung 4 ff

```
                    155                                    160                                    165
Ser Leu Phe Leu Ser Thr Asn Met Gln Glu Arg Leu Arg Ser Lys
TCC TTG TTC TTA TCA ACA AAC ATG CAA GAA AGA CTG AGA AGT AAA 503

                    170
Asp Arg Asp Leu Gly Ser Ser
GAT AGA GAC CTG GGC TCA TCT TGAAATGATTCTCATTGATTAATTTGCCATA 555

TAACACTTGCACATGTGACTCTGGTCAATTCAAAAGACTCTTATTTCGGCTTTAATCAC 614

AGAATTGACTGAATTAGTTCTGCAAATACTTTGTCGGTATATTAAGCCAGTATATGTTA 673

AAAAGACTTAGGTTCAGGGGCATCAGTCCCTAAGATGTTATTTATTTTTACTCATTTAT 732

TTATTCTTACATTTTATCATATTTATACTATTTATATTCTTATATAACAAATGTTTGCC 791

TTTACATTGTATTAAGATAACAAAACATGTTCAGCTTTCCATTTGGTTAAATATTGTAT 650

TTTGTTAT                                                    858
```

Abbildung 5

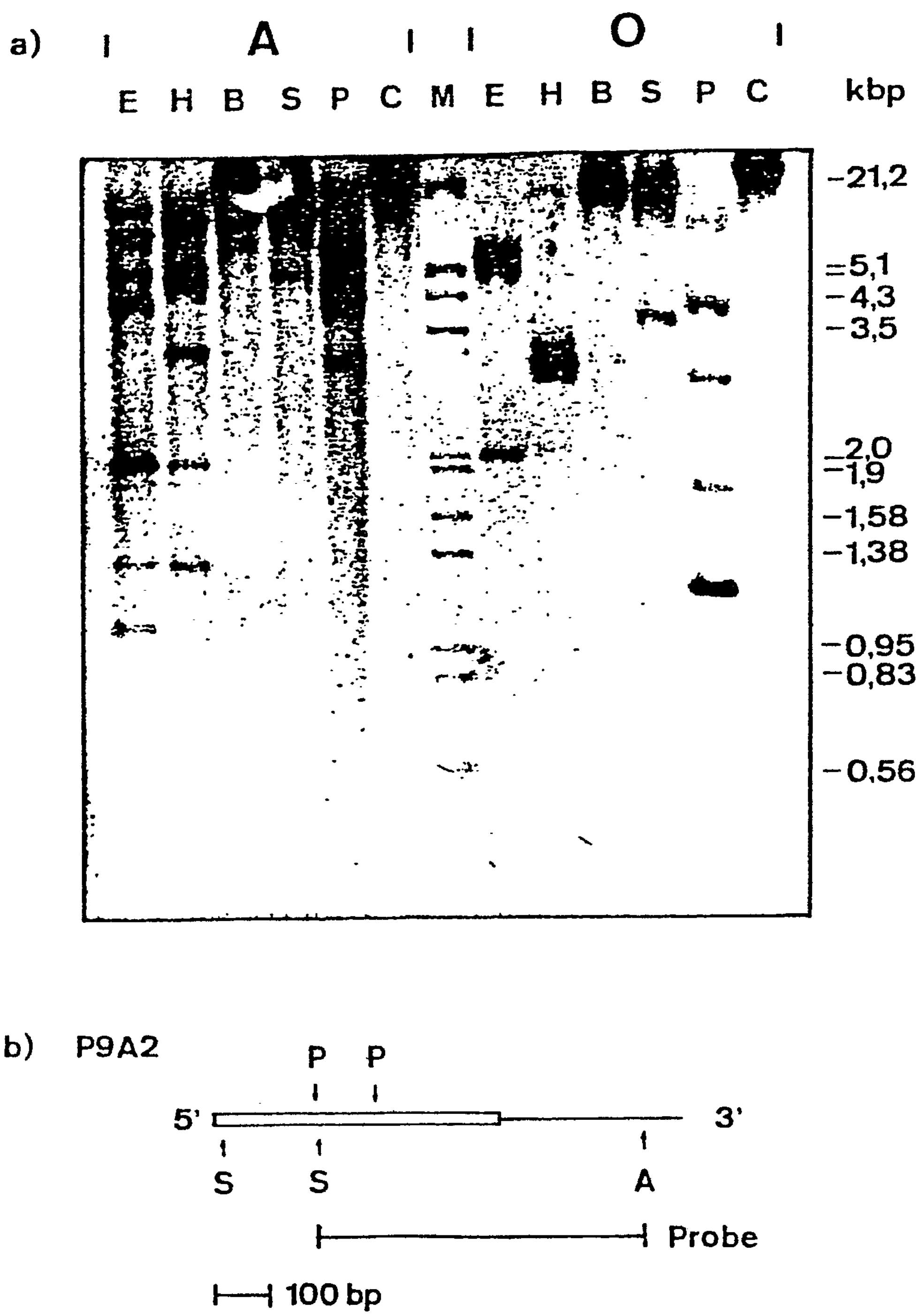
a)
A
O
E H B S P C M E H B S P C
kbp
-21,2
=5,1
-4,3
-3,5
=2,0
-1,9
-1,58
-1,38
-0,95
-0,83
-0,56
b) P9A2
P P
5'
3'
S S A
Probe
100 bp

Abbildung 6

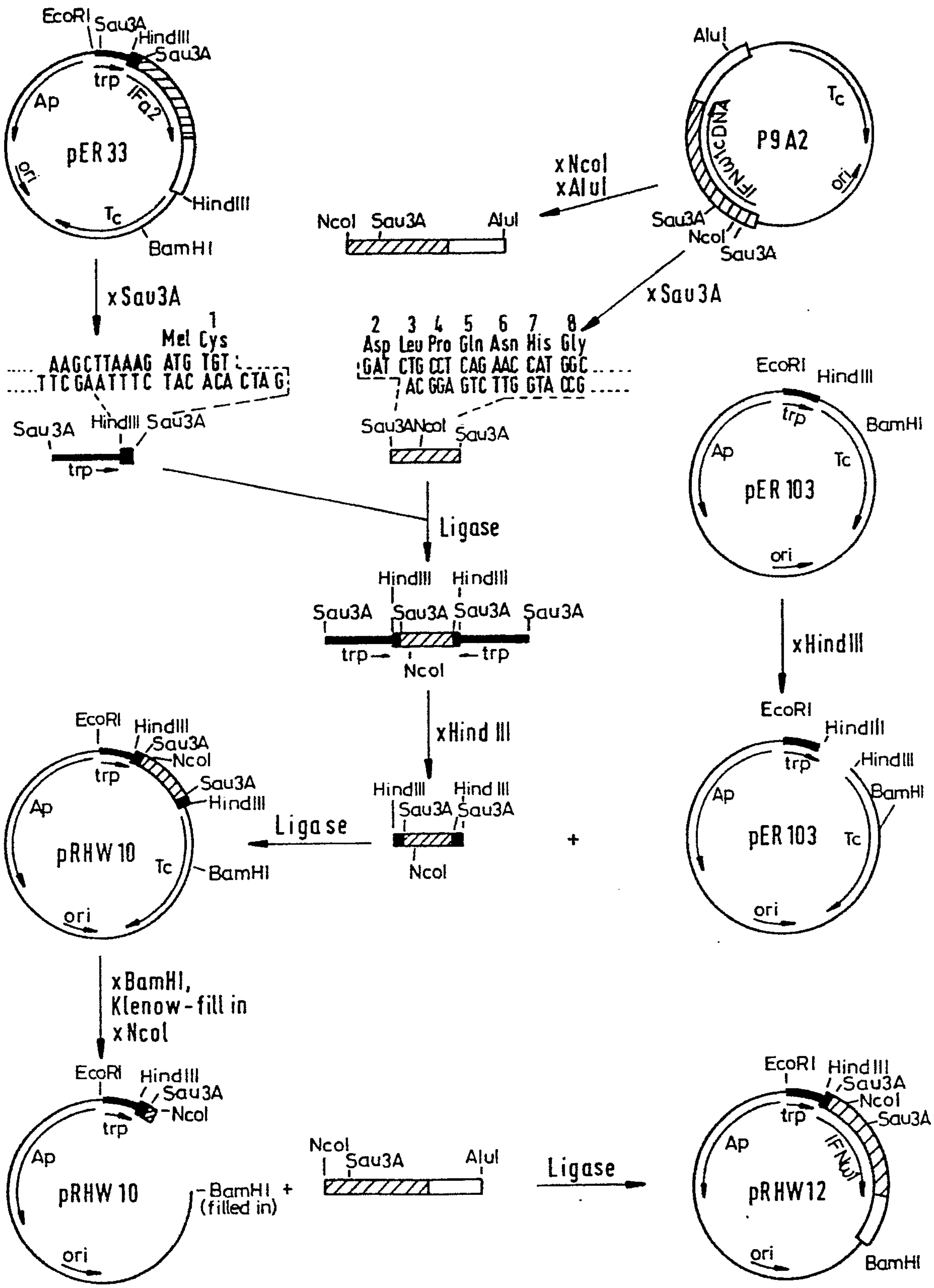
EcoRI
Sau3A
HindIII
Sau3A
trp
IFα2
Ap
pER 33
ori
Tc
HindIII
BamHI
AluI
Tc
IFNωlcDNA
P9A2
ori
Sau3A
NcoI
Sau3A
xNcoI
xAluI
NcoI Sau3A AluI
xSau3A
xSau3A
1
Met Cys
AAGCTTAAAG ATG TGT
TTCGAATTTC TAC ACA CTA G
2 3 4 5 6 7 8
Asp Leu Pro Gln Asn His Gly
GAT CTG CCT CAG AAC CAT GGC
AC GGA GTC TTG GTA CCG
Sau3A HindIII Sau3A
trp
Sau3A NcoI Sau3A
EcoRI HindIII
BamHI
pER 103
Ligase
HindIII HindIII
Sau3A Sau3A Sau3A Sau3A
trp trp
NcoI
xHindIII
xHind III
EcoRI
HindIII
HindIII
BamHI
pER 103
HindIII Hind III
Sau3A Sau3A
NcoI
Ligase
+
EcoRI HindIII
Sau3A
NcoI
Sau3A
HindIII
pRHW 10
BamHI
xBamHI,
Klenow-fill in
xNcoI
pRHW 10
-BamHI
(filled in)
+
NcoI
Sau3A
AluI
Ligase
pRHW12
EcoRI HindIII
Sau3A
NcoI
Sau3A
IFNω1
BamHI

## Abbildung 7

Amino acid differences

| | IFN-αA | IFN-αB | IFN-αC | IFN-αD | IFN-αF | IFN-αG | IFN-αH | IFN-αK | IFN-αL | IFN-β | IFN-ω |
|---|---|---|---|---|---|---|---|---|---|---|---|
| IFN-αA | | 31 (18,7) | 31 (18,7) | 29 (17,5) | 30 (18,1) | 25 (15,1) | 29 (17,5) | 33 (19,9) | 34 (20,5) | 109+4 (68,1) | 63 +6 (41,6) |
| IFN-αB | 68 (13,1) | | 32 (19,3) | 38 (22,9) | 31 (18,7) | 29 (17,5) | 30 (18,1) | 33 (19,9) | 35 (21,1) | 116+4 (72,3) | 69 +6 (45,2) |
| IFN-αC | 59 (11,4) | 65 (12,5) | | 32 (19,3) | 18 (10,8) | 27 (16,3) | 24 (14,5) | 11 ( 6,6) | 3 ( 1,8) | 113+4 (70,5) | 67 +6 (44,0) |
| IFN-αD | 60 (11,6) | 72 (13,9) | 63 (12,1) | | 28 (16,9) | 24 (14,5) | 32 (19,3) | 35 (21,1) | 34 (20,5) | 115+4 (71,7) | 70 +6 (45,8) |
| IFN-αF | 64 (12,3) | 66 (12,7) | 42 ( 8,1) | 59 (11,4) | | 20 (12,0) | 27 (16,3) | 20 (12,0) | 21 (12,7) | 115+4 (71,7) | 72 +6 (47,0) |
| IFN-αG | 55 (10,6) | 65 (12,5) | 52 (10,0) | 56 (10,8) | 53 (10,2) | | 24 (14,5) | 28 (16,9) | 29 (17,5) | 111+4 (69,3) | 68 +6 (44,6) |
| IFN-αH | 53 (10,2) | 66 (12,7) | 42 ( 8,1) | 58 (11,2) | 57 (11,0) | 46 ( 8,9) | | 24 (14,5) | 26 (15,7) | 110+4 (68,7) | 71 +6 (46,4) |
| IFN-αK | 59 (11,4) | 67 (12,9) | 18 ( 3,5) | 67 (12,9) | 43 ( 8,3) | 52 (10,0) | 41 ( 7,9) | | 12 ( 7,2) | 112+4 (69,9) | 69 +6 (45,2) |
| IFN-αL | 61 (11,8) | 67 (12,9) | 4 ( 0,8) | 65 (12,5) | 44 ( 8,5) | 53 (10,2) | 43 ( 8,3) | 18 ( 3,5) | | 113+4 (70,5) | 68 +6 (44,6) |
| IFN-β | 274 (52,8) | 287 (55,3) | 290 (55,9) | 292 (56,3) | 284 (54,7) | 281 (54,1) | 282 (54,3) | 288 (55,5) | 290 (55,9) | | 112+10 (73,5) |
| IFN-ω | 148 (28,5) | 156 (30,1) | 152 (29,3) | 154 (29,7) | 160 (30,8) | 147 (28,3) | 155 (29,9) | 154 (29,7) | 153 (29,5) | 270 (52,0) | |

Nucleotide differences

Abbildung 8a

| | 130 140 150 |
|---|---|
| IFN αA | gaggagtttgGCaaccagttccaAaaggct |
| IFN αB | gaggagtttgatgataaacagttccagaag |
| IFN αC | gaggagtttgatggcaaccagttccagaag |
| IFN αD | gaggagtttgatggcaaccagttccagaag |
| IFN αF | gaggagtttgatggcaaccagttccagaag |
| IFN αG | gaggagtttgatggcaaccagttccagaag |
| IFN αH | gaggaatttgatggcaaccagttccagaaa |
| IFN αK | gaggagtttgatggccaccagttccagaag |
| IFN αL | gaggagtttgatggcaaccagttccagaag |
| IFN omegal | gagatggtaaaagggagccagttgcagaag |
| IFN beta | gagattaagcagctgcagcagttccagaag |

Abbildung 8b

| | 340 350 360 |
|---|---|
| IFN omegal | GgggCaaTtAGcaGcCctGCActgaccTtg |
| IFN αA | ctgatgaaggaggactccattctggctgtg |
| IFN αB | ctgatgtacgaggactccatcctggctgtg |
| IFN αC | ctgatgaatgaggactccatcctggctgtg |
| IFN αD | ctgatgaatgtggactccatcttggctgtg |
| IFN αF | ctgatgaatgtggactccatcttggctgtg |
| IFN αG | ctgatgaatgtggactctatcctgactgtg |
| IFN αH | ctgatgaatgaggactccatcctggctgtg |
| IFN αK | ctgatgaatgaggacttcatcctggctgtg |
| IFN αL | ctgatgaatgaggactccatcctggctgtg |
| IFN beta | aggggaaaactcatgagcagtctgcacctg |

Abbildung 8c

| | |
|---|---|
| | 200 210 220 |
| IFN αD | tcttcaacctcttTaCcacaaaAgaTtcat |
| IFN αA | tcttcaatctcttcagcacaaaggactcat |
| IFN αB | ccttcaacctcttcagcacaaaggactcat |
| IFN αC | ccttcaatctcttcagcacagaggactcat |
| IFN αF | ccttcaatctcttcagcacaaaggactcat |
| IFN αF | ccttcaatctcttcagcacaaaggactcat |
| IFN αH | ccttcaatctcttcagcacaaagaactcat |
| IFN αK | ccttcaatctcttcagcacagaggactcat |
| IFN αL | ccttcaatctcttcagcacagaggactcat |
| IFN omegal | tcttcagcctcttccacacagagcgctcct |
| IFN beta | tctttgctattttcagacaagattcatcta |

Abbildung 9

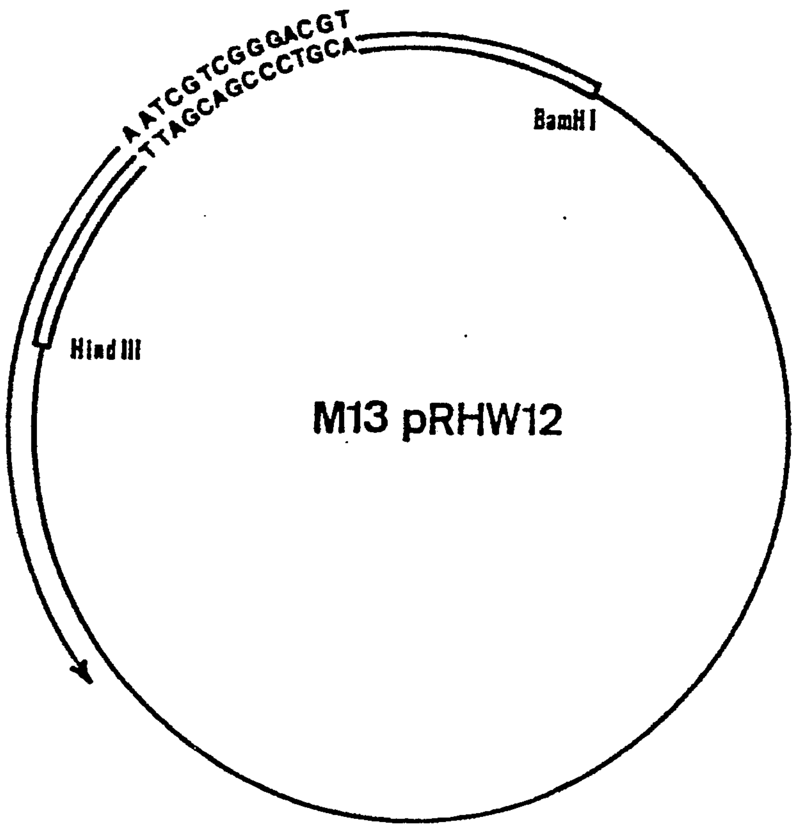


| | |
|---|---|
| ▭ | Hind III / BamH I fragment of pRHW12 |
| —— | M13mp9 |
| ——→ | Primer extension by Klenow Polymerase |

Abbildung 10

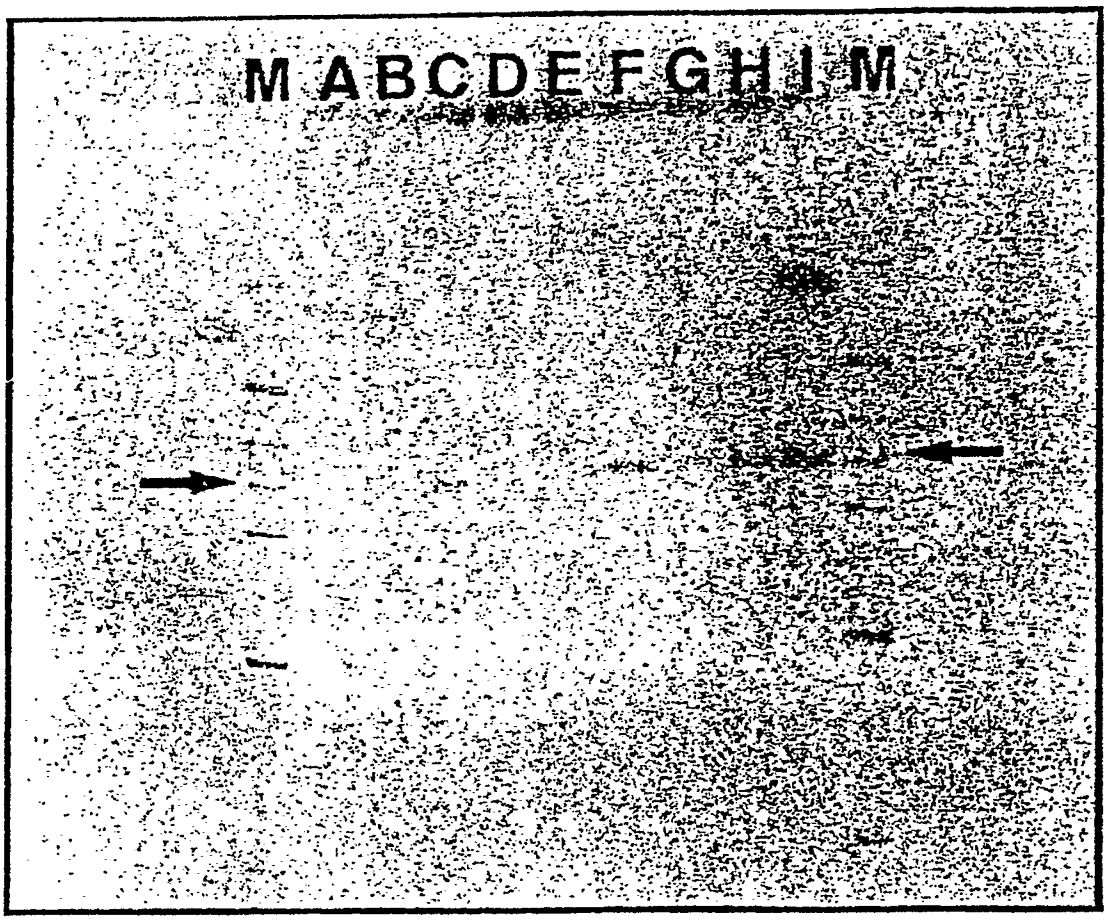

Abbildung 11 (IFN-omega1)

GATCTGGTAAACCTGAA 17

GCAAATATAGAAACCTATAGGGCCTGACTTCCTACATAAAGTAAGGAGGGTAAAAATGG 76

AGGCTAGAATAAGGGTTAAAATTTTGCTTCTAGAACAGAGAAAATGATTTTTTTCATAT 135

ATATATGAATATATATTATATATACACATATATACATATATTCACTATAGTGTGTATAC 194

ATAAATATATAATATATATATTGTTAGTGTAGTGTGTGTCTGATTATTTACATGCATAT 253

AGTATATACACTTATGACTTTAGTACCCAGACGTTTTTCATTTGATTAAGCATTCATTT 312

GTATTGACACAGCTGAAGTTTACTGGAGTTTAGCTGAAGTCTAATGCAAAATTAATAGA 371

TTGTTGTCATCCTCTTAAGGTCATAGGGAGAACACACAAATGAAAACAGTAAAAGAAAC 430

TGAAAGTACAGAGAAATGTTCAGAAAATGAAAACCATGTGTTTCCTATTAAAAGCCATG 489

CATACAAGCAATGTCTTCAGAAAACCTAGGGTCCAAGGTTAAGCCATATCCCAGCTCAG 548

-20 -16

Met Ala Leu Leu Phe Pro Leu Leu

TAAAGCCAGGAGCATCCTCATTTCCCA ATG GCC CTC CTG TTC CCT CTA CTG 599

-10 -5 -1

Ala Ala Leu Val Met Thr Ser Tyr Ser Pro Val Gly Ser Leu Gly

GCA GCC CTA GTG ATG ACC AGC TAT AGC CCT GTT GGA TCT CTG GGC 644

5 10 15

Cys Asp Leu Pro Gln Asn His Gly Leu Leu Ser Arg Asn Thr Leu

TGT GAT CTG CCT CAG AAC CAT GGC CTA CTT AGC AGG AAC ACC TTG 689

20 25 30

Val Leu Leu His Gln Met Arg Arg Ile Ser Pro Phe Leu Cys Leu

GTG CTT CTG CAC CAA ATG AGG AGA ATC TCC CCT TTC TTG TGT CTC 734

35 40 45

Lys Asp Arg Arg Asp Phe Arg Phe Pro Gln Glu Met Val Lys Gly

AAG GAC AGA AGA GAC TTC AGG TTC CCC CAG GAG ATG GTA AAA GGG 779

50 55 60

Ser Gln Leu Gln Lys Ala His Val Met Ser Val Leu His Glu Met

AGC CAG TTG CAG AAG GCC CAT GTC ATG TCT GTC CTC CAT GAG ATG 824

65 70 75

Leu Gln Gln Ile Phe Ser Leu Phe His Thr Glu Arg Ser Ser Ala

CTG CAG CAG ATC TTC AGC CTC TTC CAC ACA GAG CGC TCC TCT GCT 869

80 85 90

Ala Trp Asn Met Thr Leu Leu Asp Gln Leu His Thr Gly Leu His

GCC TGG AAC ATG ACC CTC CTA GAC CAA CTC CAC ACT GGA CTT CAT 914

Abbildung 11 ff

```
                    95                      100                     105
Gln Gln Leu Gln His Leu Glu Thr Cys Leu Leu Gln Val Val Gly
CAG CAA CTG CAA CAC CTG GAG ACC TGC TTG CTG CAG GTA GTG GGA   959

                    110                     115                     120
Glu Gly Glu Ser Ala Gly Ala Ile Ser Ser Pro Ala Leu Thr Leu
GAA GGA GAA TCT GCT GGG GCA ATT AGC AGC CCT GCA CTG ACC TTG 1004

                    125                     130                     135
Arg Arg Tyr Phe Gln Gly Ile Arg Val Tyr Leu Lys Glu Lys Lys
AGG AGG TAC TTC CAG GGA ATC CGT GTC TAC CTG AAA GAG AAG AAA  1049

                    140                     145                     150
Tyr Ser Asp Cys Ala Trp Glu Val Val Arg Met Glu Ile Met Lys
TAC AGC GAC TGT GCC TGG GAA GTT GTC AGA ATG GAA ATC ATG AAA 1094

                    155                     160                     165
Ser Leu Phe Leu Ser Thr Asn Met Gln Glu Arg Leu Arg Ser Lys
TCC TTG TTC TTA TCA ACA AAC ATG CAA GAA AGA CTG AGA AGT AAA 1139

                    170
Asp Arg Asp Leu Gly Ser Ser
GAT AGA GAC CTG GGC TCA TCT TGA AATGATTCTCATTGATTAATTTGCCAT 1190
ATAACACTTGCACATGTGACTCTGGTCAATTCAAAAGACTCTTATTTCGGCTTTAATCA 1249
CAGAATTGACTGAATTAGTTCTGCAAATACTTTGTCGGTATATTAAGCCAGTATATGTT 1308
AAAAAGACTTAGGTTCAGGGGCATCAGTCCCTAAGATGTTATTTATTTTTACTCATTTA 1367
TTTATTCTTACATTTTATCATATTTATACTATTTATATTCTTATATAACAAATGTTTGC 1426
CTTTACATTGTATTAAGATAACAAAACATGTTCAGCTTTCCATTTGGTTAAATATTGTA 1485
TTTTGTTATTTATTAAATTATTTTCAAACAAAACTTCTTGAAGTTATTTATTCGAAAAC 1544
CAAAATCCAAACACTAGTTTTCTGAACCAAATCAAGGAATGGACGGTAATATACACTTA 1603
CCTATTCATTCATTCCATTTACATAATATGTATAAAGTGAGTATCAAAGTGGCATATTT 1662
TGGAATTGATGTCAAGCAATGCAGGTGTACTCATTGCATGACTGTATCAAAATATCTCA 1721
TGTAACCAATAAATATATACACTTACTATGTATCCCACAAAAATTAAAAAGTTATTTA 1780
AAAAAGAAATACAGGTGAATAAACACAGTTTCTTTCCGTGTTGAAGAGCTTTCATTCTT 1839
ACAGGAAAAGAAACAGTAAAGATGTACCAATTTCGCTTATATGAAACACTACAAAGATA 1898
AGTAAAAGAAAATGATGTTCTCATACTAGAAGCTT                          1933
```

Abbildung 12 (IFN-pseudo-omega2)

AAGCTTGAGCCCCCAGGGAAGCATAACCACATGAACCTGAATGAATATATT 51
CTAGAAGGAGGGAAGCACCAGAGAAGTTCTTTCACTAATAACCATCAACGTCTTCTGTG 110
AATCAAATATCAAACAAAGATAGTCCTAAAAAGTTTAATTTCCAGAGATAGGTAATTTC 169
CTAACTGAATACAGAAACCCATAGGGCCCAGGGATCCTGATTTCCTATGCAAAATGGAG 228
GGTAAAACTGGAGGCTAGGATCTGGGCTAAAAGTATATACTTCTAACAGTAGCACAAAG 287
ATGTTTCTCATCTGATTGATCAATATTCATTTGGATTGATATATCTTAAGTTTACTGGG 346
AATATTGAACATCCATTGCAAAAATCAAGAGTGTAGAGTGATGACCTCCTTTTAGGTCA 405
TATAGAACAAGGTTTTTCAACCCCCATCCATGGACCGGGGTACTGGTCCTGGCCTGGTA 464
GGAACAGGGCCGCACAGCAGGAGGCAAGCAGGCCAACCAACAAGCATTAACGCCTGAGC 523
TCTGCCTCCTGTCAGATCAGCAGTGGCATTGGATTCTCAAAAGAGCAGGAACCCTATTG 582
TGAAGTGCAGATGCGAAGGATCTAGGTTGTGGTCTCCTAATGAGAATCTAATGCCTCTG 641
AAAGCATTCCCTCCCTGACCCCATTTTTCGTGGAAAAATTATCTTCCACCAAACTGGTG 700
GCCAAAAGGTTGTGGATGCTGATATAGAAGACATGTAAATGAAAACAATAAATGGAATT 759
AAAAATTTAGAGAAATGCTCAGAAAAATGAAAACTATTTGTGCTCCATTAAAGCCATGC 818
ATAGATAGAATGTCTTCATAGAACCTAGGATCCAAGGTTCTATGAAGACCTCAGCTCAA 877

-20 -16
Met Ala Leu Leu Phe Pro Leu Leu
CCAGGCCAAAAGCATCCTGATTTCTCA ATG GCC CTC CTC TTC CCT CTA CTG 928

-10 -5 -1
Ala Ala Leu Glu Val Cys Ser Cys Gly Ser Ser Gly Ser Leu Gly
GCA GCC CTA GAG GTG TGC AGC TGT GGC TCT TCT GGA TCT CTA GGA 973

5 10 15
Tyr Asn Leu Pro Gln Asn His Gly Leu Leu Gly Arg Asn Thr Leu
TAT AAC CTG CCT CAG AAC CAT GGC CTG CTA GGC AGG AAC ACC TTG 1018

20 25 30
Val Leu Leu Gly Gln Met Arg Arg Ile Ser Pro Phe Leu Cys Leu
GTG CTT TTG GGC CAA ATG AGG AGA ATC TCT CCC TTC TTG TGT CTA 1063

35 40 45
Lys Asp Arg Ser Asp Phe Arg Phe Pro Gln Glu Lys Val Glu Val
AAG GAC AGA AGT GAC TTC AGA TTC CCC CAG GAG AAG GTG GAA GTC 1108

50 55 60
Ser Gln Leu Gln Lys Ala Gln Ala Met Ser Phe Leu Tyr Asp Val
AGC CAG TTG CAG AAG GCC CAG GCT ATG TCT TTC CTC TAT GAT GTG 1153

Abbildung 12 ff

```
                    65                       70                       75
Leu Gln Gln Val Phe Asn Phe Ser His Lys Ala Leu Leu Cys Cys
TTA CAG CAG GTC TTC AAC TTC TCA CAC AAA GCG CTC CTC TGC TGC 1198

                    80                       85                       90
Met Glu His Asp Leu Pro Gly Pro Thr Pro His Phe Thr Ser Ser
ATG GAA CAT GAC CTT CCT GGA CCA ACT CCA CAC TTT ACG TCA TCA 1243

                    95                      100                      105
Ala Ala Gly Thr Pro Gly Asp Leu Leu Gly Ala Gly Asp Gly Arg
GCA GCT GGA ACA CCT GGA GAC CTG CTT GGT GCA GGA GAT GGG AGA 1288

                   110                      115                      120
Arg Arg Ser Trp Gly Gln Trp Val Ile Glu Gly Ser Thr Leu Ala
AGG AGA AGC TGG GGG CAG TGG GTG ATT GAG GGC TCT ACA CTG GCC 1333

                   125                      130
Leu Arg Arg Tyr Phe Gln Glu Ser Ile Ser Thr
TTG AGG AGG TAT TTC CAG GAA TCC ATC TCT ACC TGA AAGAGAAGAAA 1380
TAAAGATTGTGCCTGGGAAGTTGTCAGAGTGGAAATCATGAGATCCTTTTCATCCACAA 1439
GTTTGCAAGAAAGATTGAGAAGTAAGGATGAAGACCTGGGCTCATCATGAAATGATTCT 1498
CATTGACTAATCTGCCATATCACACTTGTACATGTGACTTTGGATATTCAAAAAGCTCA 1557
TTTCTGTTTCATCAGAAATTATTGAATTAGTTTTAGCAAATACTTTATTAATAGCATAA 1619
AGCAAGTTTATGTCAAAAACATTCAGCTCCTGGGGCATCCGTAACTCAGAGATAACTGC 1675
CCTGATGCTGTTTATTTATCTTCCTTCTTTTTTTTCATGCCTTGTATTTATGATATTTA 1734
TATATTTTATATTTTCATCTTCACATCGTATTAAAATTTATAAAACATTCACTTTTTCA 1793
TATTAAGTTTGCATTTTGTTTTATTAAATTCATATCAAAGAAAACTCTGTAAATGTTTC 1852
TATTCTAAAAACAATGTCTACTTTCTCTTTTTGTAAACCAAATTGAAAATATGGTAAAA 1911
TGTATTAACTCATTCATTTCATTCCTATTATATGTATAAATTGAGTAAATGGCAAACTG 1970
TGGGGTTTTCTTAAAGAAATACAGGTGAATAAAGCAAACACAGTTTCTCTCAGTCTAAG 2029
AGGGAAAGAGACGTAAAAACAGGACAAATATTTATATTATTTCAATTATGTTAAATGCT 2088
ACAAAGAGAAGTAAAGAAAAGTGATGTTCTCACATCAGAAGCTT                2132
```

Abbildung 13 (IFN-pseudo-omega 3)

```
                                                                  CCATG    5
CATAGCAGGAATGCCTTCAGAGAACCTGAAGTCCAAGGTTCATCCAGACCCCAGCTCAG      64

                                             -20             -16
                              Met Val Leu Leu Leu Pro Leu Leu
CTAGGCCAGCAGCACCCTCGTTTCCCA ATG GTC CTC CTG CTT CCT CTA CTC 115

                    -10                 -5              -1
Val Ala Leu Pro Leu Cys His Cys Gly Pro Val Gly Ser Leu Ser
GTG GCC CTG CCG CTT TGC CAC TGT GGC CCT GTT GGA TCT CTG AGC 160

                5                  10                  15
Trp Asp Leu Pro Gln Asn His Gly Leu Leu Ser Arg Asn Thr Leu
TGG GAC CTG CCT CAG AAC CAT GGC CTA CTT AGC AGG AAC ACC TTG 205

               20                  25                  30
Ala Leu Leu Gly Gln Met Cys Arg Ile Ser Thr Phe Leu Cys Leu
GCA CTT CTG GGC CAA ATG TGC AGA ATC TCC ACT TTC TTG TGT CTC 250

               35                  40                  45
Lys Asp Arg Arg Asp Phe Arg Phe Pro Leu Glu Met Trp Met Ala
AAG GAC AGA AGA GAC TTC AGG TTC CCC CTG GAG ATG TGG ATG GCA 295

               50                  55                  60
Val Ser Cys Arg Arg Pro Arg Pro Cys Leu Ser Ser Met Arg Cys
GTC AGT TGC AGA AGG CCC CGG CCC TGT CTG TCC TCC ATG AGA TGC 340

               65                  70                  75
Phe Ser Arg Ser Ser Ala Ser Ser Pro Gln Ser Ala Pro Leu Leu
TTC AGC AGA TCT TCA GCC TCT TCC CCA CAG AGT GCT CCT CTG CTG 385

Pro Gly Thr
CCT GGA ACA TGA CCCTCCTGGACCAACTCCACACTGGACTTCATCTGCAGCTGGA 440
ATGCCTGGATGCTTGCTTAGGGCAGACAAAAAGAGAGGAAGAATCTGTGGGGGTGATTG 499
GGGCCCTACACTGGCCTTGAGGACGTACTTTCAGGGAATGCATGGGAATCCAGGGAATC 558
TACCTGGAGGAGAAGAAATACAGTGACTGTGCTTGGGAGGTTGTCAGAGTGGAATCGTG 617
AAATCCTTCTCTTCATCATCAACAAACTTGCAAGAAGGACTGAGAAGTAAGGATGAAGA 676
CCTGGGTTCATCCTGAAATTATTCTCATTGATTAATCTGCCATATCACACTTGCACATG 735
TGTCTTTGGTCATTTCAATAGGTTCTTATTTCTGCAG                       772
```

Abbildung 14 (IFN-pseudo-omega4)

```
                                AAGCTTTGGGCATGACCTAGTAGGTGACTCTT   32
AGTTGGAGTGGTCAGTTGTAAGGCTCTTGCTCAGTCACGTGGCTCCCCTGTATTTCCCC   91
ACGTTTGCAGCCGTGCTCCTTCTCAATGCTATGAAAGTGTGGGCTCCTCTCCCACTGGA  150
GTGCTGACTGTAGCTTGTATCTTGGCACTCCCAGGCTGTACATAACAGCTCTGAGGTGA  209
TCTCAGGGTTAATGTTTTCTCCCCGACTTGGAGGCCATTGAAGGAAGGGACCTTAGTAG  268
TAGTTGTAACTGAGGGTCTTTTGCTTGTCTCCTGGGGGCTCCACCCCAGAGATGCAGGT  327
GAGCAATCACTCAGTGCATTCAGCTTGGGATGGGGGTGTCTGTGCTGTGGGCCCAAGAC  386
AGGGGTTCCCTGCCTGGTGATGTGAGGGGTGGGTGGTTGACCAATGGCAGACAGACTGG  445
CCTCCTCTCTTGGGTTGACAGCAGCTTGTTGGAGGTATGCATAAGGCACTTGGGGTCTT  504
GCTCCTTCATTAGTTCCAAGGTAGCTGGGGTAGTACCACTGCAGAGGCAGTGTTAGACA  563
GGCTTTCTGTTACCCCTGGGGTCTCCACCTCCTAGAAATGTGAAGTCATGTTAATGGGA  622
GTGTTTAGCCAGAGGAGTGGGGTGGCTGCATGCTGGTGTAAGTATGGGACTTCACTTCT  681
TGGGAAACAGGGAGGTGGAATCTTACCAGCAGGAGACTGTTCTCCTCACGATGTGTAAC  740
CTGCAGTGTGCTGGAAGTTTAGGTGACTGTGGCATGATGTTAGCTTGTAAACAAAGAGC  799
TTCAGACTCTTTGTCTCTTCCCCAGACCCAAGGCAGCAAGGATAAAAGCTGCTGCTGTG  858
GCAGTGGCAGAGGTAGGATGGTTGTGGGAGCCTCTCCCCAGGGAAACTCTAGTCAACTA  917
CCAGTGGATATGCTCAGCCATGGGTAGGGTGACTGTTCTGCAGTCATGGCAGGGGGCC  976
TGCTCCTGAAGAATAGGGACAGGGATTCTCAGGGAAGAGGGGCTGGACTCCTCTTCATA 1035
TAGTGGCGATGATGTGCTGGAGGTGCCAGTGTAGTGAATAGGCCTTTGTTCCTTCCCCA 1094
GCCAGAGGGCTGCTGGGGCTGTCCCACTGCAACGGTCATGGTGGATGGGTTATGGGTTG 1153
ACTGTGGGATTTCTTTCTTGGAGAAATGCTGGACTGCCTGATTGAGGAGACGAGGCAAG 1212
GGAAGAATGCCTGTGCTGGAGTCTCTGGTCAGGTGGCTCTGCCACAGAGGAGAGGTGAC 1271
CACCAGGAACTGCGTGGAGAACAGTGTAGCCACTCTTCTGTGAGGCAGTTGCTCTGTTT 1330
TGGGGATCTGGAGCAGCCGCTATTCCTTACAGATTCCCAGAGCCTGGAGACAGCAAGGG 1389
CAAGAGCTGTGAGAAAGCAAAGATAGCAACCCACCCTTCTCACTGGGAGCTCTGTTCCA 1448
GGGAGATGCAGAGCTGCCATTGCTCAATAGCCCCAGCTGGTAGCTGCAGACCCAGGCCT 1507
GGCAGACCCACCCAGTGAGCAGATAGGGGATTAGGGACCCACATAACACACAGTCTGGC 1566
CACTTTTCCATAGGGCTGCTGAATATGCTGGGGGTCCAATCCAGACCATAGTCACCTCA 1625
CATTTTTCAGTACCTGAAGATATCAACAGTGAAGGCTATGAAACAGTGAAGATGGGGAC 1684
CTGCCCCTGCCTCTGGACCTCTGTTCCAGAGAGGTACAACCTGTTGCCTCCGACATACA 1743
TGCAGGAGGTGGCTGGAGACCCGGTGGATATCCCTCCCACTGAGGAGAAGCAGCATCAG 1802
GGAATCAGGTGAAGAAACAGTCTGGCCACTTTTTGGTAGAGCAGCTGTGCTTGCTGGGG 1861
GTCTGCTACCACCCCCAGCAAAAAGAATGGCATTTGCAAGAATGGCTAAGGCTGCTAAA 1920
CAGCAACAATGGCAACCTACCATTCCCTTTGGAGCGCCATCCCAGGGATATTCGAAACT 1979
```

Abbildung 14 ff

GCTGTCCACTAGAAAACAGTGGTGGAGGTGACTGGAGACCCCAGTGGAGAGTTTCACCT 2038
GGTGAAAAGAAACAGGATTTGGGATCGACATGAATAACCAATCTGACTGCTTCCCCGTA 2097
GAGCTGCTGGACTGTGCTGGGTGGCTGCTCCAGTCCCTAGCTGCCTTGGACTCCCGAGA 2156
ACCCAAAGGCTCCAATAGCTAAGATTGTGAAAGAGCAAAGATGGCAGCCCACCCCCTGC 2215
CACAGGGAGCTCCATGTCAGGGAGGTATGAGGCTGCTACCAGTGTCTGGCTGGATTCCC 2274
AAGTCGAGTGGGTCTTACCCTGAGACAGGCCATGGAAGGTGGGCCTGTCATTGTCACTG 2333
CCCAGCGCCCTGGATGAAACCCCTTTCCTAGGGGTATGTATAGGGGTCTAGCGTCCTGC 2392
TTGGCTGGAGTTATAGCTTCTTTGTGGGGAGGCCTGGGTATCTAAGGCTCCAGGGTAC 2451
CCATGCATGCGAGAGCGGCTGCTCTGCTGAACCCTACGTAGCCCTGCATGTCAGACTAA 2510
ATGCCCTGGTAGAGTGGGTTCACTAGGAGATCTCCTGACCTGAGGATTGCAAAGATCTG 2569
TGGGAGAAGCGTGGGTCCCCAGGGCTGCTCACTTACTCACCACTTCCCTGGGCAGGAGA 2628
GGCTCCCCTGGCTCTGTGTCATCCTGGGGGGCAGTTGTCCTGCCTTACTTTGCTTTAT 2687
TCTCCATGGGTCAAGTTGTTTTCTTGAGTCTCAATGTGTGCACCTGGTTTTTCAGTTG 2746
AAGGTGCTGTATTTACTTGCCCCTTCCATTTCTCTCCATGAGAGTGGCACACACTAGCA 2805
GGTTCCAGTCGGCCATCTTGCAACCCCTGAAAACTATTTGTTTCCAGCTATAAGCCATT 2864
GAGAGAACCTGGAGTGGCATAAAAAGAATGCCTCGGGGTTCATCCCGACCCCAGCTCAG 2923

-20 -16
Met Val Leu Leu Leu Val Leu Leu
CTAGGCCAGCAGCACCCTCGTTTCCCA ATG GTC CTA CTG CTT GTT CTA CTG 2974

-10 -5 -1
Val Ala Leu Leu Leu Cys Gln Cys Gly Pro Val Gly Ser Leu Gly
GTG GCC CTG CTG CTT TGC CAA TGT GGC CCT GTT GGA TCT CTG GGC 3019

5 10 15
Phe Asp Leu Pro Gln Asn Arg Gly Leu Leu Ser Arg Asn Thr Leu
TTT GAC CTG CCT CAG AAC CGT GGC CTA CTT AGC AGG AAC ACC TTG 3064

20 25 30
Ala Phe Trp Ala Lys Cys Arg Ile Ser Thr Phe Leu Cys Leu Lys
GCA TTC TGG GCC AAA TGC AGA ATC TCC ACT TTC TTG TGT CTC AAG 3109

35 40 45
Asp Arg Arg Asp Phe Arg Phe Pro Leu Glu Met Trp Met Ala Val
GAC AGA AGA GAC TTC AGG TTC CCC CTG GAG ATG TGG ATG GCA GTC 3154

50 55 60
Ile Cys Arg Arg Pro Arg Leu Cys Leu Ser Ser Met Arg Cys Phe
ATT TGC AGA AGG CCC AGG CTG TGT CTG TCC TCC ATG AGA TGC TTC 3199

65 70 75
Ser Arg Ser Ser Ala Ser Ser Pro Gln Ser Ala Pro Leu Leu Pro
AGC AGA TCT TCA GCC TCT TCC CCA CAG AGC GCT CCT CTG CTG CCT 3244

Abbildung 14 ff

Gly Thr

GGA ACA TGA CCCTCCTGGACCAGCTCCACACTGGATTTCATCAGCAGCTCGAATAG 3300

CCTGGAGTCTTGCTTAGGGCAGGCAACAGGAGAGGAAGAATCTGTGGGGGTGATTGGGA 3359

CCCTACACTGGCCTTGAGGAGGTACTTCCAGGGAATCCATGGGAATCCAGAGAATCTAC 3418

CTGAAAGAGAAGAAATACAGTGACTGTGCTTAGGAGGTTGTCAGAATGGAATCATGAAA 3477

TCCTTCTCTTCATCAACAGACTTGCAAGGACTGAGAAGTAAGGATGAAGACCTGGGGTC 3536

TGCTTTACTCTTTCTTATTTCTTCCTCTTCCTTACTATGTGTTTATTTCTTCTTTTTC 3595

TAGTTCCTTAACTTGTAAGTAGTTCACTTGGTTTGAGGTCTTTCTTCTTTTTTAATATA 3654

AGCTT 3659

Abbildung 15

```
 M A L L F P L L A A L V M T S Y S P V G S L G C D L P Q N H
ATGGCCCTCCTGTTCCCTCTACTGGCAGCCCTAGTGATGACCAGCTATAGCCCTGTTGGATCTCTGGGCTGTGATCTGCCTCAGAACCAT
 M A L L F P L L A A L E V C S C G S S G S L G Y N L P Q N H
ATGGCCCTCCTCTTCCCTCTACTGGCAGCCCTAGAGGTGTGCAGCTGTGGCTCTTCTGGATCTCTAGGATATAACCTGCCTCAGAACCAT
 M V L L L P L L V A L P L C H C G P V G S L S W D L P Q N H
ATGGTCCTCCTGCTTCCTCTACTCGTGGCCCTGCCGCTTTGCCACTGTGGCCCTGTTGGATCTCTGAGCTGGGACCTGCCTCAGAACCAT
 M V L L L V L L V A L L L C Q C G P V G S L G F D L P Q N R
ATGGTCCTACTGCTTGTTCTACTGGTGGCCCTGCTGCTTTGCCAATGTGGCCCTGTTGGATCTCTGGGCTTTGACCTGCCTCAGAACCGT
        10        20        30        40        50        60        70        80        90

 G L L S R N T L V L L H Q M R R I S P F L C L K D R R D F R
GGCCTACTTAGCAGGAACACCTTGGTGCTTCTGCACCAAATGAGGAGAATCTCCCCTTTCTTGTGTCTCAAGGACAGAAGAGACTTCAGG
 G L L G R N T L V L L G Q M R R I S P F L C L K D R S D F R
GGCCTGCTAGGCAGGAACACCTTGGTGCTTTTGGGCCAAATGAGGAGAATCTCTCCCTTCTTGTGTCTAAAGGACAGAAGTGACTTCAGA
 G L L S R N T L A L L G Q M C R I S T F L C L K D R R D F R
GGCCTACTTAGCAGGAACACCTTGGCACTTCTGGGCCAAATGTGCAGAATCTCCACTTTCTTGTGTCTCAAGGACAGAAGAGACTTCAGG
 G L L S R N T L A   L G Q M   R I S T F L C L K D R R D F R
GGCCTACTTAGCAGGAACACCTTGGCA.TTCTGGGCCAAATG..CAGAATCTCCACTTTCTTGTGTCTCAAGGACAGAAGAGACTTCAGG
       100       110       120       130       140       150       160       170       180

 F P Q E M V K G S Q L Q K A H V M S V L H E M L Q Q I F S L
TTCCCCCAGGAGATGGTAAAAGGGAGCCAGTTGCAGAAGGCCCATGTCATGTCTGTCCTCCATGAGATGCTGCAGCAGATCTTCAGCCTC
 F P Q E K V E V S Q L Q K A Q A M S F L Y D V L Q Q V F   L
TTCCCCCAGGAGAAGGTGGAAGTCAGCCAGTTGCAGAAGGCCCAGGCTATGTCTTTCCTCTATGATGTGTTACAGCAGGTCTTCAA.CTT
 F P L E M   D G S Q L Q K A P A L S V L H E M L Q Q I F S L
TTCCCCCTGGAGATG.TGGATGGCAGTCAGTTGCAGAAGGCCCCGGCCCTGTCTGTCCTCCATGAGATGCTTCAGCAGATCTTCAGCCTC
 F P L E M   D G S H L Q K A Q A Y S V L H E M L Q Q I F S L
TTCCCCCTGGAGATG.TGGATGGCAGTCATTTGCAGAAGGCCCAGGCTGTGTCTGTCCTCCATGAGATGCTTCAGCAGATCTTCAGCCTC
       190       200       210       220       230       240       250       260       270

 F H T E R S S A A W N M T L L D Q L H T G L H Q·Q L Q   H L E
TTCCACACAGAGCGCTCCTCTGCTGCCTGGAACATGACCCTCCTAGACCAACTCCACACTGGACTTCATCAGCAACTGCAAC.ACCTGGA
   H T K R S S A A W N M T F L D Q L H T L R H Q Q L E   H L E
.CTCACACAAAGCGCTCCTCTGCTGCATGGAACATGACCTTCCTGGACCAACTCCACACTTTACGTCATCAGCAGCTGGAAC.ACCTGGA
 F P T E C S S A A W N M T L L D Q L H T G L H L Q L E   C L E
TTCCCCACAGAGTGCTCCTCTGCTGCCTGGAACATGACCCTCCTGGACCAACTCCACACTGGACTTCATCTGCAGCTGGAAT.GCCTGGA
 F P T E R S S A A W N M T L L D Q L H T G F H Q Q L E   S L E
TTCCCCACAGAGCGCTCCTCTGCTGCCTGGAACATGACCCTCCTGGACCAGCTCCACACTGGATTTCATCAGCAGCTCGAATAGCCTGGA
       280       290       300       310       320       330       340       350       360

 T C L L Q V V G E G E S A G         A I S S P A L T L R R Y F
GACCTGCTTGCTGCAGGTAGTGGGAGAAGGAGAATCTGCTGGG........GCAATTAGCAGCCCTGCACTGACCTTGAGGAGGTACTTC
 T C L V Q E M G E G E   A G         V I E G S I L A L R R Y F
GACCTGCTTGGTGCAGGAGATGGGAGAAGGAGAA...GCTGGGGGCAGTGGGTGATTGAGGGCTCTACACTGGCCTTGAGGAGGTATTTC
 S C L G Q T K R E E·E S V G         V I G   P T L A L R T Y F
GTCTTGCTTAGGGCAGACAAAAAGAGAGGAAGAATCTGTGGGG........GTGATTGGGG.CCCTACACTGGCCTTGAGGACGTACTTT
 S C L G Q A T G E E E S V G       . V I G   P T L A L R R Y F
GTCTTGCTTAGGGCAGGCAACAGGAGAGGAAGAATCTGTGGGG........GTGATTGGGA.CCCTACACTGGCCTTGAGGAGGTACTTC
       370       380       390       400       410       420       430       440       450

 Q G I R             ·V Y L K E K K Y S D C A W E·V V R M E I M K
CAGGGAATCCGT.............GTCTACCTGAAAGAGAAGAAATACAGCGACTGTGCCTGGGAAGTTGTCAGAATGGAAATCATGAA
 Q   I H              L Y L K E K K *   D C A W E V V R V E I M R
CAGGA.ATCCAT.............CTCTACCTGAAAGAGAAGAAATAAA..GATTGTGCCTGGGAAGTTGTCAGAGTGGAAATCATGAG
 Q G M H              I Y L E E K K Y S D C A W E V V R V E   V K
CAGGGAATGCATGGGAATCCAGGGAATCTACCTGGAGGAGAAGAAATACAGTGACTGTGCTTGGGAGGTTGTCAGAGTGGAA.TCGTGAA
 Q G I H              I Y L K E K K Y S D C A * E V V R M E I M K
CAGGGAATCCATGGGAATCCAGAGAATCTACCTGAAAGAGAAGAAATACAGTGACTGTGCTTAGGAGGTTGTCAGAATGGAA.TCATGAA
       460       470       480       490       500       510       520       530       540

 S L F   L S T N M Q E R L R S K D R D L G S S *
ATCCTTGTTC...TTATCAACAAACATGCAAGAAAGACTGAGAAGTAAAGATAGAGACCTGGGCTCATCTTGA
 S   F   S S T S L Q E R L R S K D E D L.G S S *
ATCC...TTT...TCATCCACAAGTTTGCAAGAAAGATTGAGAAGTAAGGATGAAGACCTGGGCTCATCATGA
 S F S S S S T N L Q E G L R S K D E D L G S S *
ATCCTTCTCTTCATCATCAACAAACTTGCAAGAAGGACTGAGAAGTAAGGATGAAGACCTGGGTTCATCCTGA
 S F S   S S T D L Q   G L R S K D E D L G S A L
ATCCTTCTCT...TCATCAACAGACTTGCAA...GGACTGAGAAGTAAGGATGAAGACCTGGGGTCTGCTTTA
       550       560       570       580       590       600       610
```

Abbildung 16

| | omega1 | omega2 | omega3 | omega4 |
|---|---|---|---|---|
| omega1 | ------ | 492/577 | 497/587 | 493/579 |
| omega2 | 85,3 % | ------ | 473/577 | 466/570 |
| omega3 | 84,7 % | 82,0 % | ------ | 554/592 |
| omega4 | 85,4 % | 81,8 % | 93,6 % | ------ |

Oberer rechter Teil: Zahl der identischen Basen / insgesamt verglichene Basen.
Unterer linker Teil: Prozent Homologie

Abbildung 17

| | omega1 | omega2 | omega3 | omega4 |
|---|---|---|---|---|
| omega1 | ------ | 16/23 | 13/23 | 13/23 |
| omega2 | 69,6 % | ------ | 14/23 | 14/23 |
| omega3 | 56,6 % | 60,9% | ------ | 19/23 |
| omega4 | 56,5 % | 60,9 % | 82,6 % | ------ |

Rechter oberer Teil: Zahl der identischen Aminosäuren von insgesamt 23 Aminosäuren.
Linker unterer Teil: Prozent Homologie.

Abbildung 18

| | omega1 | omega2 | omega3 | omega4 |
|---|---|---|---|---|
| omega1 | ------ | 128/166 | 130/169 | 131/167 |
| omega2 | 77,1 % | ------ | 118/163 | 124/161 |
| omega3 | 76,9 % | 72,4 % | ------ | 146/166 |
| omega4 | 78,4 % | 77,0 % | 88,0 % | ------ |

Oberer rechter Teil: Zahl der identischen Aminosäuren / Zahl der insgesamt verglichenen Aminosäuren.
Unterer linker Teil: Prozent Homologie.